# EUROPEAN PATENT APPLICATION

(11) **EP 4 286 383 A1**
(43) Date of publication of application: **06.12.2023**
(21) Application number: 22745294.3
(22) Date of filing: 27.01.2022
(51) Int. Cl.: C07D 471/04, C07D 403/14, C07C 211/50, C07D 519/00, A61K 31/437, A61P 35/00, A61P 37/00

(54) **BIPHENYL COMPOUND AS IMMUNOMODULATOR, PREPARATION METHOD THEREFOR AND USE THEREOF**

(30) Priority: 01.02.2021 CN 202110137428
(71) Applicant: Shenzhen Chipscreen Biosciences Co., Ltd., Guangdong 518057 (CN)
(72) Inventor: YANG, Qianjiao, Shenzhen, Guangdong 518057 (CN); SHAN, Song, Shenzhen, Guangdong 518057 (CN); XIN, Lijun, Shenzhen, Guangdong 518057 (CN); PAN, Desi, Shenzhen, Guangdong 518057 (CN); WANG, Xiaoliang, Shenzhen, Guangdong 518057 (CN); SONG, Yonglian, Shenzhen, Guangdong 518057 (CN); HUANG, Huiyun, Shenzhen, Guangdong 518057 (CN); WEI, Qi, Shenzhen, Guangdong 518057 (CN); LI, Zhibin, Shenzhen, Guangdong 518057 (CN); LU, Xianping, Shenzhen, Guangdong 518057 (CN)
(74) Representative: Lavoix
(86) International application number: PCT/CN2022/074183
(87) International publication number: WO 2022/161421

(57) **Abstract**

A biphenyl compound as shown in formula (I), a preparation method therefor and use thereof, and a pharmaceutical composition comprising the compound as an active ingredient. The compound is a novel small-molecule immunomodulator having excellent oral absorption characteristics, and can be used for treating and/or preventing various immune-related diseases.

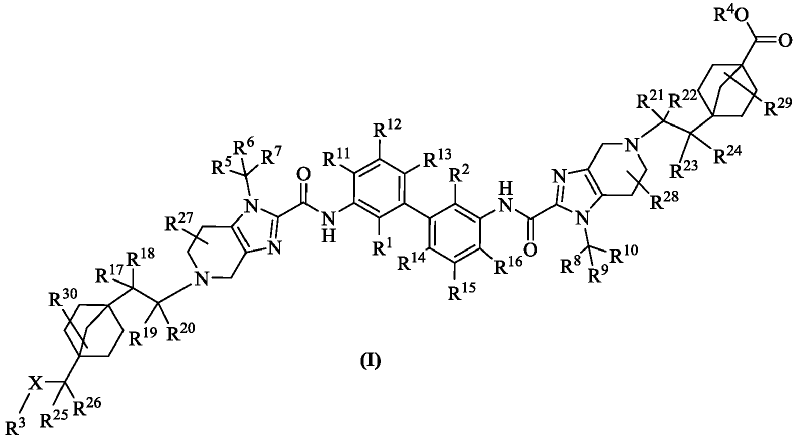

## Description

### CROSS REFERENCE TO RELATED APPLICATIONS

This application claims the priority of Chinese Patent Application No. 202110137428.0, filed with the China National Intellectual Property Administration on February 01, 2021, and titled with "BIPHENYL COMPOUND AS IMMUNOMODULATOR, PREPARATION METHOD THEREFOR AND USE THEREOF", which is hereby incorporated by reference in its entirety.

### FIELD

The present invention belongs to the field of pharmaceutical chemistry, and in particular relates to a biphenyl compound as an immunomodulator, a preparation method and use thereof.

### BACKGROUND

Tumor immunotherapy is a new treatment method that stimulates the body's immune system and enhances its own anti-tumor immunity, thereby inhibiting or killing tumor cells. This method has achieved a breakthrough after more than a hundred years of hard work. In 2013, "Science" magazine listed tumor immunotherapy as the top of the top ten scientific breakthroughs of the year (Couzin-Frankel J., 2013, Science, 342: 1432-1433), which has become one of the most promising anti-tumor therapeutic fields.

Compared with normal cells, tumor cells have a variety of genetic and epigenetic changes. The immune system can distinguish the tumor cells and normal cells through the surface antigens produced by tumor cells, thereby triggering an anti-tumor immune response. In the process of anti-tumor immune by T cells, after T cells are activated by the antigen recognition signal mediated by T cell receptor (TCR), T cell effects are comprehensively regulated by co-stimulation and co-inhibition signals, including inhibitory receptors for inhibitory signals such as cytotoxic T-lymphocyte associated antigen 4 (CTLA4), programmed death protein 1 (PD-1), V-domain immunoglobulin suppressor of T-cell activation (VISTA), T cell immunoglobulin and mucin domain-containing-3 (TIM3) and lymphocyte activation gene 3 (LAG3), and activating receptors for stimulating signals such as CD28, CD134 (OX40), glucocorticoid-induced TNFR-related protein (GITR), CD137, CD27 and HVEM (Mellman I., Coukos G., Dranoff G., 2011, Nature, 480: 480-489). Under normal physiological conditions, for one thing, immune checkpoints are involved in maintaining immune tolerance to self-antigens and avoiding autoimmune diseases; for another thing, immune checkpoints are involved in preventing excessive activation of immune responses to prevent tissue damage. However, tumor cells can evade immune killing by inhibiting the activation of T cells through immune checkpoints. Therefore, it is necessary to reactivate T cells to attack tumor cells by activating co-stimulatory signals (pressing the "accelerator") and inhibiting co-inhibitory signals (releasing the "brake") to achieve tumor immunotherapy.

PD-1 is expressed in activated T cells, B cells and bone marrow cells, and belongs to the CD28 family. It is a type 1 transmembrane glycoprotein on T cells and consists of 288 amino acids. The molecular structure of PD-1 consists of an extracellular region with IgV-like immunoglobulin (amino acids 35-145), a transmembrane region, and a cytoplasmic tail region with the function of linking signal peptides. The extracellular region binds to ligands to play important functions (Cheng X., Veverka V, Radhakrishnan A., et al. 2013, J. Biol. Chem., 288: 11771-11785). Programmed death protein ligand 1 (PD-L1) is one of the ligands of PD-1, which belongs to the B7 family and is persistently expressed in a variety of tumor cells, T cells, antigen-presenting cells (APC) and a variety of non-hematopoietic cells. It is also a type 1 transmembrane glycoprotein, and consists of 290 amino acids. The interaction of PD-1 and PD-L1 can inhibit the activation of T cells, which is crucial for maintaining the immune tolerance of the normal body. However, in tumor cells and virus infection, PD-1 on T cells is induced to be highly expressed, the expression of PD-L1 is up-regulated, leading to the continuous activation of PD-1 signaling pathway, inhibiting the proliferation of T cells, and resulting in the immune escape of tumor cells and pathogens (Fuller M.J., Callendret B., Zhu B., et al. 2013, Proc. Natl USA., 110: 15001-15006; Dolan D.E., Gupta S., 2014, Cancer Control, 21: 231-237; Chen L., Han X., 2015, J. Clin. Invest., 125 : 3384-3391; Postow M.A., Callahan M.K., Wolchok J.D., 2015, J. Clin. Oncol., 33: 1974-1982). Multiple antibody drugs of PD-1 and PD-L1 that have been marketed in recent years have fully proved that blocking the interaction of PD-1/PD-L1 is a very effective treatment in the immunotherapy of tumors and other immune-related diseases means.

Studies have found that PD-L1 can interact with CD80, which inhibits the binding of PD-L1 and PD-1 and the activation ability of T cells. Therefore, blocking the immune activation caused by the interaction of CD80/PD-L1 may also promote the enhancement of activity of T cells, thereby providing new therapeutic opportunities for immune-related diseases (Sugiura D., Maruhashi T., Okazaki ll-mi, et al. 2019, Science, 364: 558-566).

So far, significant progress has been made in targeting PD-1/PD-L1 antibody drugs. However, all antibody drugs must be administered by injection, and have various ADMET problems, serious side effects related to the immune system, etc. Compared with antibody drugs, small molecular immunomodulators have certain advantages, such as oral administration, better tissue permeability, and pharmacological properties that can be adjusted to minimize side effects. In addition, small molecular inhibitors will have lower price advantages and better medication compliance.

Clinical studies of PD-1/PD-L1 antibody drugs have shown that nivolumab has a T1/2 of 25.2 days, and is administered at a frequency of once every two weeks; pembrolizumab has a T1/2 of 25 days, and is administered at a frequency of once every three weeks; atezolizumab has a T1/2 of 27 days, and is administered at a frequency of once every three weeks. The administration frequency of the above drugs is shorter than the half-life of the drugs, indicating that the continuous exposure of such target drugs in the body is the key to obtaining ideal clinical efficacy. However, the exposure amount of the existing small molecular immunomodulators in the body is relatively low, and the duration of the continuous exposure is relatively short, which will affect the clinical efficacy. Therefore, it is of great significance to develop new small molecular PD-L1 immunomodulators with higher activity and better oral absorption characteristics, especially with sufficient *in vivo* exposure amount and sustained exposure time, and more targeted to tumor tissues to meet the unmet clinical needs.

### SUMMARY

In an aspect, the present invention relates to a small molecular biphenyl compound capable of targeting PD-L1, or an isomer, a pharmaceutically acceptable salt, a precursor or a metabolite thereof.

In another aspect, the present invention relates to a method for preparing the compound described herein.

In another aspect, the present invention relates to a pharmaceutical composition comprising the compound of the present invention as an active ingredient, and use of the compound or the pharmaceutical composition of the present invention in the manufacture of a medicament for treating and/or preventing diseases related to the target PD-L1.

Compared with antibodies, a small molecular PD-L1 inhibitor needs to be optimized in terms of the binding strength to target and the duration of continuous exposure *in vivo* to narrow the possible gap in drug efficacy between the antibodies and the small molecular PD-L1 inhibitors. Therefore, the inventors expect to develop novel small molecular PD-L1 immunomodulators with higher activity and better oral absorption characteristics, especially with sufficient *in vivo* exposure amount and continuous exposure time, and more targeted to tumor tissues to meet the unmet clinical needs.

The present invention overcomes the disadvantage that the existing PD-1/PD-L1 antibody drugs need to be administered by injection, and provides a novel small molecular immunomodulator with excellent oral absorption characteristics. In particular, the compounds of the present invention have ideal in vivo exposure amount and continuous exposure time, and are targeted to tumor tissue, which can be enriched in tumor tissue and form a higher exposure concentration at tumor tissue, so as to help to better exert anti-tumor activity during treatment, thereby achieving better efficacy.

The present invention provides a compound represented by formula (I), an isomer, a pharmaceutically acceptable salt, a precursor or a metabolite thereof to achieve the above object of the present invention.

Therefore, in the first aspect, the present invention relates to a compound represented by formula (I), an isomer, a pharmaceutically acceptable salt, a precursor or a metabolite thereof, 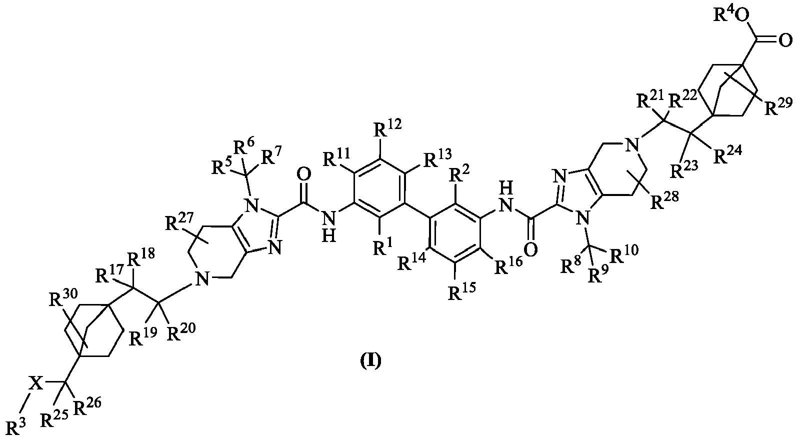 wherein,
R¹ and R² are the same or different, and are selected from the group consisting of C₁-C₆ alkyl, cyano and halogen;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono-C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, 3- to 14-membered heterocycloalkyl, C₃-C₁₄ cycloalkyl-C₁-C₄ alkyl, and 3- to 14-membered heterocycloalkyl-C₁-C₄ alkyl;
R⁴ is selected from the group consisting of hydrogen and C₁-C₆ alkyl, wherein the C₁-C₆ alkyl is optionally substituted by one or more substituents selected from the group consisting of hydroxyl, carboxyl and halogen;
X is selected from the group consisting of -O- and -S-;
⁵-R²⁶ are selected from the group consisting of hydrogen atom;
R²⁷-R³⁰ are selected from the group consisting of hydrogen atom;
wherein, at least one hydrogen atom in each group of the R¹-R³⁰ defined above is replaced by deuterium (D).

In some embodiments, R¹ and R² can be the same or different, and are selected from the group consisting of methyl, cyano, and halogen.

In some embodiments, R¹ and R² can be the same or different, and are selected from the group consisting of methyl, cyano, fluorine, and chlorine.

In some embodiments, R¹ is selected from the group consisting of C₁-C₆ alkyl, cyano, and halogen.

In some embodiments, R² is selected from the group consisting of C₁-C₆ alkyl and halogen.

In some embodiments, R¹ is selected from the group consisting of methyl, cyano, fluorine, and chlorine.

In some embodiments, R² is selected from the group consisting of methyl and chlorine.

In some embodiments, R³ is selected from the group consisting of C₁-C₆ alkyl and halogenated C₁-C₆ alkyl, wherein at least one hydrogen atom in these substituents is replaced by deuterium (D).

In some embodiments, R³ is selected from the group consisting of C₁-C₆ alkyl, and fluoro C₁-C₆ alkyl, wherein at least one hydrogen atom in these substituents is replaced by deuterium (D).

In some embodiments, R³ is selected from the group consisting of methyl, ethyl, and -CHF₂, wherein at least one hydrogen atom in these substituents is replaced by deuterium (D).

In some embodiments, R⁴ is selected from the group consisting of hydrogen, and C₁-C₆ alkyl.

In some embodiments, R⁴ is selected from the group consisting of hydrogen, and methyl.

In some embodiments, X is selected from the group consisting of -O-.

In some embodiments, R⁵-R²⁶ are selected from the group consisting of hydrogen atom, wherein preferably at least one hydrogen atom is replaced by deuterium (D).

In some embodiments, R⁵-R²⁶ are all hydrogen atoms.

In some embodiments, R⁵-R²⁶ are hydrogen atoms, wherein at least one hydrogen atom is replaced by deuterium (D).

In some embodiments, R²⁷-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R²⁷-R³⁰ are selected from the group consisting of hydrogen atom.

In some embodiments, R²⁷-R³⁰ are selected from the group consisting of hydrogen atom wherein at least one hydrogen atom is replaced by deuterium (D), wherein the deuterium can be at any position on the corresponding alicyclic ring and aliphatic heterocyclic ring.

In another aspect, the present invention relates to a compound represented by formula (I), an isomer, a pharmaceutically acceptable salt, a precursor or a metabolite thereof,
R¹ and R² can be the same or different, and are selected from the group consisting of methyl, methyl-*d₃* (CD₃), cyano, fluorine, chlorine, and bromine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono-C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, 3- to 14-membered heterocycloalkyl, C₃-C₁₄ cycloalkyl-C₁-C₄ alkyl, and 3- to 14-membered heterocycloalkyl-C₁-C₄ alkyl;
R⁴ is selected from the group consisting of hydrogen and C₁-C₆ alkyl, wherein the C₁-C₆ alkyl is optionally substituted by one or more substituents selected from the group consisting of hydroxyl and halogen;
X is selected from the group consisting of -O-;
R⁵-R²⁶ are selected from the group consisting of hydrogen atom, wherein at least one hydrogen atom is replaced by deuterium (D);
R²⁷-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R¹ and R² can be the same or different, and are selected from the group consisting of methyl, cyano, fluorine and chlorine.

In some embodiments, R¹ is selected from the group consisting of methyl, cyano, fluorine, and chlorine.

In some embodiments, R² is selected from the group consisting of methyl and chlorine.

In some embodiments, R³ is selected from the group consisting of C₁-C₆ alkyl, and halogenated C₁-C₆ alkyl.

In some embodiments, R³ is selected from the group consisting of C₁-C₆ alkyl, and fluoro C₁-C₆ alkyl.

In some embodiments, R³ is selected from the group consisting of methyl, ethyl, and -CHF₂.

In some embodiments, R⁴ is selected from the group consisting of hydrogen, and C₁-C₆ alkyl.

In some embodiments, R⁴ is selected from the group consisting of hydrogen and methyl.

In some embodiments, R²⁷-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R²⁷-R³⁰ are selected from the group consisting of hydrogen atom.

In some embodiments, R²⁷-R³⁰ are selected from the group consisting of hydrogen atom, wherein at least one hydrogen atom is replaced by deuterium (D), wherein the deuterium can be at any position on the corresponding alicyclic ring and aliphatic heterocyclic ring.

In another aspect, the present invention relates to a compound represented by formula (I), an isomer, a pharmaceutically acceptable salt, a precursor or a metabolite thereof,
R¹ and R² can be the same or different, and are selected from the group consisting of methyl, methyl-*d₃* (CD₃), cyano, fluorine, chlorine, and bromine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono-C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, 3- to 14-membered heterocycloalkyl, C₃-C₁₄ cycloalkyl-C₁-C₄ alkyl, and 3- to 14-membered heterocycloalkyl-C₁-C₄ alkyl;
R⁴ is selected from the group consisting of hydrogen and C₁-C₆ alkyl, wherein the C₁-C₆ alkyl is optionally substituted by one or more substituents selected from the group consisting of hydroxyl and halogen;
X is selected from the group consisting of -O-;
R⁵-R²⁶ are selected from the group consisting of hydrogen atom, or selected from the group consisting of hydrogen atom wherein at least one hydrogen atom is replaced by deuterium (D);
R²⁷-R³⁰ are selected from the group consisting of hydrogen atom, wherein at least one hydrogen atom is replaced by deuterium (D), wherein the deuterium can be at any position on the corresponding alicyclic ring and aliphatic heterocyclic ring.

In some embodiments, R¹ and R² can be the same or different, and are selected from the group consisting of methyl, cyano, fluorine and chlorine.

In some embodiments, R¹ is selected from the group consisting of methyl, cyano, fluorine, and chlorine.

In some embodiments, R² is selected from the group consisting of methyl and chlorine.

In some embodiments, R³ is selected from the group consisting of C₁-C₆ alkyl, and halogenated C₁-C₆ alkyl.

In some embodiments, R³ is selected from the group consisting of C₁-C₆ alkyl, and fluoro C₁-C₆ alkyl.

In some embodiments, R³ is selected from the group consisting of methyl, ethyl, and -CHF₂.

In some embodiments, R⁴ is selected from the group consisting of hydrogen, and C₁-C₆ alkyl.

In some embodiments, R⁴ is selected from the group consisting of hydrogen and methyl.

In some embodiments, R⁵-R²⁶ are selected from the group consisting of hydrogen atom, or selected from the group consisting of hydrogen atom wherein at least one hydrogen atom is replaced by deuterium (D).

In some embodiments, R⁵-R²⁶ are selected from the group consisting of hydrogen atom.

In some embodiments, R⁵-R²⁶ are selected from the group consisting of hydrogen atom, wherein at least one hydrogen atom is replaced by deuterium (D).

In a preferred aspect, the present invention relates to a compound represented by formula (I), an isomer, a pharmaceutically acceptable salt, a precursor or a metabolite thereof, wherein,
R¹ and R² can be the same or different, and are selected from the group consisting of methyl, methyl-*d₃* (CD₃), cyano, fluorine and chlorine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono-C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, and 3- to 14-membered heterocycloalkyl; wherein at least one hydrogen atom in these groups is replaced by deuterium (D);
R⁴ is selected from the group consisting of hydrogen and C₁-C₆ alkyl, wherein the C₁-C₆ alkyl is optionally substituted by halogen;
X is selected from the group consisting of -O-;
R⁵-R²⁶ are selected from the group consisting of hydrogen atom, or selected from the group consisting of hydrogen atom wherein at least one hydrogen atom is replaced by deuterium (D);
R²⁷-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R¹ is selected from the group consisting of methyl, cyano, fluorine, and chlorine.

In some embodiments, R² is selected from the group consisting of methyl and chlorine.

In some embodiments, R³ is selected from the group consisting of C₁-C₆ alkyl, and halogenated C₁-C₆ alkyl, wherein at least one hydrogen atom in these substituents is replaced by deuterium (D).

In some embodiments, R³ is selected from the group consisting of C₁-C₆ alkyl, and fluoro C₁-C₆ alkyl, wherein at least one hydrogen atom in these substituents is replaced by deuterium (D).

In some embodiments, R³ is selected from the group consisting of methyl, ethyl, and -CHF₂, wherein at least one hydrogen atom in these substituents is replaced by deuterium (D).

In some embodiments, R⁴ is selected from the group consisting of hydrogen, and C₁-C₆ alkyl.

In some embodiments, R⁴ is selected from the group consisting of hydrogen and methyl.

In some embodiments, R⁵-R²⁶ are selected from the group consisting of hydrogen atom, or selected from the group consisting of hydrogen atom wherein at least one hydrogen atom is replaced by deuterium (D).

In some embodiments, R⁵-R²⁶ are selected from the group consisting of hydrogen atom.

In some embodiments, R⁵-R²⁶ are selected from the group consisting of hydrogen atom, wherein at least one hydrogen atom is replaced by deuterium (D).

In some embodiments, R²⁷-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R²⁷-R³⁰ are selected from the group consisting of hydrogen atom.

In some embodiments, R²⁷-R³⁰ are selected from the group consisting of hydrogen atom, wherein at least one hydrogen atom is replaced by deuterium (D), wherein the deuterium can be at any position on the corresponding alicyclic ring and aliphatic heterocyclic ring.

In another preferred aspect, the present invention relates to a compound represented by formula (I), an isomer, a pharmaceutically acceptable salt, a precursor or a metabolite thereof, wherein,
R¹ and R² can be the same or different, and are selected from the group consisting of methyl, methyl-*d₃* (CD₃), cyano, fluorine and chlorine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl;
R⁴ is selected from the group consisting of hydrogen and C₁-C₆ alkyl, wherein the C₁-C₆ alkyl is optionally substituted by halogen;
X is selected from the group consisting of -O-;
R⁵-R²⁶ are selected from the group consisting of hydrogen atom, wherein at least one hydrogen atom is replaced by deuterium (D);
R²⁷-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R¹ is selected from the group consisting of methyl, cyano, fluorine, and chlorine.

In some embodiments, R² is selected from the group consisting of methyl and chlorine.

In some embodiments, R³ is selected from the group consisting of C₁-C₆ alkyl, and halogenated C₁-C₆ alkyl.

In some embodiments, R³ is selected from the group consisting of C₁-C₆ alkyl, and fluoro C₁-C₆ alkyl.

In some embodiments, R³ is selected from the group consisting of methyl, ethyl, and -CHF₂.

In some embodiments, R⁴ is selected from the group consisting of hydrogen, and C₁-C₆ alkyl.

In some embodiments, R⁴ is selected from the group consisting of hydrogen.

In some embodiments, R²⁷-R³⁰ are all selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R²⁷-R³⁰ are selected from the group consisting of hydrogen atom.

In some embodiments, R²⁷-R³⁰ are selected from the group consisting of hydrogen atom, wherein at least one hydrogen atom is replaced by deuterium (D), wherein the deuterium can be at any position on the corresponding alicyclic ring and aliphatic heterocyclic ring.

In another preferred aspect, the present invention relates to a compound represented by formula (I), an isomer, a pharmaceutically acceptable salt, a precursor or a metabolite thereof, wherein,
R¹ and R² can be the same or different, and are selected from the group consisting of methyl, methyl-*d₃* (CD₃), cyano, fluorine and chlorine;
R³ is selected from the group consisting of C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl;
R⁴ is selected from the group consisting of hydrogen and C₁-C₆ alkyl, wherein the C₁-C₆ alkyl is optionally substituted by halogen;
X is selected from the group consisting of -O-;
R⁵-R²⁷ are selected from the group consisting of hydrogen atom, or selected from the group consisting of hydrogen atom wherein at least one hydrogen atom is replaced by deuterium (D);
R²⁸-R³⁰ are selected from the group consisting of hydrogen atom, wherein at least one hydrogen atom is replaced by deuterium (D), wherein the deuterium can be at any position on the corresponding alicyclic ring and aliphatic heterocyclic ring.

In some embodiments, R¹ is selected from the group consisting of methyl, cyano, fluorine, and chlorine.

In some embodiments, R² is selected from the group consisting of methyl and chlorine.

In some embodiments, R³ is selected from the group consisting of C₁-C₆ alkyl, and halogenated C₁-C₆ alkyl.

In some embodiments, R³ is selected from the group consisting of C₁-C₆ alkyl, and fluoro C₁-C₆ alkyl.

In some embodiments, R³ is selected from the group consisting of methyl, ethyl, and -CHF₂.

In some embodiments, R⁴ is selected from the group consisting of hydrogen, and C₁-C₆ alkyl.

In some embodiments, R⁴ is selected from the group consisting of hydrogen.

In some embodiments, R⁵-R²⁶ are all selected from the group consisting of hydrogen atom, or at least one hydrogen atom is replaced by deuterium (D).

In some embodiments, R⁵-R²⁶ are all selected from the group consisting of hydrogen atom.

In some embodiments, at least one of R⁵-R²⁶ is deuterium (D).

In a particularly preferred aspect, the present invention relates to a compound represented by formula (I), an isomer, a pharmaceutically acceptable salt, a precursor or a metabolite thereof, wherein,
R¹ and R² can be the same or different, and are selected from the group consisting of methyl, methyl-*d₃* (CD₃), cyano, fluorine and chlorine;
R³ is selected from the group consisting of C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl, wherein at least one hydrogen atom in these groups is replaced by deuterium (D);
R⁴ is selected from the group consisting of hydrogen;
X is selected from the group consisting of -O-;
R⁵-R²⁷ are selected from the group consisting of hydrogen atom, or selected from the group consisting of hydrogen atom wherein at least one hydrogen atom is replaced by deuterium (D);
R²⁸-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R¹ is selected from the group consisting of methyl, cyano, fluorine, and chlorine.

In some embodiments, R² is selected from the group consisting of methyl and chlorine.

In some embodiments, R³ is selected from the group consisting of C₁-C₆ alkyl, and halogenated C₁-C₆ alkyl, wherein at least one hydrogen atom in these substituents is replaced by deuterium (D).

In some embodiments, R³ is selected from the group consisting of C₁-C₆ alkyl, and fluoro C₁-C₆ alkyl, wherein at least one hydrogen atom in these substituents is replaced by deuterium (D).

In some embodiments, R³ is selected from the group consisting of methyl, ethyl, and -CHF₂, wherein at least one hydrogen atom in these substituents is replaced by deuterium (D).

In some embodiments, R⁵-R²⁶ are selected from the group consisting of hydrogen atom, or selected from the group consisting of hydrogen atom wherein at least one hydrogen atom is replaced by deuterium (D).

In some embodiments, R⁵-R²⁶ are selected from the group consisting of hydrogen atom.

In some embodiments, R⁵-R²⁶ are selected from the group consisting of hydrogen atom, wherein at least one hydrogen atom is replaced by deuterium (D).

In some embodiments, R²⁷-R³⁰ are selected from the group consisting of hydrogen atom, or selected from the group consisting of hydrogen atom wherein at least one hydrogen atom is replaced by deuterium (D), wherein the deuterium can be at any position on the corresponding alicyclic ring and aliphatic heterocyclic ring.

In some embodiments, R²⁷-R³⁰ are selected from the group consisting of hydrogen atom.

In some embodiments, R²⁷-R³⁰ are selected from the group consisting of hydrogen atom, wherein at least one hydrogen atom is replaced by deuterium (D), wherein the deuterium substitution position can be at any position on the corresponding alicyclic ring and aliphatic heterocyclic ring.

In some embodiments, R¹ and R² are the same or different, and are selected from the group consisting of methyl, cyano, and halogen;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl, wherein at least one hydrogen atom in these groups is replaced by deuterium (D);
R⁴ is selected from the group consisting of hydrogen and C₁-C₆ alkyl;
X is selected from the group consisting of -O-;
R⁵-R²⁶ are selected from the group consisting of hydrogen atom;
R²⁷-R³⁰ are selected from the group consisting of hydrogen atom.

In some embodiments, R¹ and R² are the same or different, and are selected from the group consisting of methyl, cyano, fluorine, and chlorine.

In some embodiments, R¹ and R² are the same or different, and are selected from the group consisting of methyl, fluorine, and chlorine;

In some embodiments, R¹ and R² are the same or different, and are selected from the group consisting of methyl, and chlorine.

In some embodiments, R³ is selected from the group consisting of hydrogen, C₁-C₆ alkyl, and halogenated C₁-C₆ alkyl, wherein at least one hydrogen atom in these groups is replaced by deuterium (D).

In some embodiments, R³ is selected from the group consisting of C₁-C₆ alkyl, wherein at least one hydrogen atom is replaced by deuterium (D).

In some embodiments, R³ is selected from the group consisting of methyl, ethyl, propyl, butyl, and pentyl, wherein at least one hydrogen atom is replaced by deuterium (D).

In some embodiments, 1-15 hydrogen atoms in the group defined by R³ are replaced by deuterium (D).

In some embodiments, 1-9 hydrogen atoms in the group defined by R³ are replaced by deuterium (D).

In some embodiments, R⁴ is selected from the group consisting of hydrogen, methyl, ethyl, propyl, butyl, and pentyl.

In some embodiments, R⁴ is selected from the group consisting of hydrogen.

The present invention relates to a compound represented by formula (I), which are preferably selected from the group consisting of the following compounds:

| **Structural formula of compound** | **Name of compound** |
|---|---|
| 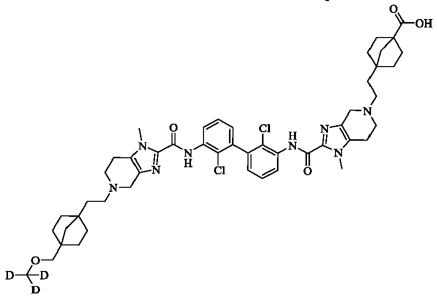 | 4-(2-(2-((2,2'-dichloro-3'-(5-(2-(4-((methoxy-*d₃* )methyl)bicyclo[2.2.1]heptane-1-yl)ethyl)-1-me thyl-4,5,6,7-tetrahydro-1H-imidazo[4,5-c]pyridine-2-carboxamido)-[1,1'-biphenyl]-3-yl)carba moyl)-1-methyl-1,4,6,7-tetrahydro-5H-imidazo[4,5-c]pyridin-5-yl)ethyl)bicyclo[2.2.1]heptane-1-carboxylic acid |
| 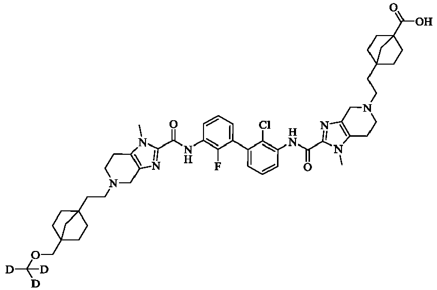 | 4-(2-(2-((2-chloro-2'-fluoro-3'-(5-(2-(4-((methoxy-*d₃*)methyl)blcyclo[2.2.1]heptane-1-yl)ethyl)-1-methyl-4,5,6,7-tetrahydro-1H-imidazo[4,5-c ]pyridine-2-carboxamido)-[1,1'-biphenyl]-3 -yl)carbamoyl)-1-methyl-1,4,6,7-tetrahydro-5H-imidazo[4,5-c]pyridin-5-yl)ethyl)bicyclo[2.2.1]heptane-1-carboxylic acid |
| 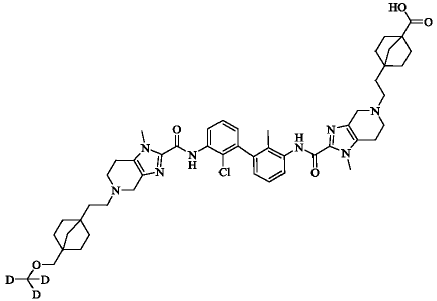 | 4-(2-(2-((2'-chloro-3'-(5-(2-(4-((methoxy-*d₃*)me thyl)bicyclo[2.2.1]heptane-1-yl)ethyl)-1-methyl-4,5,6,7-tetrahydro-1H-imidazo[4,5-c]pyridine-2-carboxamido)-2-methyl-[1,1'-biphenyl]-3 -yl) carbamoyl)-1-methyl-1,4,6,7-tetrahydro-5H-imi dazo[4,5-c]pyridin-5-yl)ethyl)bicyclo[2.2.1]hep tane-1-carboxylic acid |
| 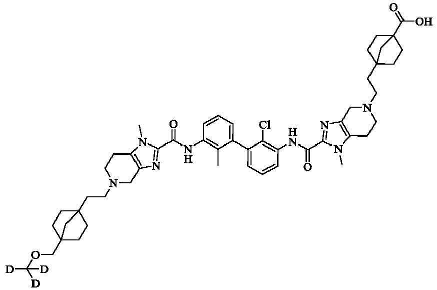 | 4-(2-(2-((2-chloro-3'-(5-(2-(4-((methoxy-*d₃*)met hyl)bicyclo[2.2.1]heptane-1-yl)ethyl)-1-methyl-4,5,6,7-tetrahydro-1H-imidazo[4,5-c]pyridine-2-carboxamido)-2'-methyl-[1,1'-biphenyl]-3-yl)carbamoyl)-1-methyl-1,4,6,7-tetrahydro-5H-imidazo[4,5-c]pyridin-5-yl)ethyl)bicyclo[2.2.1]hep tane-1-carboxylic acid |
| 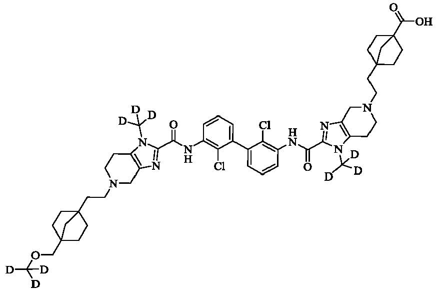 | 4-(2-(2-((2,2'-dichloro-3'-(5-(2-(4-((methoxy-*d₃* )methyl)bicyclo[2.2.1]heptane-1-yl)ethyl)-1-(methyl-*d₃*)-4,5,6,7-tetrahydro-1H-imidazo[4,5-c]pyridine-2-carboxamido)-[1,1'-biphenyl]-3-yl)carbamoyl)-1-(methyl-*d₃*)-1,4,6,7-tetrahydro-5H-imidazo[4,5-c]pyridin-5-yl)ethyl)bicyclo[2.2.1]heptane-1-carboxylic acid |
| 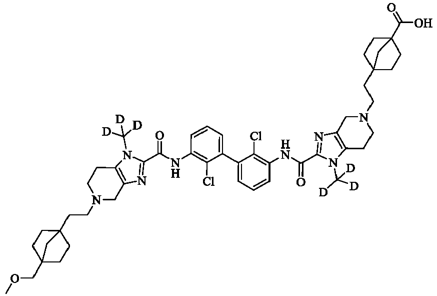 | 4-(2-(2-((2,2'-dichloro-3'-(5-(2-(4-(methoxymethyl)bicyclo[2.2.1]heptane-1-yl)ethyl)-1-(methyl*-d₃*)-4,5,6,7-tetrahydro-1H-imidazo[4,5-c]pyridine-2-carboxamido)-[1,1'-biphenyl]-3-yl)carbamoyl)-1-(methyl*-d₃*)-1,4,6,7-tetrahydro-5H-imidazo[4,5-c]pyridin-5-yl)ethyl)bicyclo[2.2.1]heptane-1-carboxylic acid |
| 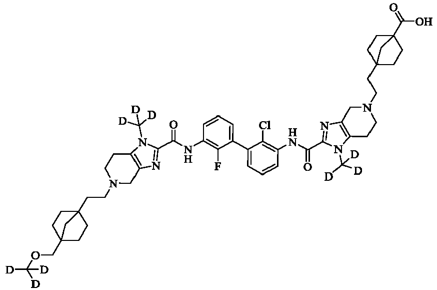 | 4-(2-(2-((2-chloro-2'-fluoro-3'-(5-(2-(4-((methoxy-*d₃*)methyl)blcyclo[2.2.1]heptane-1 -yl)ethyl)-1-(methyl-*d₃*)-4,5,6,7-tetrahydro-1H-imidazo[4,5-c]pyridine-2-carboxamido)-[1,1'-biphenyl]-3-yl)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid |
| 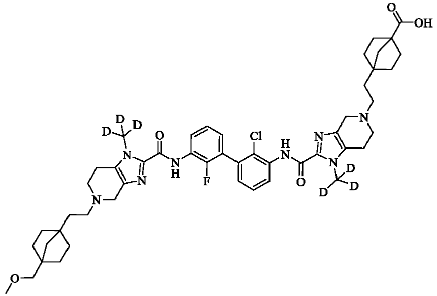 | 4-(2-(2-((2-chloro-2'-fluoro-3'-(5-(2-(4-(methoxymethyl)bicyclo[2.2.1]heptane-1-yl)ethyl)-1-(methyl-*d₃*)-4,5,6,7-tetrahydro-1H-imidazo[4,5-c]pyridine-2-carboxamido)-[1,1'-biphenyl]-3-yl)carbamoyl)-1-(methyl-*d₃*)-1,4,6,7-tetrahydro-5H-imidazo[4,5-c]pyridin-5-yl)ethyl)bicyclo[22.1]heptane-1-carboxylic acid |
| 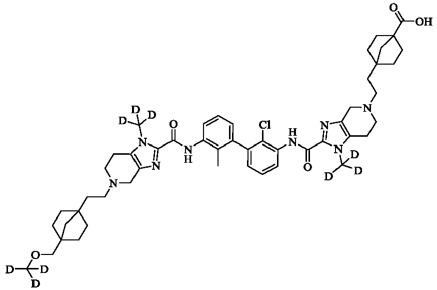 | 4-(2-(2-((2-chloro-3'-(5-(2-(4-((methoxy-*d₃*)methyl)bicyclo[2.2.1]heptane-1-yl)ethyl)-1-(methyl*-d₃*)-4,5,6,7-tetrahydro-1H-imidazo[4,5-c]pyridine-2-carboxamido)-2'-methyl-[1,1'-biphenyl]-3-yl)carbamoyl)-1-(methyl-*d₃*)-1,4,6,7-tetrahydro-5H-imidazo[4,5-c]pyridin-5-yl)ethyl)bicyclo[2.2.1]heptane-1-carboxylic acid |
| 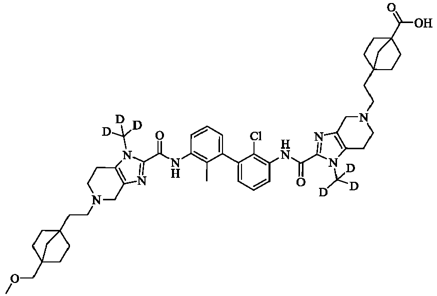 | 4-(2-(2-((2-chloro-3'-(5-(2-(4-(methoxymethyl) bicyclo[2.2.1]heptane-1-yl)ethyl)-1-(methyl-*d₃*) -4,5,6,7-tetrahydro-1H-imidazo[4,5-c]pyridine-2-carboxamido)-2'-methyl-[1,1'-biphenyl]-3-yl)carbamoyl)-1-(methyl-*d₃*)-1,4,6,7-tetrahydro-5H-imidazo [4,5 -c]pyridin-5-yl)ethyl)bicyclo[2.2.1]heptane-1-carboxylic acid |
| 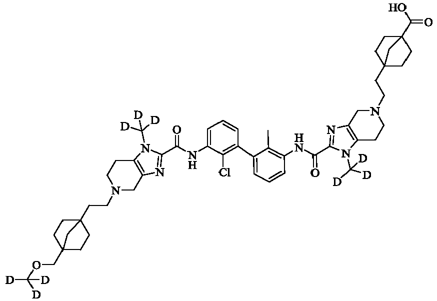 | 4-(2-(2-((2'-chloro-3'-(5-(2-(4-((methoxy-*d₃*)me thyl)bicyclo[2.2.1]heptane-1-yl)ethyl)-1-(methyl-*d₃*)-4,5,6,7-tetrahydro-1H-imidazo[4,5-c]pyrid ine-2-carboxamido)-2-methyl-[1,1'-biphenyl]-3-yl)carbamoyl)-1-(methyl-*d₃*)-1,4,6,7-tetrahydro-5H-imidazo[4,5-c]pyridin-5-yl)ethyl)bicyclo[2.2.1]heptane-1-carboxylic acid |
| 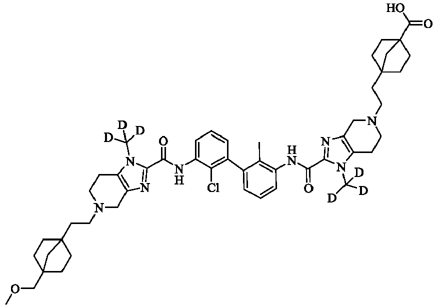 | 4-(2-(2-((2'-chloro-3'-(5-(2-(4-(methoxymethyl)bicyclo[2.2.1]heptane-1-yl)ethyl)-1-(methyl-*d₃*)-4,5,6,7-tetrahydro-1H-imidazo[4,5 -c]pyridine-2-carboxamido)-2-methyl-[1,1'-biphenyl]-3-yl) carbamoyl)-1-(methyl-*d₃*)-1,4,6,7-tetrahydro-5H-imidazo[4,5 -c]pyridin-5-yl)ethyl)bicyclo [2.2.1]heptane-1-carboxylic acid |
| 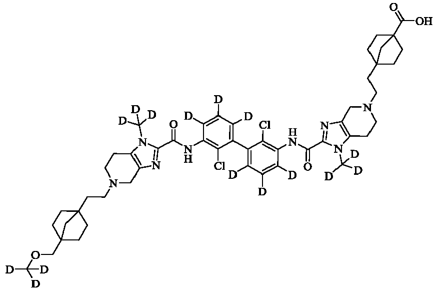 | 4-(2-(2-((2,2'-dichloro-3'-(5-(2-(4-((methoxy-*d₃*)methyl)bicyclo[2.2.1]heptane-1-yl)ethyl)-1-(methyl-*d₃*)-4,5,6,7-tetrahydro-1H-imidazo[4,5-c]pyridine-2-carboxamido)-[1,1'-biphenyl]-3-yl-4,4',5,5',6,6'-*d₆*)carbamoyl)-1-(methyl-*d₃*)-1,4,6,7-tetrahydro-5H-imidazo[4,5-c]pyridin-5-yl)ethyl)bicyclo[2.2.1]heptane-1-carboxylic acid |
| 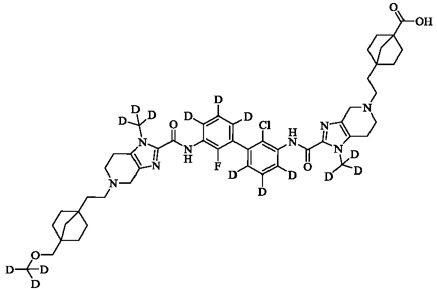 | 4-(2-(2-((2-chloro-2'-fluoro-3'-(5-(2-(4-((methoxy-*d₃*)methyl)blcyclo[2.2.1]heptane-1-yl)ethyl)-1-(methyl*-d₃*)-4,5,6,7-tetrahydro-1H-imidazo[4,5-c]pyridine-2-carboxamido)-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid |
| 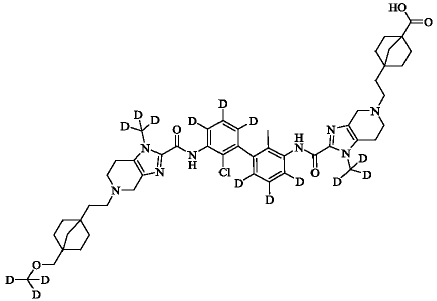 | 4-(2-(2-((2'-chloro-3'-(5-(2-(4-((methoxy-*d₃*)me thyl)bicyclo[2.2.1]heptane-1-yl)ethyl)-1-(methyl-*d*₃)-4,5,6,7-tetrahydro-1H-imidazo[4,5-c]pyridine-2-carboxamido)-2-methyl-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid |
| 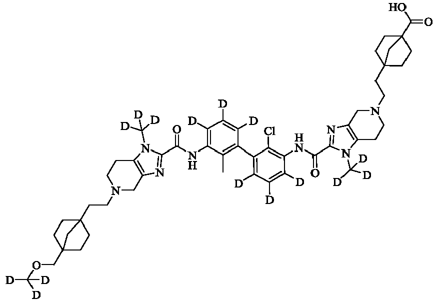 | 4-(2-(2-((2-chloro-3'-(5-(2-(4-((methoxy-*d₃*)methyl)bicyclo[2.2.1]heptane-1-yl)ethyl)-1-(methyl*-d₃*)-4,5,6,7-tetrahydro-1H-imidazo[4,5-c]pyridine-2-carboxamido)-2'-methyl-[1,1'-biphenyl]-3-yl-4,4',5,5',6,6'-d6)carbamoyl)-1-(methyl-*d*₃)-1,4,6,7-tetrahydro-5H-imidazo[4,5-c]pyridin-5-yl )ethyl)bicyclo[2.2.1]heptane-1-carboxylic acid |
| 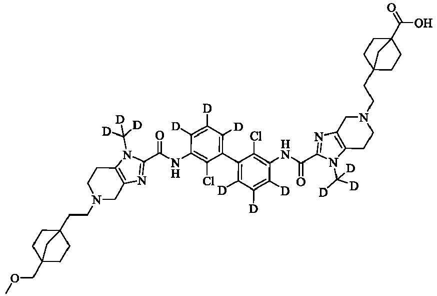 | 4-(2-(2-((2,2'-dichloro-3'-(5-(2-(4-(methoxymethyl)bicyclo[2.2.1]heptane-1-yl)ethyl)-1-(methyl*-d₃*)-4,5,6,7-tetrahydro-1H-imidazo[4,5-c]pyridine-2-carboxamido)-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[22.1]heptane-1-carboxylic acid |
| 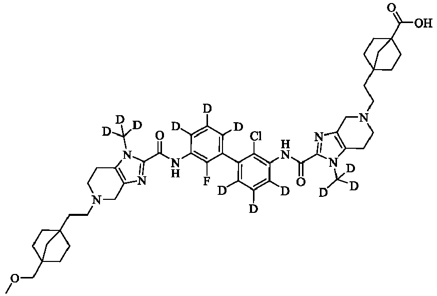 | 4-(2-(2-((2-chloro-2'-fluoro-3'-(5-(2-(4-(methoxymethyl)bicyclo[2.2.1]heptane-1-yl)ethyl)-1-(methyl-*d₃*)-4,5,6,7-tetrahydro-1H-imidazo[4,5-c]pyridine-2-carboxamido)-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5 -yl)ethyl)bicyclo[2.2.1]heptane-1-carboxylic acid |
| 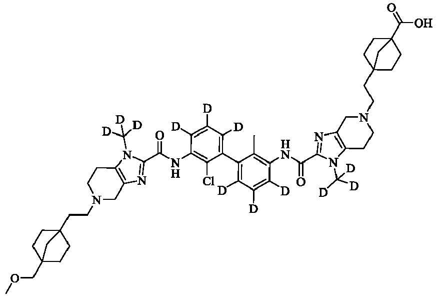 | 4-(2-(2-((2'-chloro-3'-(5-(2-(4-(methoxymethyl)bicyclo[2.2.1]heptane-1-yl)ethyl)-1-(methyl-*d₃*)-4,5,6,7-tetrahydro-1H-imidazo[4,5-c]pyridine-2-carboxamido)-2-methyl-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid |
| 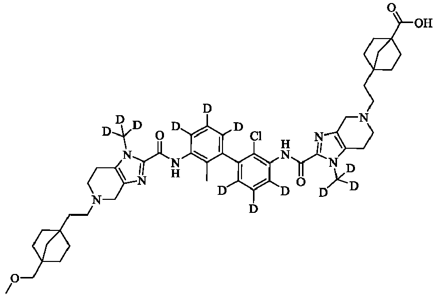 | 4-(2-(2-((2-chloro-3'-(5-(2-(4-(methoxymethyl)bicyclo[2.2.1]heptane-1-yl)ethyl)-1-(methyl-*d*₃)-4,5,6,7-tetrahydro-1H-imidazo[4,5-c]pyridine-2-carboxamido)-2'-methyl-[1,1'-biphenyl]-3-yl-4,4',5,5',6,6'-*d*₆)carbamoyl)-1-(methyl-*d*₃)-1, 4,6,7-tetrahydro-5H-imidazo[4,5-c]pyridin-5-yl)ethyl)bicyclo[2.2.1]heptane-1-carboxylic acid |
| 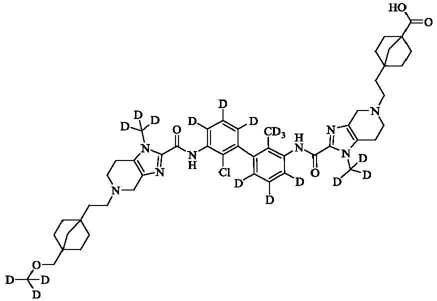 | 4-(2-(2-((2'-chloro-3'-(5-(2-(4-((methoxy-*d₃*)methyl)bicyclo[2.2.1]heptane-1-yl)ethyl)-1-(methyl*-d*₃)-4,5,6,7-tetrahydro-1H-imidazo[4,5-c]pyridine-2-carboxamido)-2-(methyl-*d*₃)-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid |
| 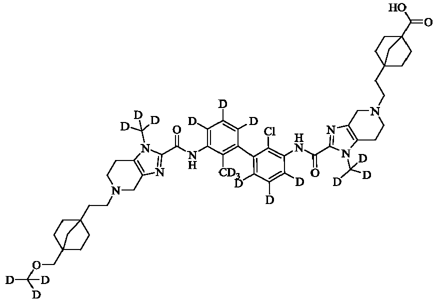 | 4-(2-(2-((2-chloro-3'-(5-(2-(4-((methoxy-*d₃*)methyl)bicyclo[2.2.1]heptane-1-yl)ethyl)-1-(methyl*-d₃*)-4,5,6,7-tetrahydro-1H-imidazo[4,5-c]pyridine-2-carboxamido)-2'-(methyl-*d*₃)-[1,1'-biphen yl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid |
| 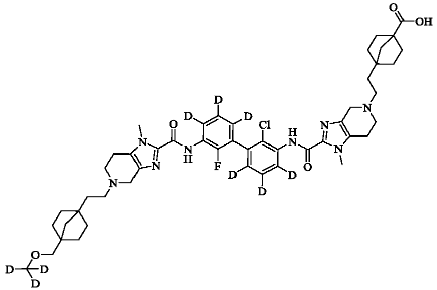 | 4-(2-(2-((2-chloro-2'-fluoro-3'-(5-(2-(4-((methoxy-*d₃*)methyl)blcyclo[2.2.1]heptane-1-yl)ethyl)-1-methyl-4,5,6,7-tetrahydro-1H-imidazo[4,5-c]pyridine-2-carboxamido)-[1,1'-biphenyl]-3-yl-4,4',5,5',6,6'-*d*₆)carbamoyl)-1-methyl-1,4,6,7-tetrahydro-5H-imidazo[4,5-c]pyridin-5-yl)ethyl)bicyclo[2.2.1]heptane-1-carboxylic acid |
| 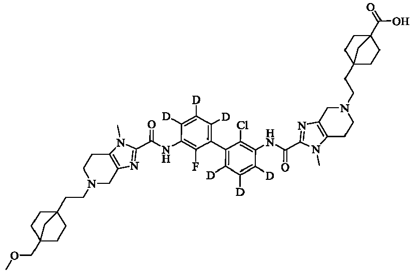 | 4-(2-(2-((2-chloro-2'-fluoro-3'-(5-(2-(4-(methoxymethyl)bicyclo[2.2.1]heptane-1-yl)ethyl)-1-methyl-4,5,6,7-tetrahydro-1H-imidazo[4,5-c]pyridine-2-carboxamido)-[1,1'-biphenyl]-3-yl-4,4',5,5',6,6'-*d*₆)carbamoyl)-1-methyl-1,4,6,7-tetrahydro-5H-imidazo[4,5-c]pyridin-5-yl)ethyl)bicyclo[2.2.1]heptane-1-carboxylic acid |

### Definition of term

In each part of this specification, the substituents of the compounds disclosed in the present invention are disclosed according to the type or range of the groups. It should be specifically noted that the present invention includes each individual subcombination of individual members of these group classes and ranges. For example, the term "C₁-C₆ alkyl" specifically refers to independently disclosed methyl, ethyl, C₃ alkyl, C₄ alkyl, C₅ alkyl and C₆ alkyl, or independently disclosed "C₁-C₄ alkyl", or independently disclosed "C₁-C₃ alkyl".

The "halogen" in the present invention refers to fluorine, chlorine, bromine or iodine, preferably fluorine or chlorine or bromine.

The "alkyl" mentioned in the present invention includes linear or branched chain alkyl. The C₁-C₆ alkyl mentioned in the present invention refers to alkyl with 1-6 carbon atoms, preferably methyl, ethyl, n-propyl or isopropyl, n-butyl, isobutyl or tert-butyl. The alkyl in the compounds of the present invention may be optionally substituted or unsubstituted, and the substituents for substitution may include alkyl, halogen, alkoxy, haloalkyl, cyano, hydroxyl, and the like. Examples of the alkyl of the present invention include methyl, ethyl, n-propyl, isopropyl, n-butyl, isobutyl, tert-butyl and the like.

The "alkenyl" in the present invention refers to a linear or branched monovalent hydrocarbon group with 2-12 carbon atoms, or 2-8 carbon atoms, or 2-6 carbon atoms, or 2-4 carbon atoms, wherein at least one position is unsaturated, that is, one C-C is a sp² double bond, wherein the alkenyl can be independently and optionally substituted by one or more substituents described in the present invention, including groups having "trans ", "cis" or "E", "Z" positions, wherein alkenyl can be C₂-C₆ alkenyl, and specific examples include, but are not limited to, vinyl (-CH=CH₂), allyl (-CH₂CH=CH₂) and so on.

The "alkynyl" in the present invention refers to a linear or branched monovalent hydrocarbon group with 2-12 carbon atoms, or 2-8 carbon atoms, or 2-6 carbon atoms, or 2-4 carbon atoms, wherein at least one position is unsaturated, that is, one C-C is a sp triple bond, wherein the alkynyl can be independently and optionally substituted by one or more substituents described in the present invention, wherein the alkynyl can be C₂-C₆ alkynyl, and specific examples include, but are not limited to, ethynyl (-C=CH), propargyl (-CH₂C=CH) and the like.

The "alkoxy" in the present invention refers to the group formed by connecting the above alkyl with an oxygen atom, wherein the oxygen atom has the ability to form bonds freely, such as "C₁-C₆ alkoxy", particularly such as methoxy, ethoxy, n-propoxy, n-butoxy, isopropoxy, tert-butoxy, and cyclopropoxy.

The "alkylamino" mentioned in the present invention refers to a group formed by connecting the above alkyl with amino, such as "C₁-C₆ alkylamino", particularly such as methylamino, ethylamino, dimethylamino, and methylisopropylamino.

The "halogenated C₁-C₆ alkyl" and "halogenated C₁-C₆ alkoxy" in the present invention mean that one or more hydrogen atoms in the alkyl and alkoxy are replaced by halogen atoms, particularly replaced by fluorine or chlorine atom. In some embodiments, one or more hydrogen atoms in the alkyl and alkoxy are preferably replaced by fluorine, such as -CF₃, -CHF₂, -CH₂F, -CH₂CH₂F, -CH₂CHF₂, -CH₂CF₃, -OCF₃, -OCHF₂, -OCH₂F, -OCH₂CH₂F, -OCH₂CHF₂ or -OCH₂CF₃.

The "cycloalkyl" in the present invention refers to a hydrocarbon group with monocyclic structure having a specified number of ring carbon atoms, which does not contain unsaturated bonds such as double bond, including C₃-C₁₄ cycloalkyl, C₃-C₆ cycloalkyl, such as, cyclopropyl, cyclobutyl, cyclopentyl, or cyclohexyl. The cycloalkyl in the compounds of the present invention may be optionally substituted or unsubstituted, and the substituents may include alkyl, halogen, alkoxy, hydrocarbyl, hydroxyl and the like.

The "heterocycle" mentioned in the present invention refers to an unsubstituted or substituted monocyclic or polycyclic non-aromatic ring system containing one or more heteroatoms, which is a partially unsaturated or fully saturated ring system. Preferred heteroatoms are selected from the group consisting of N, O and S. Monocyclic heterocycles include, but are not limited to, pyrrolidinyl, imidazolidinyl, tetrahydrofuryl, dihydroimidazolyl, dihydrofuryl, piperidinyl, morpholinyl, thiomorpholinyl, and homopiperazinyl. Polycyclic heterocyclyls are selected from the group consisting of spiro rings, bridged rings, heterocyclyl of fused rings, which may be fused to aryl, heteroaryl or cycloalkyl rings.

From all the above descriptions, it will be apparent to those skilled in the art that any group whose name is a combined name, such as "C₂-C₆ alkenyl C₁-C₆ alkyl-", shall refer to constructing from the moiety which is conventionally derived therefrom, for example, constructing from C₁-C₆ alkyl substituted by C₂-C₆ alkenyl, wherein C₂-C₆ alkenyl and C₁-C₆ alkyl are as defined above. Other similar combined names such as "C₂-C₆ alkynyl C₁-C₆ alkyl-" and "C₃-C₁₄ cycloalkyl-C₁-C₄ alkyl-" can be understood with reference to the foregoing.

Unless otherwise specifically defined, "substituted" as described herein means that one or more hydrogen atoms in a group can be independently replaced by a corresponding number of substituents. Those skilled in the art are able to determine (by experiment or theory) possible or impossible substitution positions without making undue effort. The substituents referred to in the "substituted" include but are not limited to: cyano, carboxyl, halogen, hydroxyl, C₁-C₆ alkyl, C₂-C₆ alkenyl, C₂-C₆ alkynyl, C₁-C₆ alkoxy, mono-C₁-C₆ alkylamino C₁-C₆ alkyl-, bis-C₁-C₆ alkylamino C₁-C₆ alkyl-, halogenated C₁-C₆ alkyl, C₁-C₆ alkoxy C₁-C₆ alkyl-, halogenated C₁-C₆ alkoxy C₁-C₆ alkyl-, C₃-C₁₄ cycloalkyl, 3-14-membered heterocycloalkyl, C₃-C₁₄ cycloalkyl-C₁-C₄ alkyl-, 3-14-membered heterocycloalkyl-C₁-C₄ alkyl-.

For stereoisomers in the compounds described herein, when they are specifically designated as (R)- or (S)-isomers by chemical names, it should be understood that their predominant configuration is the (R)-isomer or ( S)-isomer. Any asymmetric carbon atom may be present in the (R)-, (S)- or (R,S)-configuration, preferably in the (R)- or (S)-configuration.

The "pharmaceutically acceptable salt" in the present invention refers to the acid addition salt prepared by reacting the compound of the present invention with a pharmaceutically acceptable acid, or the salt formed by reacting a compound with an acidic group with a basic compound. Wherein, the acid is preferably selected from the group consisting of inorganic acids (such as hydrochloric acid, sulfuric acid, phosphoric acid or hydrobromic acid), and organic acids (such as oxalic acid, maleic acid, fumaric acid, malic acid, tartaric acid, lysine, histidine, citric acid or benzoic acid). The basic compound is preferably selected from the group consisting of sodium hydroxide, potassium hydroxide, calcium hydroxide, sodium carbonate and potassium bicarbonate. The above pharmaceutically acceptable salts are easy to be separated and can be purified by conventional separation methods, such as solvent extraction, dilution, recrystallization, column chromatography and preparative thin-layer chromatography.

Unless otherwise specified, the "hydrogen atom" described herein generally refers to the isotopic form of hydrogen with a mass number of 1, i.e. ¹H.

Another aspect of the present invention further relates to a pharmaceutical composition, comprising the above compound, or isomer, pharmaceutically acceptable salt, precursor or metabolite thereof as an active ingredient.

The compounds described in the present invention can optionally be used in combination with one or more other active ingredients, and their respective amounts and ratios can be adjusted by those skilled in the art according to specific diseases, specific conditions of patients, and clinical needs.

The compounds represented by the general formula (I) in the present invention, isomers, pharmaceutically acceptable salts, precursors or metabolites thereof can be prepared by those skilled in the art (through experience or reference).

When the structural formula of the compound represented by the general formula (I) in the present invention does not match the English name, the structural formula shall prevail.

Therefore, another aspect of the present invention further provides a method for preparing the above compounds according to the present invention.

The following synthetic route describes the method for preparing the compounds represented by formula (I) of the present invention. The raw materials, reagents, catalysts, solvents, etc. used in the following synthetic scheme can be prepared by methods well known to those of ordinary skill in the field of organic chemistry or commercially available. All final derivatives of the present invention can be prepared by the methods described in the schemes or similar methods, which are well known to those of ordinary skill in the field of organic chemistry. All variables used in these schemes are as defined in the context.

### Preparation method

The following variables are defined as previously described, and new variables are defined as described in this section. In addition, the compounds represented by general formula (I) and related intermediates can be purified by common separation methods, such as extraction, recrystallization and silica gel column chromatography separation. The 200-300 mesh silica gel and thin-layer chromatography silica gel plates used are all produced by Qingdao Haiyang Huagong Factory. The chemical reagents used are analytically pure or chemically pure commercially available products, and are used without further purification.

The present invention provides a method for preparing a compound represented by general formula (I), comprising the following steps: 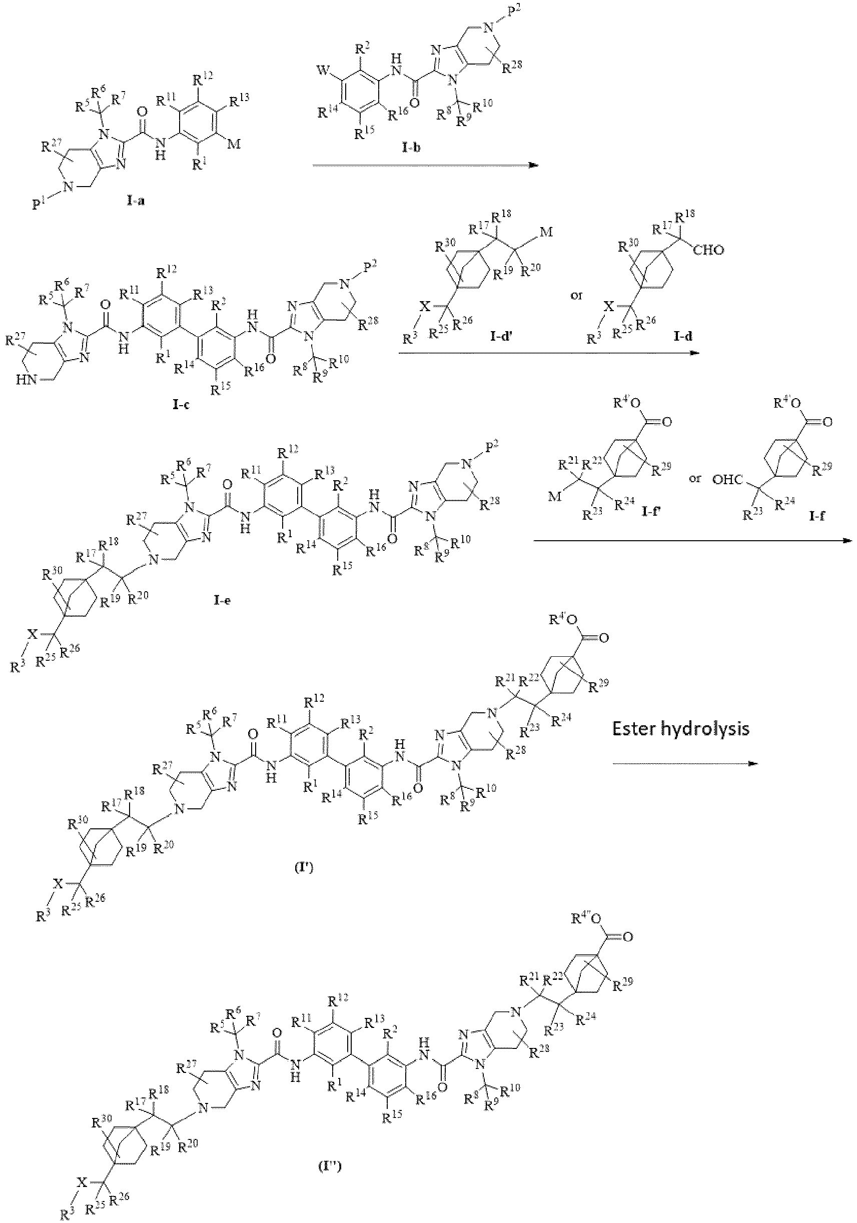
1) subjecting a compound represented by formula (I-a) to a first acid, a first base or catalytic hydrogenolysis in a first solvent to remove the protecting group P¹, further subjecting the obtained product, without separation and purification, to a Suzuki reaction with a compound represented by formula (I-b) in a second solvent and in the presence of a first catalyst and a second base to obtain a compound represented by formula (I-c);
2) subjecting the compound represented by formula (I-c) and a compound represented by formula (I-d) to a reductive amination reaction in a third solvent and in the presence of a first reducing agent to obtain a compound represented by formula (I-e); or,
   subjecting the compound represented by formula (I-c) and a compound represented by formula (I-d') to a nucleophilic substitution reaction in a fourth solvent and in the presence of a third base to obtain a compound represented by formula (I-e);
3) subjecting the compound represented by formula (I-e) and a compound represented by formula (I-f) to a reductive amination reaction in a fifth solvent and in the presence of a second reducing agent to obtain a compound represented by formula (I); or,
subjecting the compound represented by formula (I-e) and a compound represented by formula (I-f') to a nucleophilic substitution reaction in a sixth solvent and in the presence of a fourth base to obtain a compound represented by formula (I).

Optionally, the method further comprises 4) subjecting a compound represented by formula (I') to ester hydrolysis in the presence of a fifth base to obtain a compound represented by formula (I").

As mentioned above, the compound represented by the formula (I') serves as a form of the final product (I). In addition, the compound represented by the formula (I') can be further subjected to ester hydrolysis under basic conditions to obtain a compound represented by the formula (I"), and the compound represented by the formula (I") is also a form of the final product (I).
wherein:
the definitions of X and R¹-R³⁰ are as described above;
R^{4'} has the same definition as R⁴ except that it is not hydrogen, and R⁴ is as defined above;
R^{4"} is hydrogen;
M is selected from the group consisting of borate and boric acid, including but not limited to 4,4,5,5-tetramethyl-1,3,2-dioxaborolane, bis(neopentyl glycolato)diboron, 4,4,4',4',5,5,5',5'-octamethyl-2, 2'-bis(1, 3, 2-dioxaborolane) B(OBu-*n*)₃, B (OPr-*i*)₃; or,
M is selected from the group consisting of bromine, iodine, chlorine, fluorine, and CF₃SO₃-(OTf);
W is selected from the group consisting of borate and boric acid, preferably selected from the group consisting of 4,4,5,5-tetramethyl-1,3,2-dioxaborolane, bis(neopentyl glycolato)diboron, 4,4,4',4',5,5,5',5'-octamethyl-2, 2'-bis(1, 3, 2-dioxaborolane) B(OBu-*n*)₃, and B (OPr-*i*)₃; or,
W is selected from the group consisting of bromine, iodine, chlorine, fluorine, and CF₃SO₃-(OTf);
P¹ and P² are protecting groups, which can be the same or different, preferably selected from the group consisting of Boc (tert-butoxycarbonyl), Fmoc (9-fluorenylmethoxycarbonyl), Cbz (N-benzyloxycarbonyl), methanesulfonyl, p-toluenesulfonyl, acetyl, methoxycarbonyl, ethoxycarbonyl, ((2-trimethylsilyl)ethoxy)methyl (SEM), and tetrahydro-2H-pyran-2-yl (THP).

In some embodiments, the first acid is preferably selected from the group consisting of trifluoroacetic acid (TFA), hydrochloric acid (HCl), acetic acid (HOAc), and hydrobromic acid (HBr);
the first base is preferably selected from the group consisting of piperidine and diethylamine;
the first solvent is preferably selected from the group consisting of dichloromethane (DCM), 1,2-dichloroethane, methanol (MeOH), ethanol (EtOH), 1,4-dioxane, tetrahydrofuran (THF), acetonitrile (MeCN), and N, N'-dimethylformamide (DMF).

In some embodiments, the first catalyst is preferably selected from the group consisting of 1,1'-bis(dicyclohexylphosphino)ferrocenepalladium dichloride (PdCl₂(dcypf)), palladium acetate (Pd(OAc)₂), palladium dichloride (PdCl₂), tris(dibenzylideneacetone) dipalladium (Pd₂(dba)₃), [1,1'-bis(diphenylphosphino)ferrocene]palladium dichloride (PdCl₂(dppf)), a complex of [1,1'-bis(diphenylphosphine)ferrocene]palladium dichloride and dichloromethane (PdCl₂(dppf)·CH₂Cl₂), tetrakis(triphenylphosphine)palladium (Pd(PPh₃)₄), bis(tricyclohexylphosphine)palladium dichloride (PdCl₂(P(Cy)₃)₂), 2-dicyclohexylphosphine-2', 6'-dimethoxybiphenyl (SPhos);
the second base is selected from the group consisting of organic base and inorganic base, such as triethylamine (TEA), N,N-diisopropylethylamine (DIPEA), n-butyllithium, lithium diisopropylamide, lithium bis(trimethylsilyl)amide, potassium acetate (KOAc), sodium tert-butoxide (NaOBu-*t*), potassium tert-butoxide (KOBu-*t*), sodium hydride (NaH), potassium phosphate (K₃PO₄), sodium carbonate (Na₂CO₃), potassium carbonate (K₂CO₃), lithium hydroxide (KOH), and sodium hydroxide (NaOH);
the second solvent is preferably selected from the group consisting of 1,4-dioxane, tetrahydrofuran (THF), acetonitrile (MeCN), N,N'-dimethylformamide (DMF), and mixed solvents of these solvents with water in different ratios.

In some embodiments, the first reducing agent and the second reducing agent are preferably selected from the group consisting of sodium acetate borohydride, sodium borohydride, and sodium cyanoborohydride;
the third solvent and fifth solvent are preferably selected from the group consisting of dichloromethane (DCM), 1,2-dichloroethane, methanol (MeOH), ethanol (EtOH), 1,4-dioxane, tetrahydrofuran (THF), acetonitrile (MeCN), and N,N'-dimethylformamide (DMF).

In some embodiments, the fourth solvent and sixth solvent are preferably selected from the group consisting of dichloromethane (DCM), 1,2-dichloroethane, methanol (MeOH), ethanol (EtOH), 1,4-dioxane, tetrahydrofuran (THF), acetonitrile (MeCN), N,N'-dimethylformamide (DMF), N-methylpyrrolidone (NMP), and pyridine (Py);
the third base and the fourth base are preferably selected from the group consisting of triethylamine (TEA), N,N'-diisopropylethylamine (DIPEA), pyridine (Py), n-butyl lithium, lithium diisopropylamide, lithium bis(trimethylsilyl)amide, sodium tert-butoxide (NaOBu-*t*), potassium tert-butoxide (KOBu-*t*), sodium hydride (NaH), sodium carbonate (Na₂CO₃), potassium carbonate (K₂CO₃), lithium hydroxide (KOH), and sodium hydroxide (NaOH).

In some embodiments, the fifth base is preferably selected from the group consisting of lithium hydroxide (LiOH), lithium hydroxide (KOH), and sodium hydroxide (NaOH).

Particularly, the compound represented by formula (I-a) is subjected to appropriate acid, base or catalytic hydrogenolysis in an appropriate solvent to remove the protecting group P¹. The obtained product is further subjected, without separation and purification, to a Suzuki reaction with a compound represented by formula (I-b) in an appropriate basic condition and solvent under a catalyst to obtain a compound represented by formula (I-c). The appropreiate solvent for deprotection is preferably selected from the group consisting of dichloromethane (DCM), 1, 2-dichloroethane, methanol (MeOH), ethanol (EtOH), 1,4-dioxane, tetrahydrofuran (THF), acetonitrile (MeCN), and N,N'-dimethylformamide (DMF). The acid is preferably selected from the group consisting of trifluoroacetic acid (TFA), hydrochloric acid (HCl), acetic acid (HOAc), and hydrobromic acid (HBr). The base is preferably selected from the group consisting of piperidine and diethylamine. The catalyst is preferably selected from the group consisting of 1,1'-bis(dicyclohexylphosphino)ferrocenepalladium dichloride (PdCl₂(dcypf)), palladium acetate (Pd(OAc)₂), palladium dichloride (PdCl₂), tri(dibenzylideneacetone)dipalladium (Pd₂(dba)₃), [1,1'-bis(diphenylphosphino)ferrocene]palladium dichloride (PdCl₂(dppf)), a complex of [1,1'-bis(diphenylphosphine)ferrocene]palladium dichloride and dichloromethane (PdCl₂(dppf)·CH₂Cl₂), tetrakis(triphenylphosphine)palladium (Pd(PPh₃)₄), bis(tricyclohexylphosphine)palladium dichloride (PdCl₂(P(Cy)₃)₂), 2-dicyclohexylphosphine-2',6'-dimethoxybiphenyl (SPhos). The reagents for the basic conditions are selected from the group consisting of organic base and inorganic bases, preferably selected from the group consisting of triethylamine (TEA), N, N-diisopropylethylamine (DIPEA), n-butyllithium, lithium diisopropylamide, lithium bis(trimethylsilyl)amide, potassium acetate (KOAc), sodium tert-butoxide (NaOBu-*t*), potassium tert-butoxide (KOBu-*t*), sodium hydride (NaH), potassium phosphate (K₃PO₄), sodium carbonate (Na₂CO₃), potassium carbonate (K₂CO₃), lithium hydroxide (KOH), sodium hydroxide (NaOH). The solvent for the Suzuki reaction is preferably selected from the group consisting of 1,4-dioxane, tetrahydrofuran (THF), acetonitrile (MeCN), N, N'-dimethylformamide (DMF), and mixed solvents of these solvents with water in different ratios.

The compound represented by formula (I-c) and the compound represented by formula (I-d) are subjected to a reductive amination reaction in the presence of an appropriate solvent and a reducing agent to obtain the compound represented by formula (I-e). The reducing agent is preferably selected from the group consisting of sodium acetate borohydride, sodium borohydride, sodium cyanoborohydride. The solvent includes but is not limited to dichloromethane (DCM), 1,2-dichloroethane, methanol (MeOH), ethanol (EtOH), 1,4-dioxane, tetrahydrofuran (THF), acetonitrile (MeCN), and N,N'-dimethylformamide (DMF); or

The compound represented by formula (I-c) and the compound represented by formula (I-d') are subjected to a nucleophilic substitution reaction in the presence of an appropriate solvent and base to obtain the compound represented by formula (I-e). The base is preferably selected from the group consisting of triethylamine (TEA), N,N'-diisopropylethylamine (DIPEA), pyridine (Py), n-butyllithium, lithium diisopropylamide, lithium bis(trimethylsilyl)amide, sodium tert-butoxide (NaOBu-*t*), potassium tert-butoxide (KOBu-*t*), sodium hydride (NaH), sodium carbonate (Na₂CO₃), potassium carbonate (K₂CO₃), lithium hydroxide (KOH), sodium hydroxide (NaOH). The solvent includes but is not limited to dichloromethane (DCM), 1,2-dichloroethane, methanol (MeOH), ethanol (EtOH), 1,4-dioxane, tetrahydrofuran (THF), acetonitrile (MeCN), N, N'-dimethylformamide (DMF), N-methylpyrrolidone (NMP), and pyridine (Py).

The compound represented by formula (I-e) is subjected to appropriate acid, base or catalytic hydrogenolysis in an appropriate solvent to remove the protecting group P². The obtained product is further subjected, without separation and purification, to a reductive amination reaction with the compound represented by formula (I-f) in the presence of an appropriate solvent and a reducing agent to obtain the compound represented by formula (I'). The acid is preferably selected from the group consisting of trifluoroacetic acid (TFA), hydrochloric acid (HCl), acetic acid (HOAc), and hydrobromic acid (HBr). The base includes but is not limited to piperidine, diethylamine. The reducing agent is preferably selected from the group consisting of sodium acetate borohydride, sodium borohydride, and sodium cyanoborohydride. The solvent includes but is not limited to dichloromethane (DCM), 1,2-dichloroethane, methanol (MeOH), ethanol (EtOH), 1,4-dioxane, tetrahydrofuran (THF), acetonitrile (MeCN), and N, N' - Dimethylformamide (DMF); or

The compound represented by formula (I-e) is subjected to appropriate acid, base or catalytic hydrogenolysis in an appropriate solvent to remove the protecting group P². The obtained product is further subjected, without separation and purification, to a nucleophilic substitution reaction with the compound represented by formula (I-f') in the presence of an appropriate solvent and base to obtain the compound shown in formula (I'). The base is preferably selected from the group consisting of triethylamine (TEA), N,N'-diisopropylethylamine (DIPEA), pyridine (Py), n-butyllithium, lithium diisopropylamide, lithium bis(trimethylsilyl)amide, sodium tert-butoxide (NaOBu-*t*), potassium tert-butoxide (KOBu-t), sodium hydride (NaH), sodium carbonate (Na₂CO₃), potassium carbonate (K₂CO₃), lithium hydroxide (KOH), and sodium hydroxide (NaOH). The solvent includes but is not limited to dichloromethane (DCM), 1,2-dichloroethane, methanol (MeOH), ethanol (EtOH), 1, 4-dioxane, tetrahydrofuran (THF), acetonitrile (MeCN), N,N'-dimethylformamide (DMF), N-methylpyrrolidone (NMP), and pyridine (Py).

The compound represented by the above formula (I') is a form of the final product (I). In addition, the compound represented by the formula (I') can be further subjected to an ester hydrolysis reaction under basic conditions to obtain the compound represented by the formula (I"), and the compound represented by the formula (I") is also a form of the final product (I). The base is preferably selected from the group consisting of lithium hydroxide (LiOH), lithium hydroxide (KOH), and sodium hydroxide (NaOH);
M is preferably selected from the group consisting of borate and boric acid, including but not limited to 4,4,5,5-tetramethyl-1,3,2-dioxaborolane, bis(neopentyl glycolato)diboron, 4,4,4',4',5,5,5',5'-octamethyl-2, 2'-bis(1, 3, 2-dioxaborolane)B(OBu-*n*)₃ and B(OPr-*i*)₃; or,
M is preferably selected from the group consisting of bromine, iodine, chlorine, fluorine, and CF₃SO₃-(OTf);
W is preferably selected from the group consisting of borate and boric acid, including but not limited to 4,4,5,5-tetramethyl-1,3,2-dioxaborolane, bis(neopentyl glycolato)diboron, 4,4,4',4',5, 5,5',5'-octamethyl-2, 2'-bis(1,3,2-dioxaborolane)B(OBu-*n*)₃ and B(OPr-*i*)₃; or,
W is preferably selected from the group consisting of bromine, iodine, chlorine, fluorine, and CF₃SO₃-(OTf).
P¹ and P² are protecting groups, which can be the same or different, preferably selected from the group consisting of Boc (tert-butoxycarbonyl), Fmoc (9-fluorenylmethoxycarbonyl), Cbz (N-benzyloxycarbonyl), methanesulfonyl, p-toluenesulfonyl, acetyl, methoxycarbonyl, ethoxycarbonyl, ((2-trimethylsilyl)ethoxy)methyl (SEM), and tetrahydro-2H-pyran-2-yl (THP).

The method for preparing the compounds of the present invention can also be conducted by: subjecting a compound represented by the formula (I-a) to removing the protecting group P¹, further subjecting the obtained product, without separation and purification, to a Suzuki reaction as described above with a compound represented by the formula (I-b) to obtain a compound represented by the formula (I-c), first subjecting the compound represented by the formula (I-c) and a compound represented by formula (I-f) to a reductive amination reaction as described above to obtain a compound represented by formula (I-h); or subjecting the compound represented by formula (I-c) and a compound represented by formula (I-f') to a nucleophilic substitution reaction as described above to obtain a compound represented by formula (I-h), then subjecting the compound represented by (I-h) to deprotection, and then to a reductive amination reaction as described above with a compound represented by formula (I-d) to obtain a compound represented by formula (I'); or subjecting the compound represented by (I-h) to deprotection, and then to a nucleophilic substitution reaction as described above with a compound represented by formula (I-d') to obtain a compound represented by formula (I'-1), or further performing an ester hydrolysis reaction as described above to obtain a compound represented by formula (I"-1). The preparation method comprises: 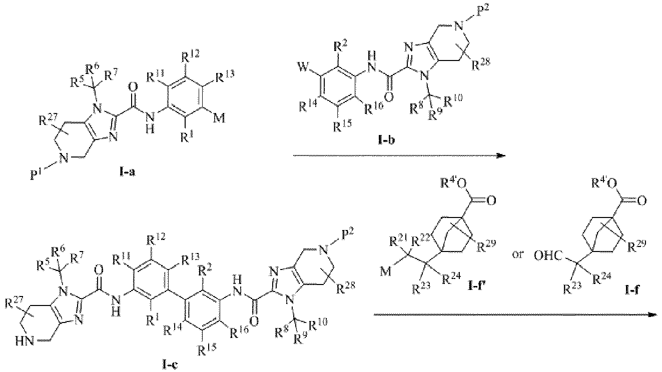 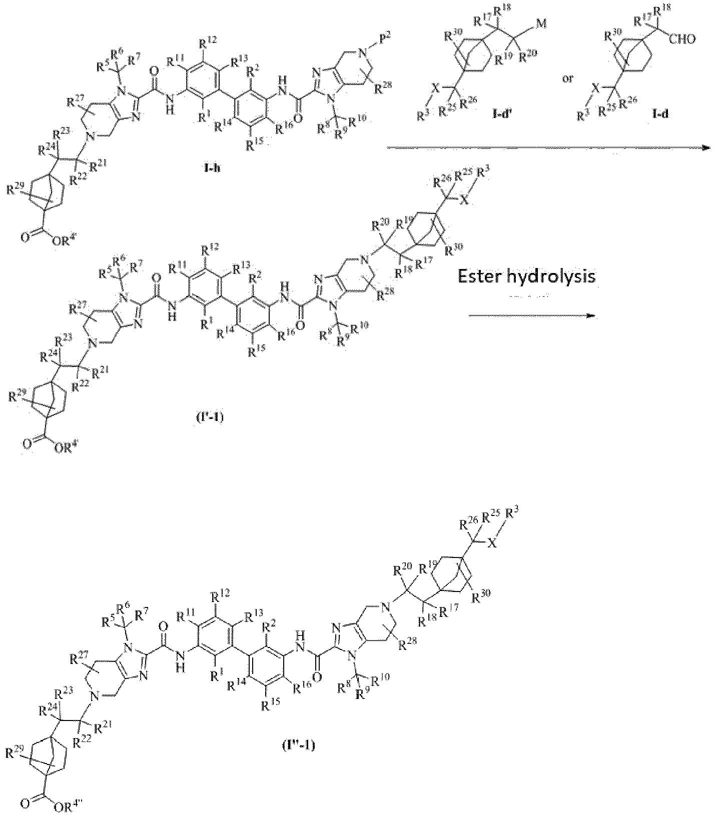
wherein, the above compound represented by formula (I'-1) is a form of the final product (I). In addition, the compound represented by formula (I') can be further subjected to ester hydrolysis under basic conditions to obtain the compound represented by formula (I"-1), and the compound represented by formula (I"-1) is also a form of the final product (I);
wherein, the definition of each substituent is as described above.

The method for preparing the compounds of the present invention can also be conducted by: subjecting a compound represented by the formula (I-a) to removing protecting group P¹, further subjecting the obtained product, without separation and purification, to a Suzuki reaction as described above with a compound represented by the formula (I-b) to obtain a compound represented by the formula (I-c), first subjecting the compound represented by the formula (I-c) and a compound represented by formula (I-f) to a reductive amination reaction as described above to obtain a compound represented by formula (I-h); or subjecting the compound represented by formula (I-c) and a compound represented by formula (I-f') to a nucleophilic substitution reaction as described above to obtain a compound represented by formula (I-h), then subjecting the compound represented by the formula (I-h) to ester hydrolysis as described above to obtain a compound represented by the formula (I-i), then subjecting the compound represented by the formula (I-i) to deprotection, and then to a reductive amination reaction as described above with a compound represented by formula (I-d) to obtain a compound represented by formula (I‴); or subjecting the compound represented by formula (I-i) to deprotection, and then to a nucleophilic substitution reaction as described above with a compound represented by formula (I-d') to obtain a compound represented by formula (I‴). The preparation method comprises: 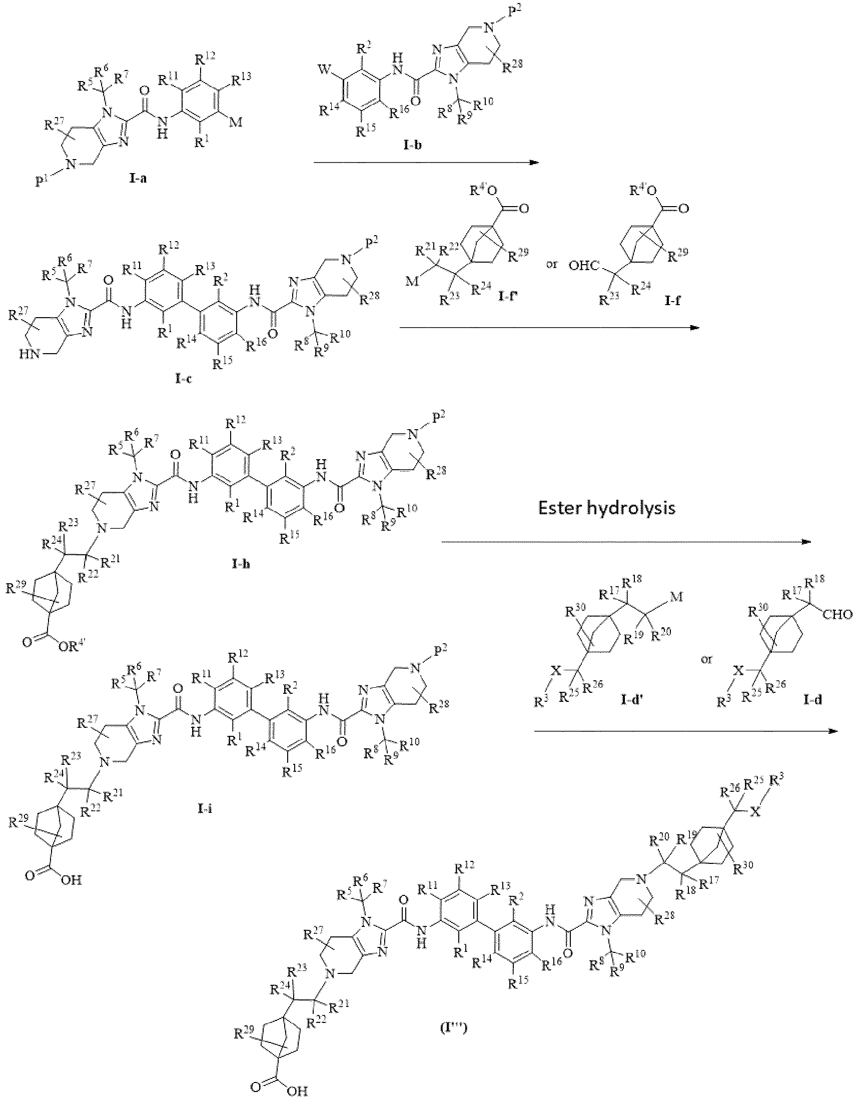
wherein, the above compound represented by formula (I‴) can be used as the final product (I);
wherein, the definition of each substituent is as described above.

During the preparation of the compound represented by the general formula (I), the present invention further provides a method for preparing the formula (I-a) used, comprising the following steps: 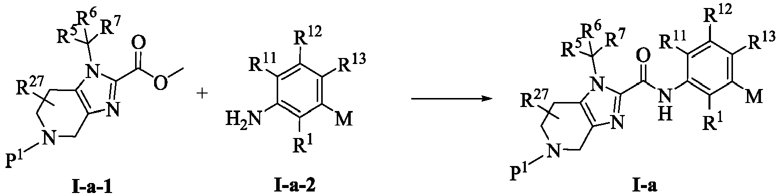
Formula (I-a-1) and formula (I-a-2) are subjected to an ammonolysis reaction under the action of a sixth base and a seventh solvent to obtain a compound represented by formula (I-a);
wherein: the definition of each substituent is as described above.

In some embodiments, the sixth base is preferably selected from the group consisting of potassium tert-butoxide (KOBu-t), sodium tert-butoxide (NaOBu-t), sodium hydride (NaH), n-butyllithium, sodium methoxide ( MeONa), sodium ethoxide (EtONa).

In some embodiments, the seventh solvent is preferably selected from the group consisting of anhydrous tetrahydrofuran (THF), anhydrous 1,4-dioxane, and anhydrous acetonitrile (MeCN).

During the preparation of the compound represented by the general formula (I), the present invention further provides a method for preparing the compound represented by the formula (I-b) used, comprising the following steps:

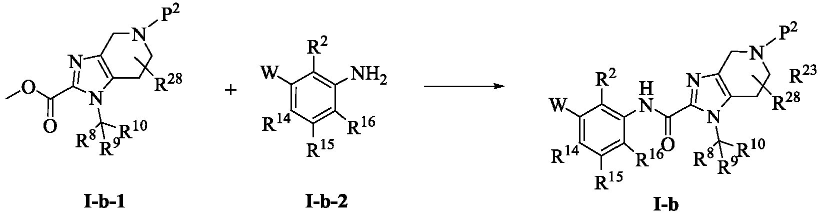

The preparation strategy for the compound represented by formula (I-b) is the same as that of the above compound represented by formula (I-a);
wherein: the definition of each substituent is as described above.

During the preparation process of the compound represented by the general formula (I-a), the present invention further provides a method for preparing the compound represented by formula (I-a-1) used, comprising the following steps: 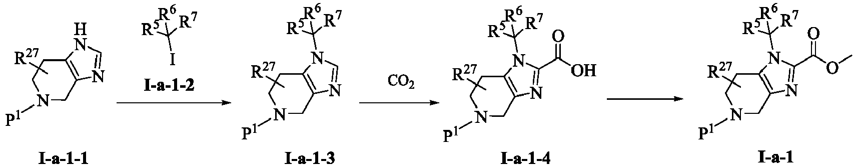
1) reacting a compound represented by formula (I-a-1-1) with a compound represented by formula (I-a-1-2) under the action of a seventh base to obtain a compound represented by formula (I-a-1-3);
   in some embodiments, the seventh base is preferably selected from the group consisting of potassium tert-butoxide (KOBu-*t*), sodium tert-butoxide (NaOBu-*t*), sodium hydride (NaH), n-butyllithium, sodium methoxide (MeONa), and sodium ethoxide (EtONa);
2) further subjecting the formula (I-a-1-3) to a carbonyl insertion reaction to obtain a compound represented by (I-a-1-4);
3) subjecting the compound represented by formula (I-a-1-4) to an esterification reaction to obtain the a compound represented by (I-a-1);
wherein: the definition of each substituent is as described above.

More specifically, a compound represented by formula (I-a-1-1) is reacted with a compound represented by formula (I-a-1-2) under the action of an appropriate base to obtain a compound represented by formula (I-a-1-3); wherein, the base is preferably selected from the group consisting of potassium tert-butoxide (KOBu-*t*), sodium tert-butoxide (NaOBu-t), sodium hydride (NaH), n-butyllithium, sodium methoxide (MeONa), and sodium ethoxide (EtONa). Then the compound represented by formula (I-a-1-3) is subjected to a carbonyl insertion reaction with CO₂ (dry ice) under the action of n-butyllithium to obtain a compound represented by (I-a-1-4). Finally, the compound represented by formula (I-a-1-4) in an activated form by acyl chloride is reacted with methanol (MeOH), or methanol (MeOH)/concentrated sulfuric acid to obtain the compound represented by (I-a-1).

During the preparation process of the compound represented by the general formula (I-a), the present invention further provides a method for preparing the compound represented by formula (I-a-1) used, comprising the following steps: 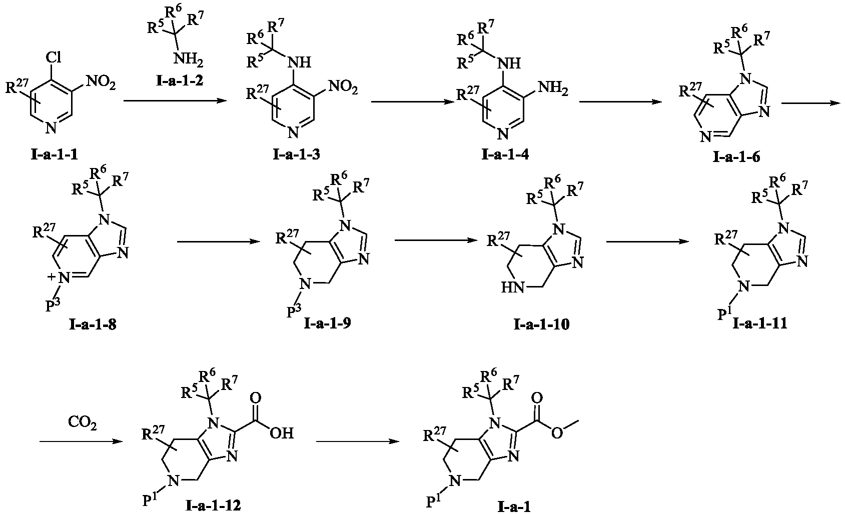
1) reacting a compound represented by the formula (I-a-1-1) with a compound represented by the formula (I-a-1-2) under the action of an eighth base to obtain a compound represented by the formula (I-a-1-3); 2) further reducing the nitro group of the compound represented by formula (I-a-1-3) to an amino group with a third reducing agent to obtain a compound represented by formula (I-a-1-4);
3) subjecting the compound represented by formula (I-a-1-4) to a cyclization reaction to obtain a compound represented by formula (I-a-1-6);
4) forming the compound represented by formula (I-a-1-6) into a quaternary ammonium salt to obtain a compound represented by formula (I-a-1-8) for further reduction reaction;
5) reducing the compound represented by formula (I-a-1-8) under the action of a fourth reducing agent to obtain a compound represented by the formula (I-a-1-9);
6) removing P³ from the compound represented by formula (I-a-1-9) to obtain a compound represented by formula (I-a-1-10);
7) further protecting the compound represented by formula (I-a-1-10) to obtain a compound represented by formula (I-a-1-11);
8) further subjecting the compound represented by formula (I-a-1-11) to a carbonyl insertion reaction to obtain a compound represented by formula (I-a-1-12);
9) subjecting the compound represented by formula (I-a-1-12) to an esterification reaction to obtain the compound represented by (I-a-1);
wherein: P³ refers to a fragment that can form a quaternary ammonium salt in order to promote the next reduction reaction, including but not limited to benzyl (Bn), p-methoxybenzyl (PMB), p-methoxycarbonylbenzyl, and allyl.

The definitions of other substituents are as described in claim 9 or 10.

In an embodiment, the eighth base is preferably selected from the group consisting of potassium carbonate (K₂CO₃), potassium tert-butoxide (KOBu-*t*), sodium tert-butoxide (NaOBu-t), sodium hydride (NaH), n-butyllithium, sodium methoxide (MeONa), and sodium ethoxide (EtONa).

In an embodiment, the third reducing agent is preferably selected from the group consisting of iron powder, zinc powder, tin dichloride (SnCl₂), sodium sulfide (Na₂S), and hydrazine hydrate.

In an embodiment, the fourth reducing agent is preferably selected from the group consisting of benzyl (Bn), p-methoxybenzyl (PMB), p-methoxycarbonylbenzyl, and allyl.

More particularly, a compound represented by formula (I-a-1-1) is reacted with a compound represented by formula (I-a-1-2) under the action of an appropriate base to obtain a compound represented by formula (I-a-1-3); wherein, the base is preferably selected from the group consisting of potassium carbonate (K₂CO₃), potassium tert-butoxide (KOBu-*t*), sodium tert-butoxide (NaOBu-*t*), sodium hydride (NaH), n-butyllithium, sodium methoxide (MeONa), and ethanol sodium (EtONa). Then the nitro group of the compound represented by formula (I-a-1-3) is reduced to an amino group under the action of a reducing agent to obtain the compound represented by formula (I-a-1-4), wherein the reducing agent is preferably selected from the group consisting of iron powder, zinc powder, tin dichloride (SnCl₂), sodium sulfide (Na₂S), and hydrazine hydrate. Further, the compound represented by formula (I-a-1-4) is subjected to a cyclization reaction to obtain a compound represented by (I-a-1-6). Then compound represented by (I-a-1-6) is formed into a quaternary ammonium salt to obtain a compound represented by formula (I-a-1-8). Then compound represented by formula (I-a-1-8) is reduced under the action of a reducing agent to obtain a compound represented by formula (I-a-1-9); wherein the reducing agent is preferably selected from the group consisting of benzyl (Bn), p-methoxybenzyl (PMB), p-methoxycarbonylbenzyl, and allyl. Further, P³ is removed to obtain a compound represented by formula (I-a-1-10), which is then subjected to P¹ protection, carbonyl insertion reaction and esterification reaction to obtain the compound represented by formula (I-a-1).

During the preparation process of the compound represented by formula (I-b), the preparation strategy of the formula (I-b-1) provided by the present invention is the same as the preparation strategy of the above formula (I-a-1).

The present invention further provides unpublished intermediates I-2a-1 (I-a-1), I-1d (I-d), I-2a-2 (I-a -2), I-3a-2 (I-a-2), I-4a-2 (I-a-2), and I-Sa-2 (I-a-2) during the preparation of the compound represented by formula (I), and these intermediates include but are not limited to:

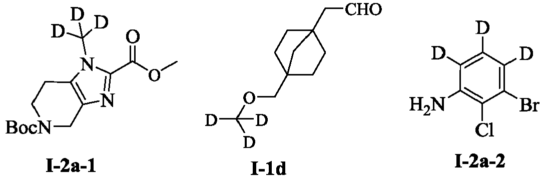

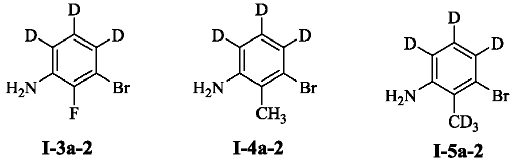

The present invention provides a method for preparing the above intermediate I-1d (I-d), comprising the following steps: 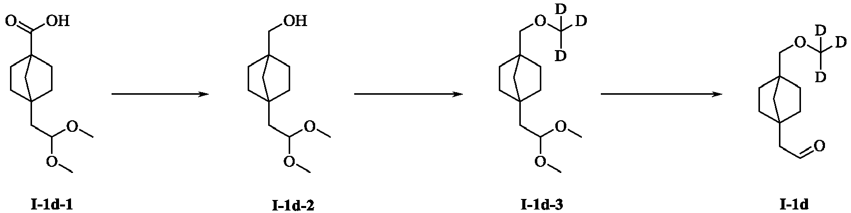
1) reducing a compound represented by formula (I-1d-1) with a fifth reducing agent to obtain a compound represented by formula (I-1d-2);
2) subjecting the compound represented by formula (I-1d-2) to a deuterated methylation reaction with a deuterated methylation reagent to obtain a compound represented by formula (I-1d-3);
3) finally deprotecting the compound represented by formula (I-1d-3) under a second acidic condition to obtain the compound represented by formula (I-1d);

wherein the fifth reducing agent is preferably selected from the group consisting of lithium aluminum hydride (LAH), borane (BH₃), and NaBH₄/AlCl₃;
wherein the methylating agent is preferably selected from the group consisting of deuterated iodomethane (CD₃I);
wherein the second acid is preferably selected from the group consisting of trifluoroacetic acid (TFA), hydrochloric acid (HCl), acetic acid (HOAc), and hydrobromic acid (HBr).

The present invention provides a method for preparing the above unknown intermediate I-2a-2 (I-a-2), comprising the following steps: 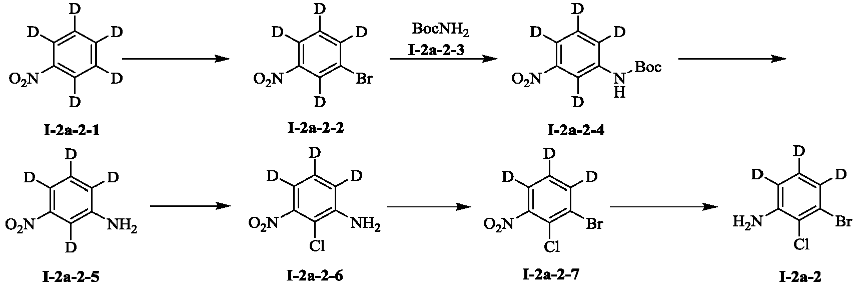
1) subjecting a compound represented by formula (I-2a-2-1) to a bromination reaction under the action of a brominating reagent to obtain a compound represented by formula (I-2a-2-2);
2) subjecting the compound represented by formula (I-2a-2-2) to C-N coupling reaction under the action of a second catalyst to obtain a compound represented by formula (I-2a-2-4);
3) deprotecting the compound represented by formula (I-2a-2-4) under a third acidic condition to obtain a compound represented by formula (I-2a-2-5);
4) subjecting the compound represented by formula (I-2a-2-5) to a chlorination reaction under the action of a chlorination reagent to obtain a compound represented by formula (I-2a-2-6);
5) subjecting the compound represented by formula (I-2a-2-6) to a diazotization reaction to obtain a compound represented by formula (I-2a-2-7);
6) reducing the nitro group of the compound represented by formula (I-2a-2-7) under the action of a sixth reducing agent to obtain the compound represented by formula (I-2a-2);

wherein, the brominating reagent is preferably selected from the group consisting of N-bromosuccinimide (NBS), sodium bromide (NaBr), potassium bromide (KBr), sodium bromate (NaBrO₃), and potassium bromate (KBrO₃);
wherein the second catalyst is preferably selected from the group consisting of 4,5-bisdiphenylphosphine-9,9-dimethylxanthene (Xantphos), 1,1'-bis(dicyclohexylphosphino)ferrocene palladium dichloride (PdCl₂(dcypf)), palladium acetate (Pd(OAc)₂), palladium dichloride (PdCl₂), tris(dibenzylidene acetone) dipalladium (Pd₂(dba)₃), [1,1'-bis(diphenylphosphino)ferrocene]palladium dichloride (PdCl₂(dppf)), a complex of [1,1'-bis(diphenylphosphino)ferrocene]palladium dichloride and dichloromethane (PdCl₂(dppf)·CH₂Cl₂), tetrakis(triphenylphosphine) palladium (Pd(PPh₃)₄), bis(tricyclohexylphosphine)palladium dichloride (PdCl₂(P(Cy)₃)₂), and 2-dicyclohexylphosphine-2',6'-dimethoxybiphenyl (SPhos);
wherein the third acid is preferably selected from the group consisting of trifluoroacetic acid (TFA), hydrochloric acid (HCl), acetic acid (HOAc), and hydrobromic acid (HBr);
wherein the chlorination reagent is preferably selected from the group consisting of N-chlorosuccinimide (NCS);
wherein, the sixth reducing agent is preferably selected from the group consisting of iron powder, zinc powder, tin dichloride (SnCl₂), sodium sulfide (Na₂S), and hydrazine hydrate.

The present invention provides a method for preparing the above unknown intermediate I-3a-2 (I-a-2), comprising the following steps: 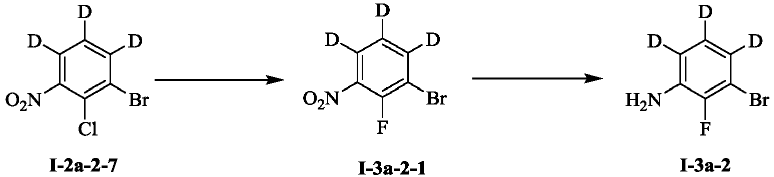
1) subjecting a compound represented by formula (I-2a-2-7) to a fluorination reaction under the action of a fluorinating reagent to obtain a compound represented by formula (I-3a-2-1);
2) reducing the nitro group of the compound represented by formula (I-3a-2-1) under the action of the seventh reducing agent to obtain the compound represented by formula (I-3a-2);

wherein the fluorinating reagent is preferably selected from the group consisting of potassium fluoride, and cesium fluoride;
wherein, the seventh reducing agent is preferably selected from the group consisting of iron powder, zinc powder, tin dichloride (SnCl₂), sodium sulfide (Na₂S), and hydrazine hydrate.

The present invention provides a method for preparing the above unknown intermediate I-4a-2 (I-a-2), comprising the following steps: 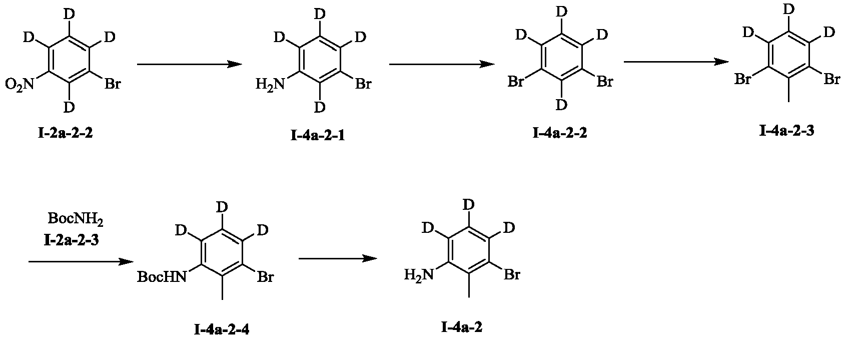
1) reducing the nitro group of a compound represented by formula (I-2a-2-2) under the action of an eighth reducing agent to obtain a compound represented by formula (I-4a-2-1);
2) subjecting the compound represented by formula (I-4a-2-1) to a diazotization reaction to obtain a compound represented by formula (I-4a-2-2);
3) subjecting the compound represented by formula (I-4a-2-2) to an electrophilic substitution reaction to obtain a compound represented by formula (I-4a-2-3);
4) subjecting the compound represented by formula (I-4a-2-3) to C-N coupling reaction under the action of a third catalyst to obtain a compound represented by formula (I-4a-2-4);
5) deprotecting the compound represented by formula (I-4a-2-4) under the fourth acidic condition to obtain the compound represented by formula (I-4a-2);

wherein, the eighth reducing agent is preferably selected from the group consisting of iron powder, zinc powder, tin dichloride (SnCl₂), sodium sulfide (Na₂S), and hydrazine hydrate;
wherein, the third catalyst is preferably selected from the group consisting of 4,5-bisdiphenylphosphine-9,9-dimethylxanthene (Xantphos), 1,1'-bis(dicyclohexylphosphino)ferrocene palladium dichloride (PdCl₂(dcypf)), palladium acetate (Pd(OAc)₂), palladium dichloride (PdCl₂), tris(dibenzylidene acetone) dipalladium (Pd₂(dba)₃), [1,1'-bis(diphenylphosphino)ferrocene]palladium dichloride (PdCl₂(dppf)), a complex of [1,1'-bis(diphenylphosphino)ferrocene]palladium dichloride and dichloromethane (PdCl₂(dppf)·CH₂Cl₂), tetrakis(triphenylphosphine) palladium (Pd(PPh₃)₄), bis(tricyclohexylphosphine)palladium dichloride (PdCl₂(P(Cy)₃)₂), and 2-dicyclohexylphosphine-2',6'-dimethoxybiphenyl (SPhos);
wherein, the fourth acid is preferably selected from the group consisting of trifluoroacetic acid (TFA), hydrochloric acid (HCl), acetic acid (HOAc), and hydrobromic acid (HBr).

In addition, the present invention provides use of the aforementioned compounds or stereoisomers, pharmaceutically acceptable salts, precursors or metabolites thereof, or the aforementioned pharmaceutical compositions in the manufacture of a medicament for treating and/or preventing a disease related to target PD-L1, or
use of the aforementioned compounds or stereoisomers, pharmaceutically acceptable salts, precursors or metabolites thereof, or the aforementioned pharmaceutical compositions in the manufacture of a medicament for inhibiting PD-L1 activity, or
use of the aforementioned compounds or stereoisomers, pharmaceutically acceptable salts, precursors or metabolites thereof, or the aforementioned pharmaceutical compositions in the manufacture of a medicament as a PD-L1 inhibitor, or
use of the aforementioned compounds or stereoisomers, pharmaceutically acceptable salts, precursors or metabolites thereof, or the aforementioned pharmaceutical compositions in the manufacture of a medicament as an immunomodulator targeting the PD-L1 signaling pathway.

In some embodiments, the disease related to target PD-L1 is selected from the group consisting of tumors, cancers and other immune-related diseases.

In another aspect, the present invention provides the aforementioned compounds or stereoisomers, pharmaceutically acceptable salts, precursors or metabolites thereof or the aforementioned pharmaceutical composition, for use in treating and/or preventing a disease related to target PD-L1, or
for inhibiting PD-L1 activity, or
as a PD-L1 inhibitor, or
as an immunomodulator targeting the PD-L1 signaling pathway.

In some embodiments, the disease related to target PD-L1 is selected from the group consisting of tumors, cancers and other immune-related diseases.

In another aspect, the present invention provides a method for treating and/or preventing a disease related to target PD-L1, comprising administering to a subject in need thereof a therapeutically and/or preventively effective amount of the aforementioned compound or stereoisomer, pharmaceutically acceptable salt, precursor or metabolite thereof, or the aforementioned pharmaceutical composition.

The present invention further provides a method for inhibiting PD-L1 activity, comprising administering to cells (such as mammalian cells) an effective amount of the aforementioned compound or stereoisomer, pharmaceutically acceptable salt, precursor or metabolite thereof or the aforementioned pharmaceutical composition.

In some embodiments, the disease related to target PD-L1 is selected from the group consisting of tumors, cancers and other immune-related diseases.

In the present invention, "subject" refers to a vertebrate. In certain embodiments, the vertebrate is a mammal. The mammal is selected from the group consisting of bovines, equines, ovines, porcines, canines, felines, rodents, and primates, such as humans, cats, dogs or pigs. The mammal includes, but is not limited to, livestock (such as cattle), pets (such as cats, dogs, and horses), primates, mice, and rats. In certain embodiments, the mammal is a human.

In the present invention, the term "therapeutically effective amount" or "preventively effective amount" refers to an amount sufficient to treat or prevent a patient's disease but low enough to avoid serious side effects (at a reasonable benefit/risk ratio) within the scope of sound medical judgment. The therapeutically effective amount of a compound will depend on the factors such as the particular compound selected (e.g., taking into account the potency, effectiveness, and half-life of the compound), the route of administration selected, the disease being treated, the severity of the disease being treated, and the age, size, weight and physical ailments of the patient being treated, and the medical history, duration of treatment, nature of concurrent therapy, and desired therapeutic effect of the patient being treated. The therapeutically effective amount of a compound can still be routinely determined by those skilled in the art.

In addition, it should be noted that the specific administration dosage and administration method of the compound or stereoisomer, pharmaceutically acceptable salt, precursor or metabolite thereof for different patients depend on many factors, including the patient's age, body weight, gender, natural health status, nutritional status, active strength of the drug, duration of administration, metabolic rate, severity degree of the disease, and the subjective judgment of the treating physician. A dose of 0.001-1000 mg/kg body weight/day is preferably used here.

The present invention provides a novel biphenyl small molecular immunosuppressant with excellent oral absorption characteristics, which is used for treating or preventing immune-related diseases. Meanwhile, these compounds or the pharmaceutical compositions containing them as active ingredients can maximize the clinical curative effect on these diseases within a safe treatment window.

### BRIEF DESCRIPTION OF DRAWINGS

FIG. 1 shows the tissue distribution of Compound I-1 (50 mg/kg, PO) in hPD1/ hPD-L1 mice after administration for 18 days.
FIG. 2 shows the tissue distribution relative to plasma of Compound I-1, Example 17, Compound 14 (INCB086550) and Example 180 in mice.

### DETAILED DESCRIPTION

The examples and preparation examples provided in the present invention further illustrate and exemplify the compounds of the present invention and the preparation methods therefor. It should be understood that the following preparations examples and examples do not limit the scope of the present invention in any way.

### LC-MS analysis method:

Mass spectrometry conditions: Instrument: Thermo MSQ Plus; ion source: ESI (EA+ EA-); cone voltage: 30 V; capillary voltage: 3.00 KV; source temperature: 350 °C.
Chromatographic conditions: Instrument: Thermo U3000; detector: DAD-3000 (RS) (diode array detector); chromatographic column: Shimadzu Inertsil ODS-HL HP 3 µm 3.0×100 mm; flow rate: 0.4 mL/min; column temperature: 30 °C; mobile phase: CH₃OH/H₂O/HCOOH (75/25/0.2).

### HPLC analysis method (1):

Instrument: Thermo U3000; detector: VWD-3×00 (RS) (UV detector); chromatographic column: Shimadzu Shim-pack GIST C8 5 µm 4.6×250 mm; flow rate: 1.0 mL/min; column temperature: 30 °C; mobile phase CH₃OH/H₂O/TEA/HOAc (80/20/0.2/0.1).

### HPLC analysis method (2):

Instrument: Thermo U3000; detector: VWD-3×00 (RS) (UV detector); chromatographic column: Shimadzu Shim-pack GIST C8 5 µm 4.6×250 mm; flow rate: 1.0 mL/min; column temperature: 30 °C; mobile phase: CH₃OH/pH4.5 HCOONH₄ (85/15).

### ¹H-NMR analysis method:

¹H-NMR was measured at room temperature in DMSO-*d*₆ or CDCl₃ using a BRUKER AVANCE-400 MHz nuclear magnetic resonance spectrometer with TMS as the internal standard. The signal peaks are expressed as s (singlet), d (doublet), t (triplet), q (quartet), m (multiplet), dd (double doublet). The unit of coupling constant (J) is hertz (Hz).

### Key abbreviations:

| | | | |
|---|---|---|---|
| 1,4-dioxane | 1, 4-dioxane; | DCM | Dichloromethane |
| MeOH | Methanol; | EtOAc | Ethyl acetate |
| TEA | Triethylamine; | DIPEA | N, N'-Diisopropylethylamine |
| TFA | Trifluoroacetic acid; | THF | Tetrahydrofuran |
| NaHCO₃ | Sodium bicarbonate; | Na₂SO₄ | Sodium sulfate |
| NaBH(OAc)₃ | Sodium borohydride acetate; | LiOH•H₂O | Lithium hydroxide hydrate |
| PdCl₂(dcypf) | 1,1'-Bis(dicyclohexylphosphino)ferrocene palladium dichloride | | |
| TLC | Thin layer chromatography | | |

According to the method described above, the present invention has prepared representative compounds I-1~I-15 (see Table 1).

**Table 1 Representative compounds I-1~I-15 described in the present invention**

| Number of compound (Example) | %Purity (HPLC) | Retention time (min) | Detection wavelength (nm) | HPLC analysis method |
|---|---|---|---|---|
| I-1 | 92.0 | 10.74 | 254 | (1) |
| I-2 | 96.6 | 8.54 | 254 | (1) |
| I-3 | 98.3 | 8.10 | 254 | (1) |
| I-4 | 98.8 | 8.12 | 254 | (1) |
| I-5 | 98.1 | 13.86 | 254 | (1) |
| I-6 | 96.1 | 13.09 | 254 | (1) |
| I-7 | 96.5 | 10.27 | 254 | (1) |
| I-8 | 98.6 | 9.33 | 254 | (1) |
| I-9 | 97.6 | 8.33 | 254 | (1) |
| I-10 | 97.6 | 8.46 | 254 | (1) |
| I-11 | 93.8 | 8.55 | 254 | (1) |
| I-12 | 93.6 | 8.54 | 254 | (1) |
| I-13 | 96.2 | 13.50 | 297 | (1) |
| I-14 | 97.0 | 4.25 | 254 | (2) |
| I-15 | 99.2 | 4.09 | 254 | (2) |

The content of the present invention will be further described below in conjunction with specific examples, but the protection scope of the present invention is not limited to these examples. Unless otherwise specified, the percentages described in the present invention are all percentages by weight. Numerical ranges described in the specification, such as units of measurement, reaction conditions, and physical states of compounds or percentages, are all intended to provide an unambiguous written reference. It is still possible for those skilled in the art, when implementing the present invention, to obtain expected results by using temperature, concentration, amount, number of carbon atoms, etc. outside this range or different from a single numerical value. In addition, unless otherwise specified, the raw materials in the following examples are commercially available, for example, they can be purchased from Shanghai Bide Pharmaceutical Technology Co., Ltd., Jiangsu Aikon Biomedical Research and Development Co., Ltd., Nanjing PharmaBlock Sciences Co., Ltd., Shanghai Shaoyuan Reagent Co., Ltd., Hechun Biotechnology (Shanghai) Co., Ltd.

### Preparation of intermediate tert-butyl 2-((3-bromo-2-chlorophenyl)carbamoyl)-1-methyl-1,4,6,7-tetrahydro-5H-imidazo [4,5 -c]pyridine-5 -carboxylate I-1a

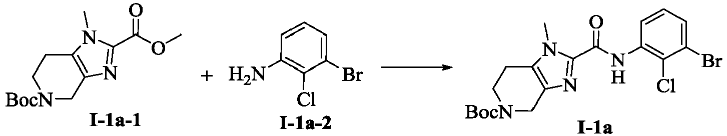

I-1a-1 (16.02 g, 54.24 mmol, 1.0 eq) and I-1a-2 (11.2 g, 54.24 mmol, 1.0 eq) were dissolved in anhydrous THF (30 mL). After stirring for 5 min, a solution of KOBu-t (18.26 g, 162.72 mmol, 3.0 eq) in anhydrous THF (20 mL) was added, and the mixture was stirred at ambient temperature for 1 h. The reaction was quenched with saturated brine (200 mL), and the reaction mixture was extracted twice with EtOAc (200 mL). The organic phases were combined, and dried over anhydrous Na₂SO₄. The crude product was separated by a silica gel column (n-Hep/EtOAc (v/v) = 3/1) to obtain light yellow solid I-1a. (23.70 g, yield 93.0%). LC-MS MS-ESI (m/z) 469.1 [M+H]⁺.

### Preparation of intermediate tert-butyl 2-((3-bromo-2-chlorophenyl)carbamoyl)-1-methyl-d₃-1,4,6,7-tetrahydro-5H-imidazo[4,5-c]pyridi ne-5-carboxylate I-2a

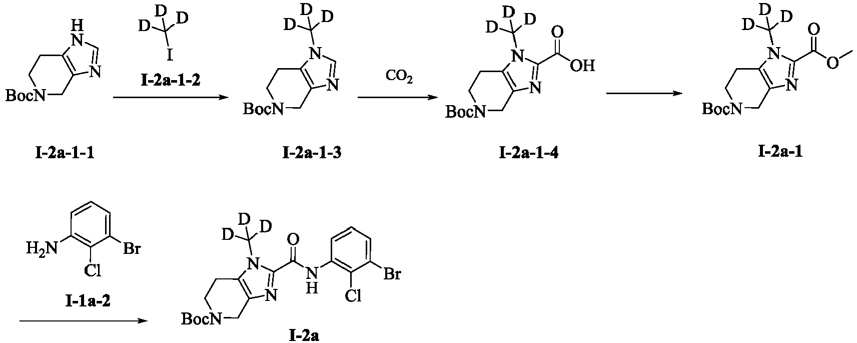

Commercially available I-2a-1-1 (10.00 g, 44.8 mmol, 1.0 eq), NaH (2.69 g, 112.0 mmol, 2.5 eq), and THF (40 mL) were stirred at 0 °C for 0.5 h, and added with I-2a-1-2 (10.00 g, 69.0 mmol, 1.5 eq). The mixture was continued to be stirred for 16 h. The reaction solution was diluted with ethyl acetate (EtOAc, 60 mL), and washed once with saturated brine. The organic phase was dried over anhydrous Na₂SO₄, and concentrated to obtain a crude product, which was separated by column chromatography (DCM/MeOH (v/v) = 98/2) to obtain pale yellow oil I-2a-1-3. (7.00 g, yield 64.5%). LC-MS MS-ESI (m/z) 241.2 [M+H]⁺.

Self-made I-2a-1-3 (7.00 g, 28.9 mmol, 1.0 eq), n-butyllithium (2.5 M, 13.9 mL, 1.2 eq), and anhydrous THF (80 mL) were stirred below -50 °C for 1 h. Then the mixture was introduced with CO₂ gas, and continued to be stirred for 1 h. The reaction solution was warmed to room temperature, added with water (4 mL), heated to 70 °C and stirred for 1 h. The reaction solution was concentrated to obtain a crude product, which was separated by column chromatography (DCM/MeOH (v/v) = 10/1) to obtain a yellow solid I-2a-1-4. (6.50 g, yield 79.2%). LC-MS MS-ESI (m/z) 285.2 [M+H]⁺.

Self-made I-2a-1-4 (2.00 g, 7.03 mmol, 1.0 eq), oxalyl chloride (1.07 g, 8.44 mmol, 1.2 eq), a catalytic amount of DMF, and DCM (10 mL) were stirred at room temperature for 1.5 h, and MeOH (2 mL) was added. The reaction solution was concentrated to obtain a crude product, which was separated by column chromatography (DCM/MeOH (v/v) = 98/2) to obtain a yellow solid I-2a-1. (700.00 mg, yield 33.4%). LC-MS MS-ESI (m/z) 299.2 [M+H]⁺.

Self-made I-2a-1 (700.00 mg, 2.35 mmol, 1.0 eq), I-1a-2 (485.21 mg, 2.35 mmol, 1.0 eq), KOBu-t (659.18 mg, 5.88 mmol, 2.5 eq), and THF (40 mL) were stirred at 0°C for 0.5 h. The reaction solution was diluted with EtOAc (60 mL), and washed once with saturated brine. The organic phase was dried over anhydrous Na₂SO₄ and concentrated to obtain a crude product, which was separated by column chromatography (PE/EtOAc (v/v) = 5/1) to obtain a white solid I-2a. (300.00 mg, yield 27.0%). LC-MS MS-ESI (m/z) 472.1 [M+H]⁺.
Alternative preparation of intermediate 5-(tert-butyl)2-methyl 1-methyl-*d*3-1,4,6,7-tetrahydro-5H-imidazo[4,5-c]pyridine-2,5-dicarboxylate I-2a-1

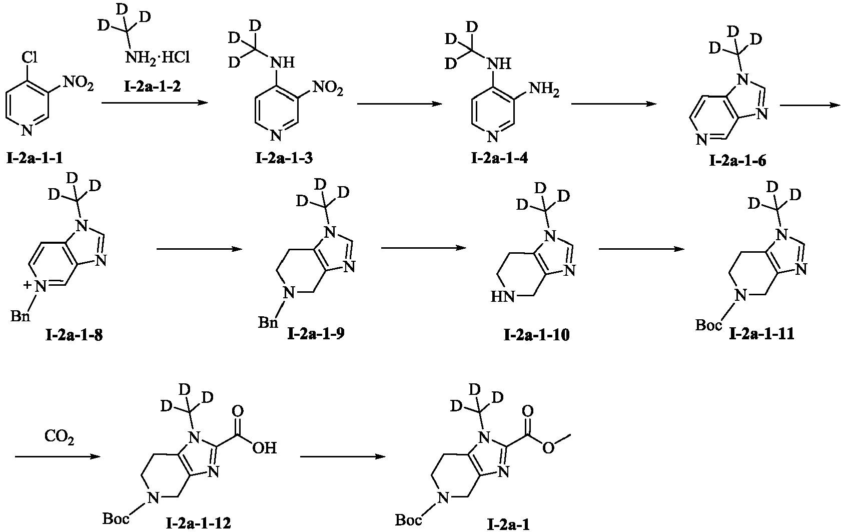

Commercially available I-2a-1-1 (15.85 g, 100.0 mmol, 1.0 eq) was dissolved in isopropanol (150 mL), and added with K₂CO₃ (41.46 g, 300.0 mmol, 3.0 eq). The mixture was cooled in an ice bath, and added with deuterated methylamine hydrochloride I-2a-1-2 (10.57 g, 150.0 mmol, 1.5 eq) in batch. The mixture was stirred mechanically at room temperature for 2 h. The reaction solution was diluted with water (150 mL), extracted with DCM (150 mLx3), and washed once with saturated NaHCO₃ aqueous solution. The organic phase was dried over anhydrous Na₂SO₄ and concentrated to obtain a yellow solid I-2a-1-3, which was directly used in the next reaction. (12.50 g, yield 80%). LC-MS MS-ESI (m/z) 157.1 [M+H]⁺.

I-2a-1-3 (9.50 g, 80.0 mmol, 1.0 eq) was dissolved in a mixed solution of methanol (100 mL) and formic acid (20 mL). Commercially available reduced iron powder (22.34 g, 400.0 mmol, 1.5 eq) was slowly added in batch. The mixture was mechanically stirred for 2 h, and filtered with suction, and the filtrate was spin-dried to obtain a light brown solid I-2a-1-4, which was directly used in the next reaction. (8.50 g, yield 84.2%). LC-MS MS-ESI (m/z) 127.1 [M+H]⁺.

I-2a-1-4 (8.50 g, 67.36 mmol, 1.0 eq) was suspended in triethyl orthoformate (100 mL), and added with formic acid (1 mL). The reaction solution was heated to 100°C for 3 h of reaction. Then the reaction solution was concentrated. The crude product was separated by silica gel column chromatography (DCM/MeOH (v/v) = 20/1) to obtain a light yellow solid I-2a-1-6. (6.40 g, yield 69.7%). LC-MS MS-ESI (m/z) 137.1 [M+H]⁺.

I-2a-1-6 (6.40 g, 47.0 mmol, 1.0 eq) was dissolved in DCM (20 mL), and added with benzyl bromide (BnBr) (8.04 g, 47.0 mmol, 1.0 eq) for 3 h of reaction at room temperature. The reaction solution was concentrated to obtain a foamy solid I-2a-1-8, which was directly used in the next reaction. (15.50 g, the yield was calculated as 100%). LC-MS MS-ESI (m/z) 227.1 [M+H]⁺.

I-2a-1-8 (15.50 g, 47.0 mmol, 1.0 eq) was dissolved in methanol (50 mL), and slowly added with NaBH₄ (5.33 g, 141.0 mmol, 3.0 eq) for 2 h of reaction at room temperature. The reaction mixture was diluted with water (100 mL), and extracted with DCM (100 mL). The organic phase was concentrated to obtain a transparent viscous oil I-2a-1-9, which was directly used in the next reaction. (16.20 g, the yield was calculated as 100%). LC-MS MS-ESI (m/z) 231.1 [M+H]⁺.

I-2a-1-9 (16.20 g, 47.0 mmol, 1.0 eq) was dissolved in methanol (100 mL), added with ammonium formate (29.64 g, 470.0 mmol, 10.0 eq) and palladium hydroxide (20 wt %, 3.24 g). The reaction solution was heated to 80°C for 4 h of reaction, then cooled to room temperature, and filtered with suction. The filtrate was concentrated to obtain a transparent viscous oil I-2a-1-10, which was directly used in the next reaction. (14.50 g, the yield was calculated as 100%). LC-MS MS-ESI (m/z) 141.1 [M+H]⁺.

I-2a-1-10 (14.50 g, 47.0 mmol, 1.0 eq) was dissolved in DCM (80 mL), and added with saturated NaHCO₃ solution (60 mL) for 16 h of reaction at room temperature. The reaction solution was subjected to liquid separation. The organic phase was concentrated, and the crude product was separated through silica gel column chromatography (DCM/MeOH (v/v) = 20/1) to obtain a transparent viscous oil I-2a-1-11. (8.20 g, yield 72.6%). LC-MS MS-ESI (m/z) 241.2 [M+H]⁺.

I-2a-1-11 (8.20 g, 34.1 mmol, 1.0 eq) was dissolved in THF (80 mL). The reaction solution was cooled to below -70 °C with a dry ice/ethanol bath. n-Butyllithium (2.5 M, 27.3 mL, 68.2 mmol, 2.0 eq) was dropwise added. After the dropwise addition was completed, the temperature and stirring were maintained for 30 min. CO₂ was introduced into the reaction solution for 30 min of reaction. The reaction was quenched by adding water dropwise (5 mL). The reaction solution was slowly warmed to room temperature. The organic phase was concentrated, the crude product was separated by silica gel column chromatography (DCM/MeOH (v/v) = 7/1) to obtain a light yellow solid I-2a-1-12. (4.50 g, yield 46.4%). LC-MS MS-ESI (m/z) 285.2 [M+H]⁺.

I-2a-1-12 (4.50 g, 15.8 mmol, 1.0 eq) was dissolved in DCM (80 mL). The reaction solution was cooled in an ice bath, added with DMF (0.1 mL), and dropwise added with oxalyl chloride (4.01 g, 31.6 mmol , 2.0 eq). After the dropwise addition was completed, the reaction solution was continued to be stirred at room temperature for 30 min, then cooled in an ice-salt bath to below 0°C, added with methanol (10 mL), and then added with TEA (5 mL). The mixture was stirred for 10 min, diluted with water, and subjected to liquid separation. The organic phase was concentrated, and the crude product was separated by silica gel column chromatography (DCM/MeOH (v/v) = 10/1) to obtain off-white solid I-2a-1. (3.60 g, yield 76.2%). LC-MS MS-ESI (m/z) 299.2 [M+H]⁺.

### Preparation of intermediate 3-bromo-2-chlorobenzene-4,5,6-d₃-amine I-2a-2

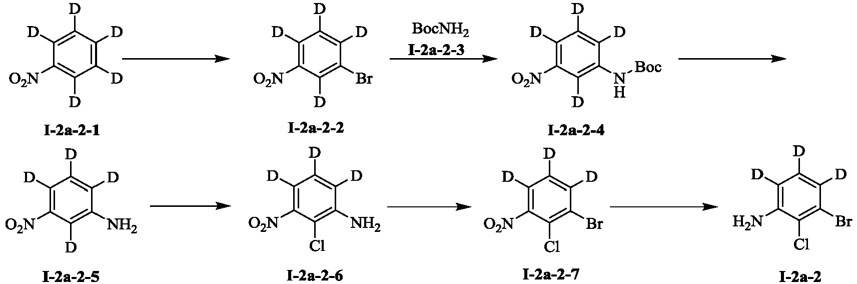

Commercially available I-2a-2-1 (25.00 g, 195.0 mmol, 1.0 eq) and NaBrO₃ (29.40 g, 195.0 mmol, 1.0 eq) were added to a mixed solvent of concentrated sulfuric acid (100 mL) and water (100 mL). The mixture was stirred at 40-45°C, and then slowly added with K₂SO₄ (241.00 mg, 1.39 mmol, 0.007 eq) at this temperature. Then the reaction solution was continued to be stirred at this temperature for 16 h. The reaction was monitored to be completed by HPLC. At 20 °C, the pH of the reaction solution was adjusted to 7-8 with Na₂CO₃. The reaction solution was extracted with EtOAc (3000 mL × 2). The organic phases were combined and washed once with saturated brine. The organic phase was dried over anhydrous Na₂SO₄ and concentrated to obtain a yellowish brown solid I-2a-2-2. (36.50 g, yield 90.8%).

I-2a-2-2 (36.50 g, 177.0 mmol, 1.0 eq), Xantphos (10.30 g, 17.7 mmol, 0.1 eq), Cs₂CO₃ (115.00 g, 354.0 mmol, 2.0 eq), Pd₂(dpa)₃ (9.73 g, 10.6 mmol, 0.06 eq), and commercially available I-2a-2-3 (24.90 g, 213.0 mmol, 1.2 eq) were dissolved in toluene (60 mL). The mixture was stirred at 100 °C for 16 h. The reaction was monitored to be completed by LC-MS. The reaction solution was cooled to 25 °C, diluted with water (1500 mL), and then extracted with EtOAc (1500 mL × 3). The organic phases were combined and washed once with saturated brine. The organic phase was dried over anhydrous Na₂SO₄ and concentrated to obtain a crude product, which was separated by column chromatography (PE/EtOAc (v/v) = 100/1-90/10) to obtain a yellow solid I-2a-2-4. (49.50 g, crude product).

I-2a-2-4 (49.50 g, 204.3 mmol, 1.0 eq) was dissolved in DCM, and then added with TFA (349.45 g, 3.06 mol, 15 eq). The reaction solution was stirred at 40 °C for 16 h. The raw materials were monitored to be partially reacted by TLC. The reaction was quenched with water. Then the pH of the reaction solution was adjusted to 9 with 1M NaOH. The reaction solution was extracted with EtOAc (1000 mL × 2). The organic phases were combined and washed once with saturated brine. The organic phase was dried over anhydrous Na₂SO₄ and concentrated to obtain a crude product, which was separated by column chromatography (PE/EtOAc (v/v) = 100/1-92/8) to obtain a yellow solid I-2a-2-5. (18.00 g, yield 61.9%).

I-2a-2-5 (18.00 g, 127.0 mmol, 1.0 eq) was added to a solution of N-chlorosuccinimide (NCS, 16.9 g, 127.0 mmol, 1.0 eq) in DMF (50 mL). The reaction solution was stirred at 75 °C for 1 h. The reaction was monitored to be completed by TLC. The reaction was quenched with water. Then the reaction solution was extracted with EtOAc (100 mL). The organic phases were combined and washed with saturated brine. The organic phase was dried over anhydrous Na₂SO₄ and concentrated to obtain a crude product, which was separated by column chromatography (PE/EtOAc (v/v) = 100/1-90/1) to obtain a yellow solid I-2a-2-6. (15.50 g, yield 69.7%).

At 25°C, tert-butyl nitrite (12.8 g, 124.0 mmol, 1.5 eq) was added to a solution of CuBr₂ (22.2 g, 99.2 mmol, 1.2 eq) in acetonitrile (MeCN, 100 mL). Then the mixture was heated to a temperature of 65 °C. A self-made solution of I-2a-2-6 (14.5 g, 82.6 mmol, 1.0 eq) in MeCN (50 mL) was added. The reaction solution was stirred at this temperature for 1 h. The reaction was monitored to be completed by TLC. The reaction solution was cooled to 25 °C. The reaction was quenched with NaHCO₃ (500 mL) aqueous solution. The reaction solution was extracted with EtOAc (500 mL × 2). The organic phases were combined and washed with sodium thiosulfate aqueous solution (300 mL) and saturated brine (500 mL). The organic phase was dried over anhydrous MgSO₄ and concentrated to obtain a crude product, which was separated by column chromatography (PE/EtOAc (v/v) = 100/1) to obtain a white solid I-2a-2-7. (9.30 g, yield 47.0%).

I-2a-2-7 ((9.88 g, 41.3 mmol, 1.0 eq) was dissolved in a mixed solvent of ethanol (EtOH, 90 mL) and water (30 mL). Fe powder (6.91 g, 124 mmol, 3.0 eq) and HOAc (13.2 mL, 230 mmol, 5.6 eq) were added. The reaction solution was stirred at 30 °C for 16 h. The reaction was monitored to be completed by TLC. The pH of the reaction solution was adjusted to 7-8 with 5M NaOH. The reaction solution was diluted with water (100 mL), and then extracted with EtOAc (200 mL). The organic phases were combined and washed with saturated brine. The organic phase was dried over anhydrous Na₂SO₄ and concentrated to obtain a yellowish brown oil I-2a-2. (8.50 g, yield 97.8%).¹H-NMR (400MHz, CDCl₃) *δ* ppm 4.18 (s, 2H).

### Preparation of intermediate tert-butyl 2-((3-bromo-2-chlorophenyl-4,5,6-d₃)carbamoyl)-1-(methyl-d₃)-1, 4, 6, 7-tetrahydro-5H-imidazo[4,5-c]pyridine-5-carboxylate I-3a

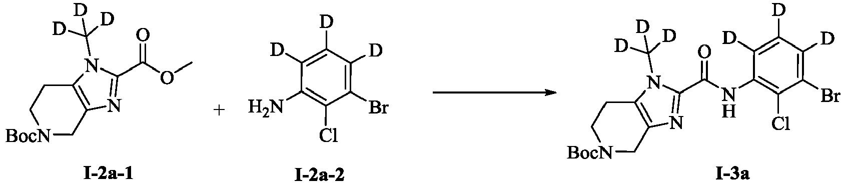

The previously prepared I-2a-2 (1.05 g, 5.0 mmol, 1.0 eq) and I-2a-1 (1.49 g, 5.0 mmol, 1.0 eq) were dissolved in anhydrous THF (50 mL), and added with KOBu-*t* (1.12 g, 10.0 mmol, 2.0 eq). The mixture was stirred at room temperature under N₂ protection for 1 h. The reaction was quenched with saturated brine (100 mL), and extracted twice with EtOAc (80 mL). The organic phases were combined and dried over anhydrous Na₂SO₄. The crude product was separated by silica gel column chromatography (EtOAc/hexane (v/v) = 1/3) to obtain a white solid I-3a. (950.00 mg, yield 39.9%). LC-MS MS-ESI (m/z) 475.1 [M+H]⁺.

### Preparation of intermediate 3-bromo-2-fluorobenzene-4,5,6-d₃-amine I-3a-2

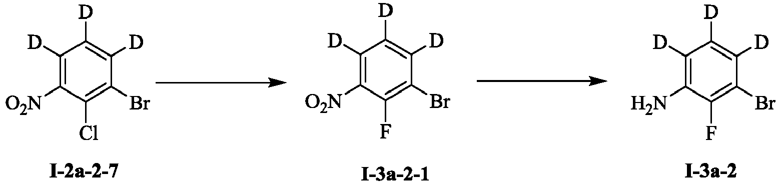

The previously prepared I-2a-2-7 (21.60 g, 89.8 mmol, 1.0 eq), potassium fluoride (10.40 g, 178.0 mmol, 2.0 eq) and cesium fluoride (13.60 g, 89.8 mmol, 1.0 eq) were dissolved in DMF (200 mL). The reaction solution was stirred at 140°C for 5 h. The reaction was monitored to be completed by LC-MS. Then the reaction solution was cooled to 25°C, diluted with water (100 mL), and extracted with EtOAc (150 mL × 2). The organic layers were combined, washed 5 times with saturated brine, dried over anhydrous Na₂SO₄, and concentrated to obtain a crude product. After being separated by column chromatography (PE), the compound obtained after concentration was further separated by preparative HPLC (chromatographic column model: Welch Xtimate C18 10 µm 250×50 mm; mobile phase: water (1/1000 ammonia water + 1/1000 ammonium bicarbonate)-acetonitrile; B%: 25%-65%, 23min), and concentrated to obtain a yellow liquid I-3a-2-1. (14.50 g, yield 72.4%).

I-3a-2-1 (13.00 g, 58.2 mmol, 1.0 eq) was dissolved in a mixed solvent of EtOH (120 mL) and water (40 mL), and iron powder (9.80 g, 174.0 mmol, 3.0 eq) and HOAc (19.60 g, 325.0 mmol, 5.6 eq) were added. The reaction solution was stirred at 30 °C for 16 h. The reaction was monitored to be completed by LC-MS. The reaction solution was filtered by suction and concentrated. Then the mixture was separated by column chromatography (PE/ EtOAc ( v/v) = 100/1-90/10) and concentrated. The compound obtained after concentration was further separated by preparative HPLC (column model: Phenomenex luna C18 10 µm 250×80 mm; mobile phase: water (1/1000 ammonium bicarbonate) - acetonitrile; B%: 20%-60%, 25min) and concentrated to obtain a yellow oil I-3a-2. (5.1 g, yield 44.8%). ¹H-NMR (400MHz, DMSO) *δ* ppm 7.11 (s, 2H).

### Preparation of intermediate tert-butyl 2-((3-bromo-2-chlorophenyl-4,5,6-d₃)carbamoyl)-1-methyl-1,4,6,7-tetrahydro-5H-imidazo[4,5-c] pyridine-5-carboxylate I-14a

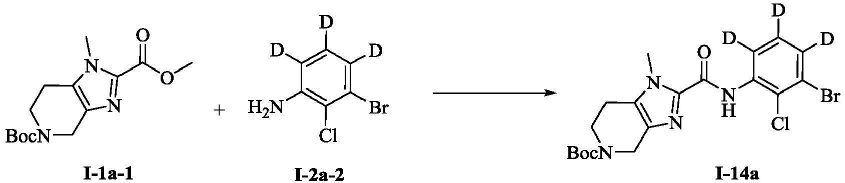

Commercially available I-1a-1 (788.32 mg, 2.67 mmol, 1.0 eq) and previously prepared I-2a-2 (560.00 mg, 2.67 mmol, 1.0 eq) and KOBu-*t* (593.91 mg , 5.53 mmol, 2.0 eq) were subjected to a procedure similar to that of preparing intermediate I-3a to obtain a white solid I-14a. (780.00 mg, yield 61.9%). LC-MS MS-ESI (m/z) 472.1 [M+H]⁺.

### Preparation of intermediate 3-bromo-2-methylbenzene-4,5,6-d₃-amine I-4a-2

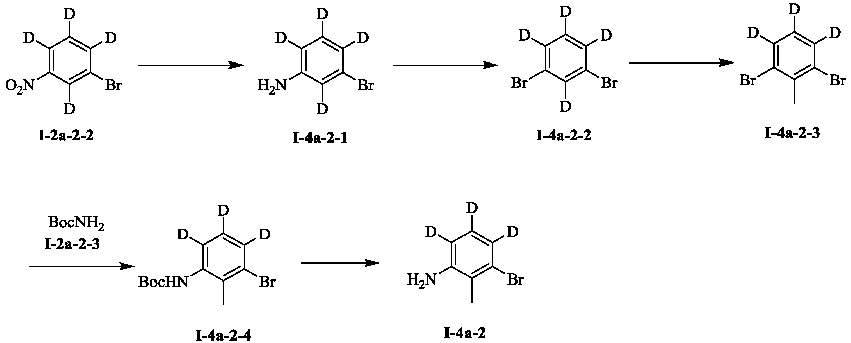

The previously prepared I-2a-2-2 (11.00 g, 53.3 mmol, 1.0 eq) was dissolved in a mixed solvent of EtOH (120 mL) and water (90 mL). Iron powder (17.80 g, 320.0 mmol , 6.0 eq) and NH₄Cl (17.10 g, 320.0 mmol, 6.0 eq) were added. The reaction solution was stirred at 60°C for 16 h. The reaction was monitored to be completed by TLC. The reaction mixture was filtered by suction, concentrated, washed with EtOAc (600 mLx3), and then concentrated to obtain a yellow oil I-4a-2-1. (45.0 g, yield 78.0%).

I-4a-2-1 (19.00 g, 107.0 mmol, 1.0 eq), levocamphorsulfonic acid (30.00 g, 129.0 mmol, 1.2 eq), and tert-butyl nitrite (13.3 g, 129.0 mmol, 1.2 eq) were added to a solution of TBAB (69.5 g, 215.0 mmol, 2.0 eq) and CuBr₂ (241.00 mg, 1.08 mmol, 0.01 eq) in acetonitrile (MeCN, 200 mL). The reaction mixture was stirred at 60°C for 3 h. The reaction was monitored to be completed by HPLC. The reaction solution was concentrated, adjusted to a pH of 8 with NaHCO₃ (500 mL) aqueous solution, and extracted with MTBE (100 mL × 3). The organic phases were combined, dried over anhydrous Na₂SO₄, and concentrated to obtain a crude product, which was distilled under reduced pressure (146°C, oil pump) to obtain a yellow liquid I-4a-2-2 (22.00 g, yield 42.4%).

In a three-necked flask, I-4a-2-2 (7.50 g, 31.2 mmol, 1.0 eq) and CH₃I (4.88 g, 34.3 mmol, 1.1 eq) were dissolved in anhydrous THF under argon protection. The reaction solution was cooled to -78°C, and slowly added with LDA (2M, 21.8 mL). The mixture was continuously stirred at this temperature for 1 h. After 71% product of the reaction was detected by HPLC, the reaction was quenched with neutral water, then extracted with EtOAc (300 mLx3), dried over anhydrous Na₂SO₄, and concentrated to obtain a red liquid I-4a-2-3 (8.70 g, crude product).

I-4a-2-3 (7.00 g, 27.6 mmol, 1.0 eq), Xantphos (1.60 g, 2.77 mmol, 0.1 eq), Cs₂CO₃ (18.00 g, 55.3 mmol, 2.0 eq), Pd₂(dpa)₃ (1.52 g, 1.7 mmol, 0.06 eq) and commercially available I-2a-2-3 were dissolved in toluene (60 mL). The mixture was stirred at 100°C for 16 h. The reaction was monitored to be completed by HPLC. The reaction solution was cooled to 25°C, diluted with water, and then extracted with EtOAc (100 mL × 2). The organic phases were combined and washed once with saturated brine. The organic phase was dried over anhydrous Na₂SO₄ and concentrated to obtain a crude product, which was separated by column chromatography (PE/EtOAc (v/v) = 98/1-2/1) to obtain a yellow solid I-4a-2-4. (3.80 g, 47.4%).

I-4a-2-4 (3.80 g, 13.1 mmol, 1.0 eq) was dissolved in EtOAc (10 mL), and then added with HCl/EtOAc (1.16 g, 13.1 mmol, 1.0 eq). The reaction solution was stirred at 40°C for 24 h. The reaction of the raw materials was monitored to be completed by HPLC. The reaction solution was filtered with suction. The filter cake was washed with EtOA (10 mL), and concentrated to obtain a white solid, which is hydrochloride salt of I-4a-2. (2.77 g, yield 93.4%). ¹H-NMR (400MHz, CDCl₃) *δ* ppm 2.28 (s, 3H).

### Preparation of intermediate tert-butyl 2-((2-chloro-3-(4,4,5,5-tetramethyl-1,3,2-dioxaborolan-2-yl)phenyl)carbamoyl)-1-methyl-1,4,6,7 -tetrahydro-5H-imidazo[4, 5-c]pyridine-5-carboxylate I-1b

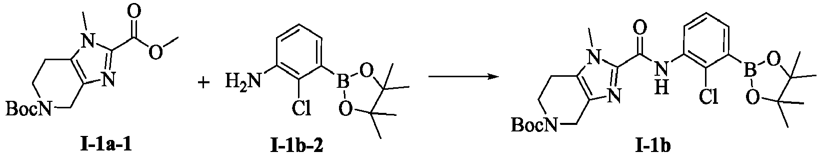

Commercially available I-1a-1 (1.41 g, 4.77 mmol, 1.0 eq) and commercially available I-1b-2 (1.21 g, 4.77 mmol, 1.0 eq) were dissolved in anhydrous THF (30 mL). The mixture was stirred for 5 min, and then a solution of KOBu-t (1.61 g, 14.31 mmol, 3.0 eq) in anhydrous THF (20 mL) was added. The mixture was stirred at ambient temperature for 1 h. The reaction was quenched with saturated brine (200 mL). The reaction mixture was extracted twice with EtOAc (200 mL). The organic phases were combined and dried over anhydrous Na₂SO₄. The crude product was separated by silica gel column (n-Hep/EtOAc (v/v) = 3/1) to obtain a white solid I-1b. (2.23 g, yield 90.5%). LC-MS MS-ESI (m/z) 517.2 [M+H]⁺.

### Preparation of intermediate tert-butyl 2-((2-chloro-3-(4,4,5,5-tetramethyl-1,3,2-dioxaborolan-2-yl)phenyl)carbamoyl)-1-methyl-d₃-1,4, 6,7-tetrahydro-5H-imidazo[4,5-c]pyridine-5-carboxylate I-2b

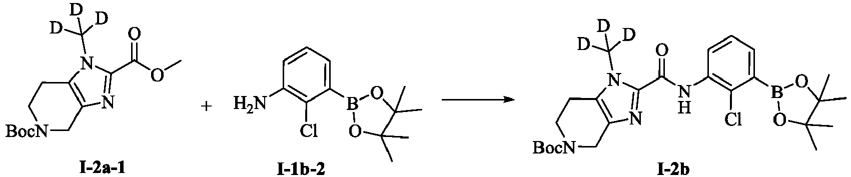

The previously prepared I-2a-1 (700.00 mg, 2.35 mmol, 1.0 eq), I-1b-2 (595.73 mg, 2.35 mmol, 1.0 eq), KOBu-*t* (659.18 mg, 5.88 mmol, 2.5 eq), and THF (40 mL) were stirred at 0°C for 0.5 h. The reaction solution was diluted with EtOAc (60 mL), and washed once with saturated brine. The organic phase was dried over anhydrous Na₂SO₄ and concentrated to obtain a crude product, which was separated by column chromatography (PE/EtOAc (v/v) = 5/1) to obtain a white solid I-2b. (460.00 mg, yield 35.0%). LC-MS MS-ESI (m/z) 520.2 [M+H]⁺.

### Preparation of intermediate 2-chloro-3-(4,4,5,5-tetramethyl-1,3,2-dioxaborolan-2-yl) phenyl-4,5,6-d₃-amine 1-3b-2

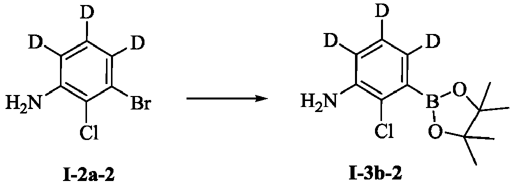

A mixture of the previously prepared 1-2a-2 (2.09 g, 10.0 mmol, 1.0 eq), bis(pinacolato)diboron (3.81 g, 15.0 mmol, 1.5 eq), Pd(dppf)Cl₂·CH₂Cl₂ (408.32 mg, 0.5 mmol, 0.05 eq), anhydrous potassium acetate (2.94 g, 30.0 mmol, 3.0 eq) and 1,4-dioxane (50 mL) was heated to 100°C under N₂ protection, stirred for 3 h, and cooled to ambient temperature. The reaction solution was concentrated. The crude product was separated by silica gel column chromatography (EtOAc/hexane (v/v) = 1/4) to obtain a white solid I-3b-2. (1.83 g, yield 71.3%). LC-MS MS-ESI (m/z) 257.2 [M+H]⁺.

### Preparation of intermediate tert-butyl 2-((2-chloro-3-(4,4,5,5-tetramethyl-1,3,2-dioxaborolan-2-yl)phenyl-4,5,6-d₃)carbamoyl)-1-(meth yl-d₃)-1,4,6,7-tetrahydro-5H-imidazo[4,5-c]pyridine-5-carboxylate I-3b

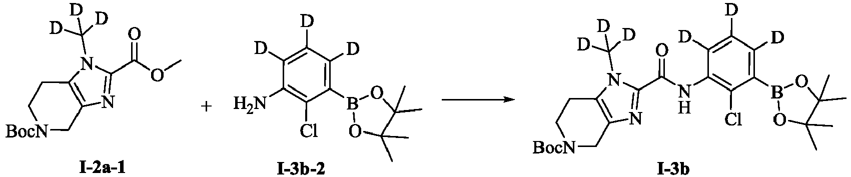

The previously prepared I-3b-2 (1.28 g, 5.0 mmol, 1.0 eq) and I-2a-1 (1.49 g, 5.0 mmol, 1.0 eq) were dissolved in anhydrous THF (50 mL), and added with KOBu-*t* ( 1.12 g, 10.0 mmol, 2.0 eq). The mixture was stirred at room temperature under N₂ protection for 1 h. The reaction was quenched with saturated brine (100 mL). The reaction mixture was extracted twice with EtOAc (80 mL). The organic phases were combined and dried over anhydrous Na₂SO₄. The crude product was separated by silica gel column chromatography (EtOAc/hexane (v/v) = 1/3) to obtain an off-white solid I-3b. (660.00 mg, yield 25.2%). LC-MS MS-ESI (m/z) 523.3 [M+H]⁺.

### Preparation of intermediate tert-butyl 2-((2-fluoro-3-(4,4,5,5-tetramethyl-1,3,2-dioxaborolan-2-yl)phenyl)carbamoyl)-1-(methyl-d₃)-1,4 ,6,7-tetrahydro-5H-imidazo[4,5-c]pyridine-5-carboxylate I-7b

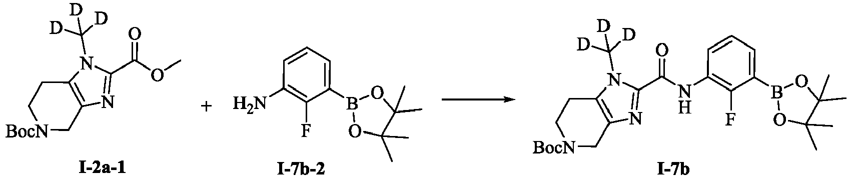

Commercially available 1-7b-2 (1.00 g, 4.20 mmol, 1.0 eq), the previously prepared intermediate I-2a-1 (1.24 g, 4.20 mmol, 1.0 eq), and KOBu-*t* (935.71 mg, 8.40 mmol, 2.0 eq) were subjected to a procedure similar to that of preparing the intermediate 1-3b to obtain a white solid intermediate I-7b. (1.00 g, yield 47.2%). LC-MS MS-ESI (m/z) 504.3 [M+H]⁺.

### Preparation of intermediate tert-butyl 1-(methyl-d₃)-2-((2-methyl-3-(4,4,5,5-tetramethyl-1,3,2-dioxaborolan-2-yl)phenyl)carbamoyl)-1-(methyl-d₃)-1,4,6,7-tetrahydro-5H-imidazo[4,5-c]pyridine-5-carboxylate I-9b

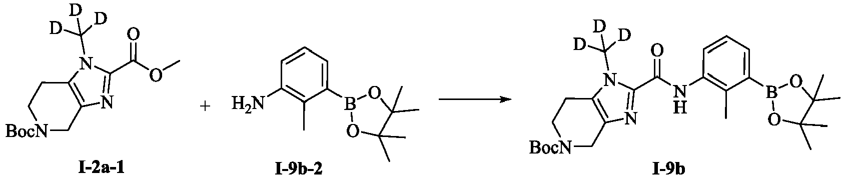

Commercially available 1-9b-2 (1.50 g, 6.44 mmol, 1.0 eq), the previously prepared intermediate I-2a-1 (1.90 g, 4.44 mmol, 1.0 eq), and KOBu-t (1.44 g, 12.88 mmol, 2.0 eq) were subjected to a procedure similar to that of preparing the intermediate 1-3b to obtain a white solid intermediate I-9b. (1.80 g, yield 56.0%). LC-MS MS-ESI (m/z) 500.3 [M+H]⁺.

### Preparation of intermediate 2-fluoro-3-(4,4,5,5-tetramethyl-1,3,2-dioxaborolan-2-yl)phenyl-4,5,6-d₃-amine 1-14b-2

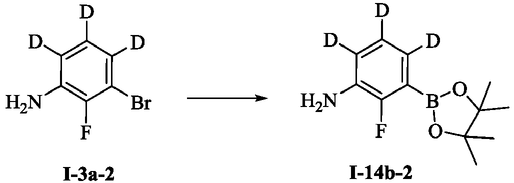

The previously prepared intermediate I-3a-2 (1.00 g, 5.2 mmol, 1.0 eq), bis(pinacolato)diboron (1.97 g, 7.77 mmol, 1.5 eq), Pd(dppf)Cl₂•CH₂Cl₂ (409.11 mg, 0.5 mmol, 0.1 eq), and anhydrous potassium acetate (1.52 g, 30.2 mmol, 3.0 eq) were subjected to a procedure similar to that of preparing the intermediate I-3b-2 to obtain a white solid 1-14b-2. (880.00 mg, yield 70.5%). LC-MS MS-ESI (m/z) 241.2 [M+H]⁺.

### Preparation of intermediate tert-butyl 2-((2-fluoro-3-(4,4,5,5-tetramethyl-1,3, 2-dioxaborolan-2-yl)phenyl-4,5,6-d₃)carbamoyl)-1-methyl-1,4,6,7-tetrahydro-5H-imidazo[4,5-c] pyridine-5-carboxylate I-14b

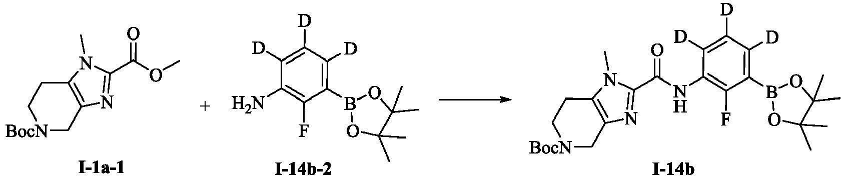

The previously prepared intermediate I-14b-2 (880.00 mg, 3.65 mmol, 1.0 eq), commercially available intermediate I-1a-1 (1.08 g, 3.65 mmol, 1.0 eq), and KOBu-t (813.66 mg, 7.30 mmol, 2.0 eq) were subjected to a procedure similar to that of preparing the intermediate I-3a to obtain a white solid intermediate I-14b. (1.00 g, yield 54.4%). LC-MS MS-ESI (m/z) 504.3 [M+H]⁺.

### Preparation of intermediate 2-(4-((methoxy-d₃)methyl)bicyclo[2.2.1]heptan-1-yl)acetaldehyde I-1d

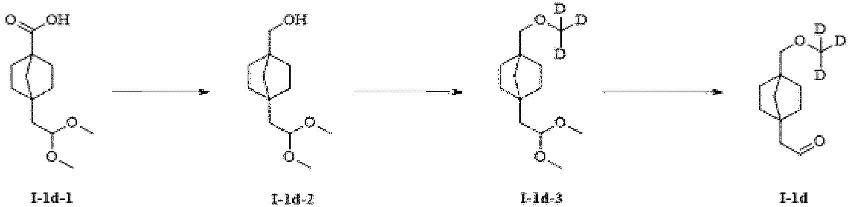

Commercially available I-1d-1 (300.0 g, 1.31 mol, 1.0 eq) was dissolved in THF (2.5 L). The reaction solution was cooled to 0°C under the protection of nitrogen, and dropwise added with BH₃-Me₂S (1.57 mol, 157.0 mL, 1.2 eq). After the dropwise addition was completed, the reaction solution was naturally warmed up to 20°C, and continued to be stirred for 16 h. TLC showed the reaction was completed. The reaction solution was cooled to 0°C, and MeOH (500 mL) was added dropwise to quench the reaction. After the dropwise addition was completed, the reaction solution was directly concentrated to dryness to obtain a colorless oily substance I-1d-2. (280.00 g, yield 99.4%). ¹H-NMR (400MHz, CDCl₃) *δ* ppm 4.48 (t, *J* = 5.5 Hz, 1H), 3.82 (s, 2H), 3.32 (s, 6H), 1.81 (d, 2H), 1.32-1.58 (m, 10H).

I-1d-2 (20.00 g, 93.3 mmol, 1.0 eq) was dissolved in DMF (150 mL), and added with silver oxide (Ag₂O, 64.88 g, 280.0 mmol, 3.0 eq) and deuteroiodomethane (CD₃I, 50.00 g, 344.9 mmol, 3.7 eq). After the addition was completed, the reaction system was covered with a balloon to prevent CD₃I from escaping, and stirred at 20°C for 32 h. The reaction was monitored by gas phase to have 30% raw material remaining, and was directly processed. The reaction solution was filtered to remove insoluble matter. The filtrate was added with water (150 mL), extracted with methyl tert-butyl ether (MTBE, 300 mLx2), washed with saturated brine (100 mLx3), dried over anhydrous magnesium sulfate, and filtered. The filtrate was desolvated under reduced pressure. The crude product was separated by silica gel column chromatography (n-Hep/EtOAc (v/v)=100/1-30/1) to obtain a yellow oil I-1d-3. (14.00 g, yield 64.8%). ¹H-NMR (400MHz, CDCl₃) *δ* ppm 4.47 (t, *J* = 5.5 Hz, 1H), 3.42 (s, 2H), 3.31 (s, 6H), 1.81 (d, *J* = 5.5 Hz, 2H), 1.22-1.60 (m, 10H).

I-1d-3 (14.00 g, 60.5 mmol, 1.0 eq) was dissolved in acetone (50 mL), and added with 3 M hydrochloric acid (60.52 mL, 3.0 eq). The reaction solution was stirred at 20°C for 16 h. TLC showed the reaction was completed. The reaction mixture was concentrated to remove acetone, then added with water, and extracted with MTBE (100 mLx3). The organic phases were combined, washed with saturated brine (50 mL), and dried over anhydrous magnesium sulfate. The crude product was separated by silica gel column chromatography (n-Hep/ EtOAc (v/v) = 100/1-30/1) to obtain a colorless liquid I-1d. (7.47 g, 66.6%). ¹H-NMR (400MHz, CDCl₃) *δ* ppm 9.81 (t, 1H), 3.43 (s, 2H), 2.57 (d, 2H), 1.35-1.65 (m, 10H).

### Preparation of intermediate 2-(4-((methoxy)methyl)bicyclo[2.2.1]heptan-1-yl)acetaldehyde I-2d

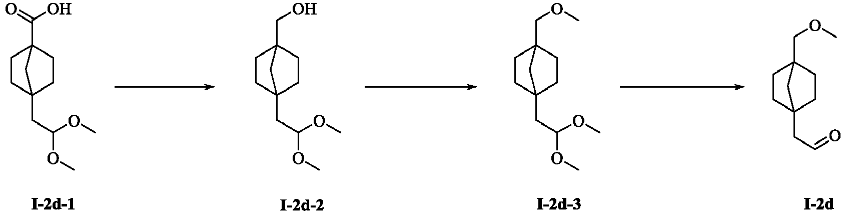

Commercially available I-2d-1 (300.00 g, 1.31 mol, 1.0 eq) was dissolved in THF (2.5 L). The reaction solution was cooled to 0°C under the protection of nitrogen, and dropwise added with a complex of dimethyl sulfide of borane (1.57 mol, 157 mL, 1.2 eq). After the dropwise addition was completed, the reaction solution was naturally warmed up to 20°C, and continued to be stirred for 16 h. TLC showed the reaction was completed. The reaction solution was cooled to 0°C, and dropwise added with MeOH (500 mL) to quench the reaction. After the dropwise addition was completed, the reaction solution was directly concentrated to dryness to obtain a colorless oily substance I-2d-2 (280.00 g, yield 99.4%). ¹H-NMR (400MHz, CDCl₃) *δ* ppm 4.48 (t, *J* = 5.5 Hz, 1H), 3.82 (s, 2H), 3.32 (s, 6H), 1.81 (d, 2H), 1.32-1.58 (m, 10H).

I-2d-2 (5.00 g, 23.33 mmol, 1.0 eq) was dissolved in N, N'-dimethylformamide (50 mL), and subsequently added with iodomethane (33.12 g, 233.30 mmol, 10.0 eq) and Ag₂O (16.22 g, 70.00 mmol, 3.0 eq). The reaction solution was stirred at 20°C for 16 h. TLC showed a small amount of raw material remaining and a major point was formed. The reaction solution was poured into water (50 mL) and extracted with MTBE (50 mLx3). The organic phases were combined, washed with saturated brine (30 mLx3), and dried over anhydrous magnesium sulfate. The crude product was separated by silica gel column chromatography (n-Hep/EtOAc(v/v)=100/1-10/1) to obtain a colorless oil I-2d-3 (4.50 g, yield 84.5%). ¹H-NMR (400MHz, CDCl₃) *δ* ppm 4.47 (t, *J* = 5.5 Hz, 1H), 3.37 (s, 3H), 3.42 (s, 2H), 3.30 (s, 6H), 1.82 (d, *J* = 5.5 Hz, 2H), 1.22-1.60 (m, 10H).

I-2d-3 (4.50 g, 19.71 mmol, 1.0 eq) was dissolved in acetone (40 mL), and added with 2M hydrochloric acid (29.56 mL, 3.0 eq). The reaction solution was stirred at 25 °C for 4 h. TLC showed the reaction was completed. The reaction solution was concentrated to remove acetone, added with water (30 mL), and extracted with MTBE (20 mLx3). The organic phases were combined, washed with saturated brine (30 mL), and dried over anhydrous magnesium sulfate. The crude product was separated by silica gel column chromatography (n-Hep/ EtOAc (v/v) = 100/1-10/1) to obtain a colorless liquid I-2d (2.31 g, yield 64.3%). ¹H-NMR (400MHz, CDCl₃) *δ* ppm 9.81 (t, 1H), 3.43 (s, 2H), 3.41 (s, 3H), 2.56 (d, 2H), 1.39-1.68 (m, 10H).

### Example 1

### 4-(2-(2-((2,2'-dichloro-3'-(5-(2-(4-((methoxy-d₃)methyl)bicyclo[2.2.1]heptane-1-yl)eth yl)-1-methyl-4,5,6,7-tetrahydro-1H-imidazo[4,5-c]pyridine-2-carboxamido)-[1,1'-biphenyl]-3-yl) carbamoyl)-1-methyl-1,4,6,7-tetrahydro-5H-imidazo[4,5-c]pyridin-5-yl)ethyl)bicyclo[2.2.1]hepta ne-1-carboxylic acid 1-1

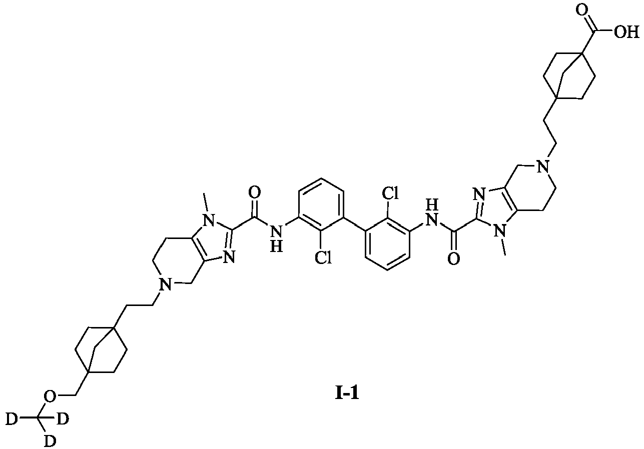

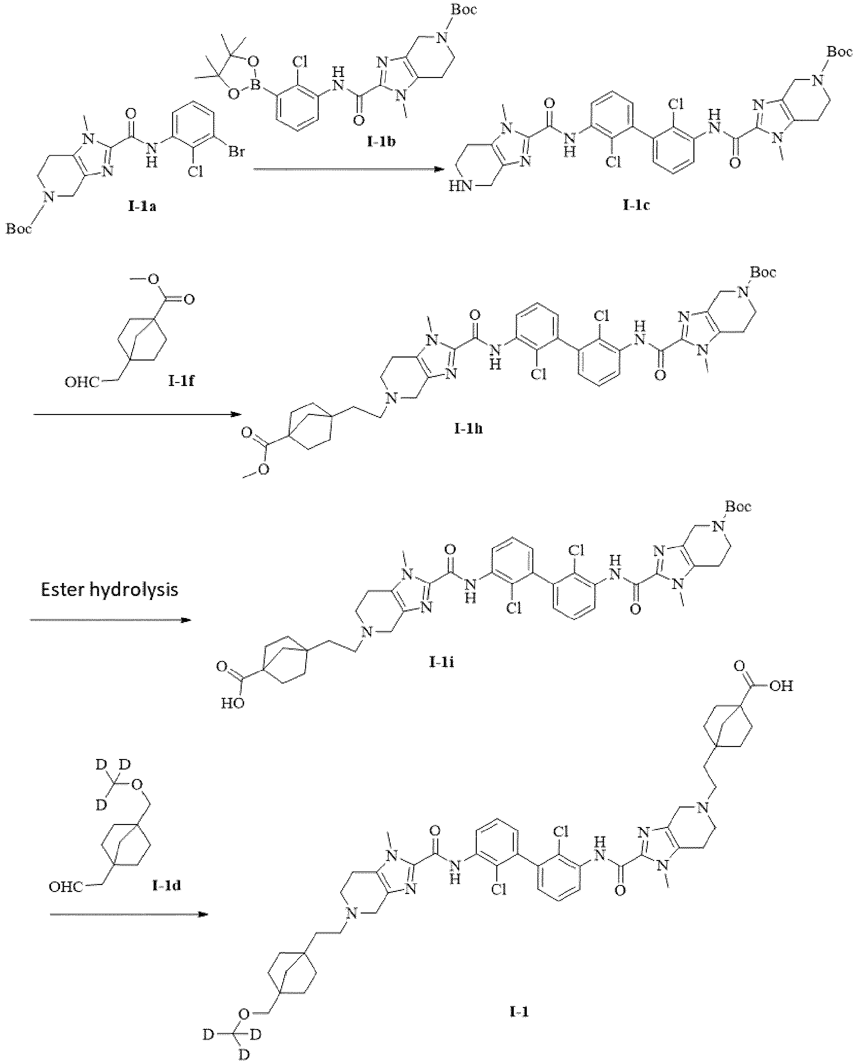

### Preparation of intermediate: tert-butyl 2-((2,2'-dichloro-3'-(1-methyl-4,5,6,7-tetrahydro-1H-imidazo[4,5-c]pyridine-2-carboxamido)-[1, 1'-biphenyl]-3-yl)carbamoyl)-1-methyl-1,4,6,7-tetrahydro-5H-imidazo[4,5-c]pyridine-5-carboxyl ate I-1c

Tert-butyl 2-((3-bromo-2-chlorophenyl)carbamoyl)-1-methyl-1,4,6,7-tetrahydro-5H-imidazo[4,5-c]pyridine-5-carboxylate I-1a (530.0 mg, 1.13 mmol, 1.0 eq) was dissolved in DCM (10 mL), and added with TFA (10 mL). The mixture was stirred at ambient temperature for 1 h. The reaction solution was concentrated. The residue was dissolved in 1,4-dioxane (10 mL), and added with tert-butyl 2-((2-chloro-3-(4,4,5,5-tetramethyl-1,3,2-dioxaborolan-2-yl)phenyl) carbamoyl)-1-methyl-1,4,6,7-tetrahydro-5H-imidazo[4,5-c]pyridine-5-carboxylate I-1b (583.08 mg, 1.13 mmol, 1.0 eq), PdCl₂(dcypf) (83.05 mg, 0.11 mmol, 0.1 eq), anhydrous Na₂CO₃ (359.34 mg, 3.39 mmol, 3.0 eq) and water (5 mL). The resulting mixture was heated to 110°C by microwave for 1 h of reaction, and then cooled to ambient temperature. The reaction was quenched with water (100 mL). The reaction mixture was extracted 3 times with DCM (100 mL). The organic phases were combined and concentrated. The crude product was separated by silica gel column chromatography (DCM/MeOH (v/v) = 10/1) to obtain a yellow solid I-1c. (363.0 mg, yield 47.3%). LC-MS MS-ESI (m/z) 679.2 [M+H]⁺.

### Preparation of intermediate: tert-butyl 2-((2,2'-dichloro-3'-(5-(2-(4-(methoxycarbonyl)bicycle [2.2.1]heptan-1-yl)ethyl)-1-methyl-4,5,6,7-tetrahydro-1H-imidazo[4,5-c]pyridine-2-carboxamido )-[1,1'-biphenyl]-3-yl)carbamoyl)-1-methyl-1,4,6,7-tetrahydro-5H-imidazo[4,5-c]pyridine-5-carb oxylate I-1h

I-1c (2.40 g, 3.53 mmol, 1.0 eq) was dissolved in DCM (60 mL), and added with TEA (3 mL) and methyl 4-(2-oxoethyl)bicyclo[2.2.1]heptane-1-carboxylate I-1f (1.04 g, 5.29 mmol, 1.5 eq). The resulting mixture was stirred at ambient temperature for 1 h, added with NaBH(OAc)₃ (4.49 g, 21.18 mmol, 6.0 eq), and continued to be stirred for 16 h. The reaction solution was quenched with saturated NaHCO₃ solution and extracted 3 times with DCM/MeOH (10/1, 300 mL). The organic phases were combined, dried over anhydrous Na₂SO₄ and concentrated. The crude product was separated by silica gel column chromatography (DCM/MeOH (v/v) = 20/1) to obtain a yellow solid I-1h. (2.76 g, yield 92.1%). LC-MS MS-ESI (m/z) 859.3 [M+H]⁺.

### Preparation of intermediate: 4-(2-(2-((3'-(5-(tert-butoxycarbonyl)-1-methyl-4,5,6,7-tetrahydro -1H-imidazo[4,5-c]pyridine-2-carboxamido)-2,2'-dichloro-[1,1'-biphenyl]-3-yl)carbamoyl)-1-me thyl-1,4,6,7-tetrahydro-5H-imidazo[4,5-c]pyridin-5-yl)ethyl)bicyclo[2.2.1]heptane-1-carboxylic acid I-1i

Intermediate I-1h (2.67 g, 3.11 mmol, 1.0 eq) was dissolved in THF (300 mL), and added with aqueous solution (300 mL) of lithium hydroxide monohydrate (LiOH•H₂O, 2.61 g, 62.20 mmol, 20.0 eq) in one go. The resulting mixture was stirred at ambient temperature for 16 h, concentrated to remove THF, and adjusted to a pH of 5-6 with 1 M dilute hydrochloric acid. The mixture was filtered to collect the solid, which was dried to obtain a light yellow solid I-1i. (1.84 g, yield 69.9%). LC-MS MS-ESI (m/z) 845.3 [M+H]⁺.

### Preparation of compound: 4-(2-(2-((2, 2'-dichloro-3'-(5-(2-(4-((methoxy-d₃)methyl)bicyclo[2.2.1] heptane-1-yl)ethyl)-1-methyl-4,5,6,7-tetrahydro-1H-imidazo[4,5-c]pyridine-2-carboxamido)-[1,1 '-biphenyl]-3-yl)carbamoyl)-1-methyl-1,4,6,7-tetrahydro-SH-imidazo[4,5-c]pyridin-5-yl)ethyl)bi cyclo[2.2.1]heptane-1-carboxylic acid 1-1

Intermediate 1-1i (1.84 g, 2.17 mmol, 1.0 eq) was dissolved in DCM (30 mL), and added with TFA (30 mL). The resulting solution was stirred at ambient temperature for 1 h. The reaction solution was concentrated. The residue was dissolved in DCM (60 mL) and concentrated again to obtain a yellow solid that was used directly in the next stage. The resulting trifluoroacetate salt was dissolved in DCM (60 mL), and added with TEA (3 mL) and 2-(4-((methoxy-*d*₃)methyl)bicyclo[2.2.1]heptane-1-yl)acetaldehyde I-1d (602.23 mg, 3.25 mmol, 1.5 eq). After the resulting mixture was stirred at ambient temperature for 1 h, NaBH(OAc)₃ (2.76 g, 13.02 mmol, 6.0 eq) was added. The mixture was continued to be stirred for 16 h. The reaction solution was quenched with saturated NaHCO₃ solution and extracted 3 times with DCM/MeOH (10/1, extracted 3 times with 300 mL). The organic phases were combined, dried over anhydrous Na₂SO₄ and concentrated. The crude product was separated by silica gel column chromatography (DCM/MeOH (v/v) = 15/1) to obtain an off-white solid I-1. (1.24 g, yield 62.4%). LC-MS MS-ESI (m/z) 914.5 [M+H]⁺, ¹H-NMR (400MHz, DMSO-*d*₆) *δ* ppm 12.00 (s, 1H), 9.90 (s, 2H), 8.37 (d, *J* = 8.2 Hz, 2H), 7.49 (t, *J* = 7.9 Hz, 2H), 7.14 (d, *J* = 7.5 Hz, 2H), 3.90 (s, 6H), 3.46-3.43 (m, 4H), 3.32 (s, 2H), 2.80 (s, 4H), 2.68 (s, 4H), 2.58 (s, 4H), 1.88-1.81 (m, 2H), 1.73-1.69 (m, 4H), 1.57-1.21 (m, 16H), 1.12 (s, 2H).

### Example 2

### 4-(2-(2-((2-chloro-2'-fluoro-3'-(5-(2-(4-((methoxy-d₃)methyl)bicyclo[2.2.1]heptane-1-y 1)ethyl)-1-methyl-4,5,6,7-tetrahydro-1H-imidazo[4,5-c]pyridine-2-carboxamido)-[1,1'-biphenyl]-3-yl)carbamoyl)-1-methyl-1,4,6,7-tetrahydro-5H-imidazo[4,5-c]pyridin-5-yl)ethyl)bicyclo[2.2.1] heptane-1-carboxylic acid I-2

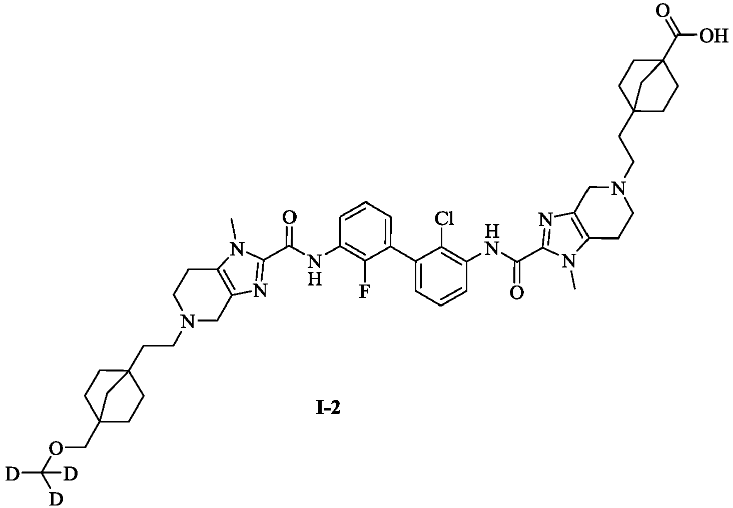

### Preparation of compound: 4-(2-(2-((2-chloro-2'-fluoro-3'-(5-(2-(4-((methoxy-d₃)methyl) bicyclo[2.2.1]heptane-1-yl)ethyl)-1-methyl-4,5,6,7-tetrahydro-1H-imidazo[4,5-c]pyridine-2-carb oxamido)-[1,1'-biphenyl]-3-yl)carbamoyl)-1-methyl-1,4,6,7-tetrahydro-5H-imidazo[4,5-c]pyridi n-5-yl)ethyl)bicyclo[2.2.1]heptane-1-carboxylic acid I-2

The intermediate 4-(2-(2-((3'-(5-(tert-butoxycarbonyl)-1-methyl-4,5,6,7-tetrahydro-1H -imidazo[4,5-c]pyridine-2-carboxamido)-2-chloro-2'-fluoro-[1,1'-biphenyl]-3-yl)carbamoyl)-1-methyl-1,4,6,7-tetrahydro-5H-imidazo[4,5-c]pyridin-5-yl)ethyl)bicyclo[2.2.1]heptane-1-carboxyl ic acid I-2i (400.00 mg, 0.48 mmol, 1.0 eq, synthesized according to the preparation of intermediate I-1i), TFA (5 mL), TEA (1 mL), 2-(4-((methoxy-*d*₃)methyl) bicyclo[2.2.1]heptan-1-yl)acetaldehyde I-1d (133.20 mg, 0.72 mmol, 1.5 eq) and NaBH(OAc)₃ (610.56 mg, 2.88 mmol, 6.0 eq) were subjected to a procedure similar to that of preparing compound I-1 to obtain a pale yellow solid I-2. (95.00 mg, yield 22.0%). LC-MS MS-ESI (m/z) 898.5 [M+H]⁺. ¹H-NMR (400MHz, DMSO-*d*₆) *δ* ppm 9.93 (s, 1H), 9.78 (s, 1H), 8.34 (d, *J* = 8.3 Hz, 1H), 8.07 (t, *J* = 7.3 Hz, 1H), 7.48 (t, *J* = 7.9 Hz, 1H), 7.31 (t, *J* = 7.9 Hz, 1H), 7.21 (d, *J =* 7.5 Hz, 1H), 7.16 (t, *J* = 6.7 Hz, 1H), 3.89 (s, 3H), 3.87 (s, 3H), 3.41-3.39 (m, 4H), 3.32 (s, 2H), 2.78-2.71 (m, 4H), 2.68-2.60 (m, 4H), 2.56-2.51 (m, 4H), 1.90-1.81 (m, 2H), 1.72-1.69 (m, 4H), 1.59-1.19 (m, 16H), 1.11 (s, 2H).

### Example 3

### 4-(2-(2-((2'-chloro-3'-(5-(2-(4-((methoxy-d₃)methyl)blcyclo[2.2.1]heptane-1-yl)ethyl)-1-methyl-4,5,6,7-tetrahydro-1H-imidazo[4,5-c]pyridine-2-carboxamido)-2-methyl-[1,1'-biphenyl ]-3-yl)carbamoyl)-1-methyl-1,4,6,7-tetrahydro-5H-imidazo[4,5-c]pyridin-5-yl)ethyl)bicyclo[2.2. 1]heptane-1-carboxylic acid I-3

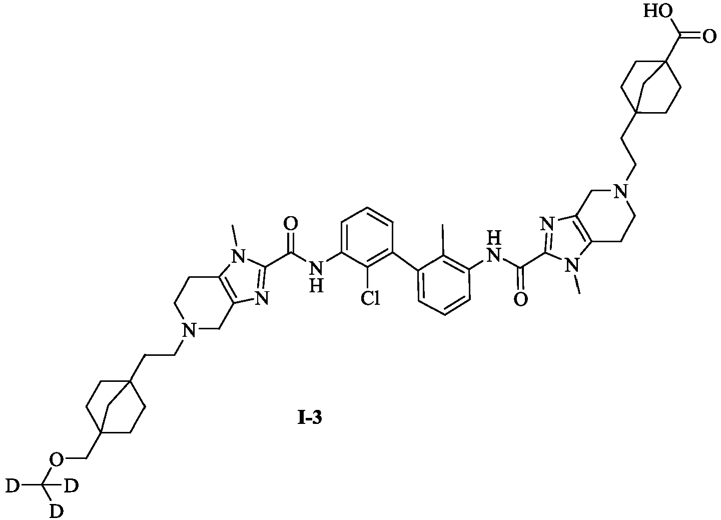

### Preparation of compound: 4-(2-(2-((2'-chloro-3'-(5-(2-(4-((methoxy-d₃)methyl)bicyclo[2.2.1]heptane-1-yl)ethyl)-1-methyl-4,5,6,7-tetrahydro-1H-imidazo[4,5-c]pyridine-2-carboxamido)-2-methyl-[1,1'-biphenyl]-3-yl)car bamoyl)-1-methyl-1,4,6,7-tetrahydro-5H-imidazo[4,5-c]pyridin-5-yl)ethyl)bicyclo[2.2.1]heptane-1-carboxylic acid I-3

The intermediate 4-(2-(2-((3'-(5-(tert-butoxycarbonyl)-1-methyl-4,5,6,7-tetrahydro-1H -imidazo[4,5-c]pyridine-2-carboxamido)-2'-chloro-2-methyl-[1,1'-biphenyl]-3-yl)carbamoyl)-1-methyl-1,4,6,7-tetrahydro-5H-imidazo[4,5-c]pyridin-5-yl)ethyl)bicyclo[2.2.1]heptane-1-carboxyl ic acid I-3i (400.00 mg, 0.48 mmol, 1.0 eq, synthesized according to the preparation of intermediate I-1i), TFA (5 mL), TEA (1 mL), 2-(4-((methoxy-*d*₃)methyl)bicyclo[2.2.1]heptan-1-yl)acetaldehyde I-1d (133.2 mg, 0.72 mmol, 1.5 eq) and NaBH(OAc)₃ (610.56 mg, 2.88 mmol, 6.0 eq) were subjected to a procedure similar to that of preparing compound I-1 to obtain a pale yellow solid I-3. (71.00 mg, yield 16.5%). LC-MS MS-ESI (m/z) 894.5 [M+H]⁺. ¹H-NMR (400MHz, DMSO-*d*₆) *δ* ppm 9.90 (s, 1H), 9.73 (s, 1H), 8.34 (d, *J* = 7.1 Hz, 1H), 7.73 (d, *J* = 7.9 Hz, 1H), 7.45 (t, *J* = 7.9 Hz, 1H), 7.29 (t, *J* = 7.8 Hz, 1H), 7.06 (d, *J* = 6.5 Hz, 1H), 7.00 (d, *J* = 7.5 Hz, 1H), 3.89 (s, 3H), 3.86 (s, 3H), 3.40 (d, *J* = 3.8 Hz, 4H), 3.32 (s, 2H), 2.79-2.70 (m, 4H), 2.68-2.61 (m, 4H), 2.56-2.50 (m, 4H), 1.98 (s, 3H), 1.91-1.80 (m, 2H), 1.73-1.67 (m, 4H), 1.58-1.17 (m, 16H), 1.11 (s, 2H).

### Example 4

### 4-(2-(2-((2-chloro-3'-(5-(2-(4-((methoxy-d₃)methyl)bicyclo[2.2.1]heptane-1-yl)ethyl)-1 -methyl-4,5,6,7-tetrahydro-1H-imidazo[4,5-c]pyridine-2-carboxamido)-2'-methyl-[1,1'-biphenyl] -3-yl)carbamoyl)-1-methyl-1,4,6,7-tetrahydro-5H-imidazo[4,5-c]pyridin-5-yl)ethyl)bicyclo[2.2.1 ]heptane-1-carboxylic acid I-4

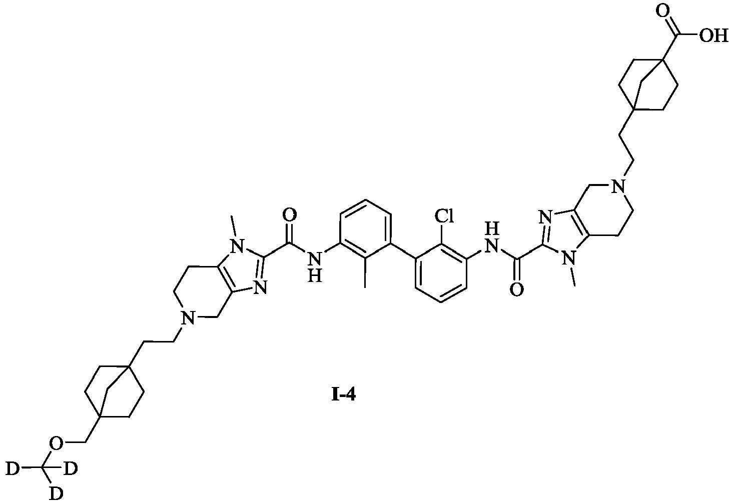

### Preparation of compound: 4-(2-(2-((2-chloro-3'-(5-(2-(4-((methoxy-d₃)methyl)bicycle [2.2.1]heptane-1-yl)ethyl)-1-methyl-4,5,6,7-tetrahydro-1H-imidazo[4,5-c]pyridine-2-carboxamid o)-2'-methyl-[1,1'-biphenyl]-3-yl)carbamoyl)-1-methyl-1,4,6,7-tetrahydro-5H-imidazo[4,5-c]pyr idin-5-yl)ethyl)bicyclo[2.2.1]heptane-1-carboxylic acid I-4

The intermediate 4-(2-(2-((3'-(5-(tert-butoxycarbonyl)-1-methyl-4,5,6,7-tetrahydro-1H -imidazo[4,5-c]pyridine-2-carboxamido)-2-chloro-2'-methyl-[1,1'-biphenyl]-3-yl)carbamoyl)-1 -methyl-1,4,6,7-tetrahydro-5H-imidazo[4,5-c]pyridin-5-yl)ethyl)bicyclo[2.2.1]heptane-1-carboxy lic acid I-4i (400.00 mg, 0.48 mmol, 1.0 eq, synthesized according to the preparation of intermediate I-1i), TFA (5 mL), TEA (1 mL), 2-(4-((methoxy-*d*₃)methyl)bicyclo[2.2.1]heptan-1-yl)acetaldehyde I-1d (133.2 mg, 0.72 mmol, 1.5 eq) and NaBH(OAc)₃ (610.56 mg, 2.88 mmol, 6.0 eq) were subjected to a procedure similar to that of preparing compound I-1 to obtain a pale yellow solid I-4. (64.00 mg, yield 14.9%). LC-MS MS-ESI (m/z) 894.5 [M+H]⁺. ¹H-NMR (400MHz, DMSO-*d*₆) *δ* ppm 9.90 (s, 1H), 9.72 (s, 1H), 8.35 (dd, *J* = 8.2, 1.4 Hz, 1H), 7.73 (d, *J* = 7.8 Hz, 1H), 7.45 (t, *J* = 7.9 Hz, 1H), 7.29 (t, *J* = 7.8 Hz, 1H), 7.06 (dd, *J* = 7.6, 1.5 Hz, 1H), 7.00 (d, *J* = 7.6 Hz, 1H), 3.89 (s, 3H), 3.86 (s, 3H), 3.40 (s, 4H), 3.32 (s, 2H), 2.78-2.70 (m, 4H), 2.69-2.60 (m, 4H), 2.56-2.50 (m, 4H), 1.98 (s, 3H), 1.91-1.80 (m, 2H), 1.71-1.67 (m, 4H), 1.58-1.19 (m, 16H), 1.12 (s, 2H).

### Example 5

### 4-(2-(2-((2,2'-dichloro-3'-(5-(2-(4-((methoxy-d₃)methyl)bicyclo[2.2.1]heptane-1-yl)eth yl)-1-(methyl-d₃)-4,5,6,7-tetrahydro-1H-imidazo[4,5-c]pyridine-2-carboxamido)-[1,1'-biphenyl]-3-yl)carbamoyl)-1-(methyl-d₃)-1,4,6,7-tetrahydro-5H-imidazo[4,5-c]pyridin-5-yl)ethyl)bicyclo[2. 2.1]heptane-1-carboxylic acid I-5

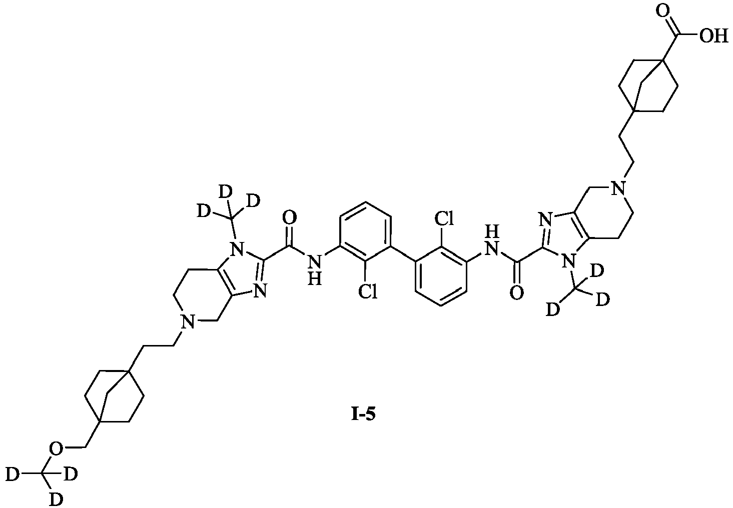

### Preparation of intermediate: tert-butyl 2-((2,2'-dichloro-3'-(1-(methyl-d₃)-4,5,6,7-tetrahydro-1H-imidazo[4,5-c]pyridine-2-carboxamido )-[1,1'-biphenyl]-3-yl)carbamoyl)-1-(methyl-d₃)-1,4,6,7-tetrahydro-5H-imidazo[4,5-c]pyridine-5-carboxylate I-2c

Tert-butyl 2-((3-bromo-2-chlorophenyl)carbamoyl)-1-(methyl-*d*₃)-1,4,6,7-tetrahydro-5H-imidazo[4,5-c]pyridine-5-carboxylate I-2a (1.10 g, 2.33 mmol, 1.0 eq) was dissolved in DCM (10 mL), and added with TFA (10 mL). The mixture was stirred at ambient temperature for 30 min. The reaction solution was concentrated. The residue was dissolved in 1,4-dioxane (10 mL), and tert-butyl 2-((2-chloro-3-(4,4,5,5-tetramethyl-1,3,2-dioxaborolan-2-yl)phenyl)carbamoyl)-1-(methyl-*d*₃)-1, 4,6,7-tetrahydro-5H-imidazo[4,5-c]pyridine-5-carboxylate I-2b (950.00 mg, 1.82 mmol, 1.0 eq), PdCl₂ (dcypf) (137.51 mg, 0.18 mmol, 0.1 eq), anhydrous Na₂CO₃ (866.40 mg, 8.19 mmol, 4.5 eq) and water (5 mL) were added. The mixture was subjected to a procedure similar to that of preparing intermediate I-1c to obtain a white solid I-2c. (620.00 mg, yield 49.7%). LC-MS MS-ESI (m/z) 685.4 [M+H]⁺.

### Preparation of intermediate: tert-butyl 2-((2, 2'-dichloro-3'-(5-(2-(4-(methoxycarbonyl)bicyclo[2.2.1]heptan-1-yl)ethyl)-1-(methyl-d₃)-4, 5, 6, 7-tetrahydro-1H-imidazo[4,5-c]pyridine-2-carboxamido)-[1,1'-biphenyl]-3-yl)carbamoyl)-1-(met hyl-d₃)-1,4,6,7-tetrahydro-5H-imidazo[4,5-c]pyridine-5-carboxylate I-2h

I-2c (620.00 mg, 0.89 mmol, 1.0 eq) was dissolved in DCM (20 mL), and added with TEA (1 mL) and methyl 4-(2-oxoethyl)bicyclo[2.2.1]heptane-1-carboxylate I-1f (350.80 mg, 1.78 mmol, 2.0 eq). The resulting mixture was stirred at ambient temperature for 30 min, and added with NaBH(OAc)₃ (954.00 mg, 4.50 mmol, 5.0 eq). The mixture was subjected to a procedure similar to that of preparing intermediate I-1h to obtain a white solid I-2h. (490.00 mg, yield 63.6%). LC-MS MS-ESI (m/z) 865.4 [M+H]⁺.

### Preparation of intermediate: 4-(2-(2-((3'-(5-(tert-butoxycarbonyl)-1-(methyl-d₃)-4,5,6,7 -tetrahydro-1H-imidazo[4,5-c]pyridine-2-carboxamido)-2,2'-dichloro-[1,1'-biphenyl]-3-yl)carba moyl)-1-(methyl-d₃)-1,4,6,7-tetrahydro-5H-imidazo[4,5-c]pyridin-5-yl)ethyl)bicyclo[2.2.1]hepta ne-1-carboxylic acid I-2i

LiOH•H₂O (239.40 mg, 5.70 mmol) was weighed and dissolved in water (20 mL). Intermediate I-2h (490.00 mg, 0.57 mmol, 1.0 eq) was dissolved in THF (20 mL), and then added with the above lithium hydroxide solution. The mixture was stirred overnight at room temperature, then concentrated to remove THF, adjusted to a pH of 5-6 with 1 M dilute hydrochloric acid, and extracted twice with EtOAc (30 mL). The organic phases were combined, dried over anhydrous Na₂SO₄ and concentrated to obtain intermediate I-2i. (300.00 mg, yield 57.2%). LC-MS MS-ESI (m/z) 920.6 [M+H]⁺.

### Preparation of compound: 4-(2-(2-((2,2'-dichloro-3'-(5-(2-(4-((methoxy-d₃)methyl) bicyclo[2.2.1]heptane-1-yl)ethyl)-1-(methyl-d₃)-4,5,6,7-tetrahydro-1H-imidazo[4,5-c]pyridine-2-carboxamido)-[1,1'-biphenyl]-3-yl)carbamoyl)-1-(methyl-d₃)-1,4,6,7-tetrahydro-5H-imidazo[4,5-c]pyridin-5-yl)ethyl)bicyclo[2.2.1]heptane-1-carboxylic acid I-5

Intermediate I-2i (200.00 mg, 0.22 mmol, 1.0 eq) was dissolved in DCM (5 mL), and added with TFA (5 mL). The resulting solution was stirred at ambient temperature for 30 min. The reaction was concentrated. The residue was dissolved in DCM and concentrated again to obtain a yellow solid that was used directly in the next stage. The resulting trifluoroacetate was dissolved in DCM, and added with TEA (1 mL) and 2-(4-((methoxy-*d*₃)methyl)blcyclo[2.2.1]heptan-1-yl)acetaldehyde I-1d (119.90 mg, 0.44 mmol, 2.0 eq). The resulting mixture was stirred at ambient temperature for 1 h, added with NaBH(OAc)₃ (237.01 mg, 1.10 mmol, 5.0 eq), and continued to be stirred for 16 h. The reaction solution was quenched with water. The organic phase was separated, dried over anhydrous Na₂SO₄, and concentrated. The crude product was separated by preparative column (DCM/MeOH (v/v) = 10/1) to obtain 1-5. (105.00 mg, yield 51.8%). LC-MS MS-ESI (m/z) 920.5 [M+H]⁺. ¹H-NMR (400MHz, DMSO-*d*₆) *δ* ppm 9.89 (s, 2H), 8.38 (d, *J* = 7.5 Hz, 2H), 7.48 (t, *J=* 7.9 Hz, 2H), 7.14 (dd, *J* = 7.6 Hz, 1.4 Hz, 2H), 3.46-3.38 (m, 4H), 3.32 (s, 2H), 2.77-2.71 (m, 4H), 2.69-2.59 (m, 4H), 2.56-2.51 (m, 4H), 1.91-1.80 (m, 2H), 1.76-1.64 (m, 4H), 1.57-1.18 (m, 16H), 1.12 (s, 2H).

### Example 6

### 4-(2-(2-((2,2'-dichloro-3'-(5-(2-(4-((methoxy)methyl)bicyclo[2.2.1]heptane-1-yl)ethyl)-1-(methyl-d₃)-4,5,6,7-tetrahydro-1H-imidazo[4,5-c]pyridine-2-carboxamido)-[1,1'-biphenyl]-3-yl )carbamoyl)-1-(methyl-d₃)-1,4,6,7-tetrahydro-5H-imidazo[4,5-c]pyridin-5-yl)ethyl)bicyclo[2.2.1] heptane-1-carboxylic acid I-6

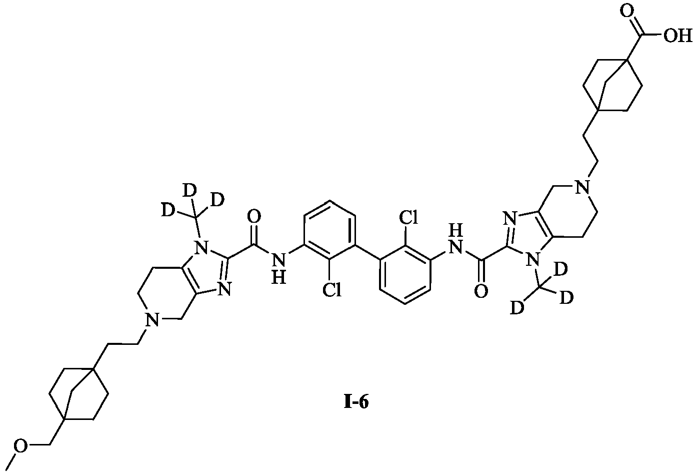

### Preparation of compound: 4-(2-(2-((2,2'-dichloro-3'-(5-(2-(4-((methoxy)methyl)bicycle [2.2.1]heptane-1-yl)ethyl)-1-(methyl-d₃)-4,5,6,7-tetrahydro-1H-imidazo[4,5-c]pyridine-2-carboxa mido)-[1,1'-biphenyl]-3-yl)carbamoyl)-1-(methyl-d₃)-1,4,6,7-tetrahydro-5H-imidazo[4,5-c]pyridi n-5-yl)ethyl)bicyclo[2.2.1]heptane-1-carboxylic acid I-6

Intermediate I-2i (100.00 mg, 0.11 mmol, 1.0 eq) was dissolved in DCM (5 mL), and added with TFA (5 mL) in one go. The mixture was stirred at room temperature for 30 min. The reaction solution was spin-dried to obtain a light yellow transparent oil. The light yellow transparent oil was dissolved in DCM, added with TEA (1 mL) and 2-(4-((methoxy)methyl)bicyclo[2.2.1]heptan-1-yl)acetaldehyde I-2d (60.00 mg, 0.22 mmol, 2eq), and stirred at room temperature for 30 min. The mixture was added with NaBH(OAc)₃ (166.5 mg, 0.55 mmol, 5.0 eq) and subjected to a procedure similar to that of preparing compound I-5 to obtain compound I-6. (50.00 mg, yield 49.5%). LC-MS MS-ESI (m/z) 917.5 [M+H]⁺. ¹H-NMR (400MHz, DMSO-*d*₆) *δ* ppm 9.89 (s, 2H), 8.38 (d, *J* = 7.8 Hz, 2H), 7.48 (t, *J* = 7.9 Hz, 2H), 7.14 (d, *J* = 6.5 Hz, 2H), 3.38-3.43 (m, 4H), 3.32 (s, 2H), 3.23 (s, 3H), 2.79-2.70 (m, 4H), 2.69-2.59 (m, 4H), 2.56-2.51 (m, 4H), 1.91-1.80 (m, 2H), 1.76-1.64 (m, 4H), 1.57-1.20 (m, 16H), 1.11 (s, 2H).

### Example 7

### 4-(2-(2-((2-chloro-2'-fluoro-3'-(5-(2-(4-((methoxy-d₃)methyl)bicyclo[2.2.1]heptane-1-y l)ethyl)-1-(methyl-d₃)-4,5,6,7-tetrahydro-1H-imidazo[4,5-c]pyridine-2-carboxamido)-[1,1'-biphe nyl]-3-yl)carbamoyl)-1-(methyl-d₃)-1,4,6,7-tetrahydro-5H-imidazo[4,5-c]pyridin-5-yl)ethyl)bicy clo[2.2.1]heptane-1-carboxylic acid I-7

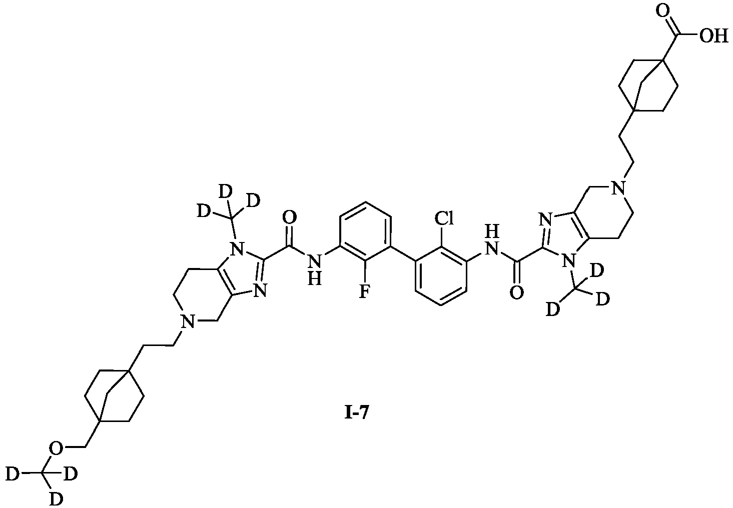

### Preparation of intermediate: tert-butyl2-((2'-chloro-2-fluoro-3'-(1-(methyl-d₃)-4,5,6,7-tetrahydro-1H-imidazo[4,5-c]pyridine-2-carboxamido)-[1,1'-biphenyl]-3-yl)carbamoyl)-1 -(methyl-d₃)-1,4,6,7-tetrahydro-5H-imidazo[4,5-c]pyridine-5-carboxylate I-7c

Tert-butyl 2-((3-bromo-2-chlorophenyl)carbamoyl)-1-(methyl-*d*₃)-1,4,6,7-tetrahydro -5H-imidazo[4,5-c]pyridine-5-carboxylate I-2a (718.7 mg, 1.52 mmol, 1.0 eq) was dissolved in DCM (10 mL), added with TFA (10 mL), and stirred at ambient temperature for 30 min. The reaction solution was concentrated. The residue was dissolved in 1,4-dioxane (10 mL), and self-made tert-butyl 2-((2-chloro-3-(4,4,5,5-tetramethyl-1,3,2-dioxaborolan-2-yl)phenyl) carbamoyl)-1-(methyl-*d*₃)-1,4,6,7-tetrahydro-5H-imidazo[4,5-c]pyridine-5-carboxylate I-7b (766.54 mg, 1.52 mmol, 1.0 eq), PdCl₂(dcypf) (114.59 mg, 0.152 mmol, 0.1 eq), and anhydrous Na₂CO₃ (722.00 mg, 6.80 mmol , 4.5 eq) were added. The mixture was subjected to a procedure similar to that of preparing intermediate I-1c to obtain I-7c. (625.00 mg, yield 59.2%). LC-MS MS-ESI (m/z) 669.3 [M+H]⁺.

### Preparation of intermediate: tert-butyl 2-((2'-chloro-2-fluoro-3'-(5-(2-(4-(methoxycarbonyl)bicyclo[2.2.1]heptan-1-yl)ethyl)-1-(methyl-d₃)-4,5,6,7-tetrahydro-1H-imidazo [4,5-c]pyridine-2-carboxamido)-[1,1'-biphenyl]-3-yl)carbamoyl)-1-(methyl-d₃)-1,4,6,7-tetrahydr o-5H-imidazo[4,5-c]pyridine-5-carboxylate I-7h

I-7c (625.00 mg, 0.94 mmol, 1.0 eq) was dissolved in DCM (20 mL), and added with TEA (1 mL) and methyl 4-(2-oxoethyl)bicyclo[2.2.1]heptane-1-carboxylate I-1f (370.50 mg, 1.88 mmol, 2.0 eq). The resulting mixture was stirred at ambient temperature for 30 min, and added with NaBH(OAc)₃ (996.00 mg, 4.7 mmol, 5.0 eq). The mixture was subjected to a procedure similar to that of preparing intermediate I-1h to obtain a yellow solid I-7h. (600.00 mg, yield 75.1%). LC-MS MS-ESI (m/z) 849.4 [M+H]⁺.

### Preparation of intermediate: 4-(2-(2-((3'-(5-(tert-butoxycarbonyl)-1-(methyl-d₃) -4,5,6,7-tetrahydro-1H-imidazo[4,5-c]pyridine-2-carboxamido)-2-chloro-2'-fluoro-[1,1'-biphenyl ]-3-yl)carbamoyl)-1-(methyl-d₃)-1,4,6,7-tetrahydro-5H-imidazo[4,5-c]pyridin-5-yl)ethyl)bicyclo[ 2.2.1]heptane-1-carboxylic acid I-7i

The intermediate I-7h (600.00 mg, 0.71 mmol, 1.0 eq) and LiOH•H₂O (314.77 mg, 7.10 mmol) were subjected to a procedure similar to that of preparing intermediate I-2i to obtain a yellow intermediate I-7i. (450.00 mg, yield 75.9%). LC-MS MS-ESI (m/z) 835.4 [M+H]⁺.

### Preparation of compound: 4-(2-(2-((2-chloro-2'-fluoro-3'-(5-(2-(4-((methoxy-d₃) methyl)bicyclo[2.2.1]heptan-1-yl)ethyl)-1-(methyl-d₃)-4,5,6,7-tetrahydro-1H-imidazo[4,5-c]pyri dine-2-carboxamido)-[1,1'-biphenyl]-3-yl)carbamoyl)-1-(methyl-d₃)-1,4,6,7-tetrahydro-5H-imida zo[4,5-c]pyridin-5-yl)ethyl)bicyclo[2.2.1]heptane-1-carboxylic acid I-7

The intermediate I-7i (225 mg, 0.27mmol, 1.0 eq), TFA (5 mL), TEA (1 mL) and 2-(4-((methoxy-*d*₃)methyl)blcyclo[2.2.1]heptan-1-yl)acetaldehyde I-1d (147.15 mg, 0.54 mmol, 2.0 eq), and NaBH(OAc)₃ (290.88 mg, 1.35 mmol, 5.0 eq) were subjected to a procedure similar to that of preparing compound I-5 to obtain a white compound I-7. (120.00 mg, yield 49.1%). LC-MS MS-ESI (m/z) 904.5 [M+H]⁺. ¹H-NMR (400MHz, DMSO-*d*₆) *δ* ppm 9.94 (s, 1H), 9.79 (s, 1H), 8.34 (d, *J* = 8.0 Hz, 1H), 8.06 (t, *J* = 7.4 Hz, 1H), 7.49 (t, *J* = 7.9 Hz, 1H), 7.31 (t, *J* = 7.9 Hz, 1H), 7.21 (d, *J* = 7.5 Hz, 1H), 7.16 (t, *J* = 6.8 Hz, 1H), 3.45-3.36 (m, 4H), 3.32 (s, 2H), 2.77-2.71 (m, 4H), 2.69-2.59 (m, 4H), 2.57-2.52 (m, 4H), 1.91-1.80 (m, 2H), 1.76-1.64 (m, 4H), 1.58-1.19 (m, 16H), 1.12 (s, 2H).

### Example 8

### 4-(2-(2-((2-chloro-2'-fluoro-3'-(5-(2-(4-(methoxymethyl)bicyclo[2.2.1]heptane-1-yl)eth yl)-1-(methyl-d₃)-4,5,6,7-tetrahydro-1H-imidazo[4,5-c]pyridine-2-carboxamido)-[1,1'-biphenyl]-3-yl)carbamoyl)-1-(methyl-d₃)-1,4,6,7-tetrahydro-5H-imidazo[4,5-c]pyridin-5-yl)ethyl)bicyclo[2. 2.1]heptane-1-carboxylic acid I-8

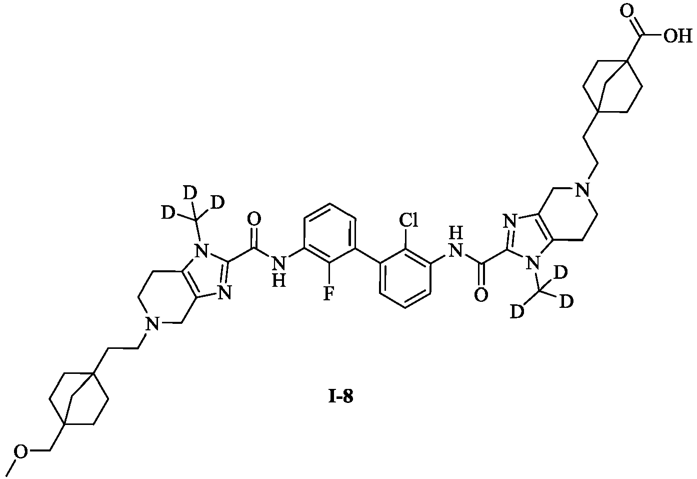

### Preparation of compound: 4-(2-(2-((2-chloro-2'-fluoro-3'-(5-(2-(4-(methoxymethyl) bicyclo[2.2.1]heptane-1-yl)ethyl)-1-(methyl-d₃)-4,5,6,7-tetrahydro-1H-imidazo[4,5-c]pyridine-2-carboxamido)-[1,1'-biphenyl]-3-yl)carbamoyl)-1-(methyl-d₃)-1,4,6,7-tetrahydro-5H-imidazo[4,5-c]pyridin-5-yl)ethyl)bicyclo[2.2.1]heptane-1-carboxylic acid I-8

The intermediate I-7i (225.00 mg, 0.27 mmol, 1.0 eq), TFA (5 mL), TEA (1 mL) and 2-(4-((methoxy)methyl)bicyclo[2.2.1]heptane-1-yl)acetaldehyde I-2d (143.05 mg, 0.54 mmol, 2.0 eq), and NaBH(OAc)₃ (290.88 mg, 1.35 mmol, 5.0 eq) were subjected to a procedure similar to that of preparing compound I-5 to obtain a white compound I-8. (120.00 mg, yield 50.1%). LC-MS MS-ESI (m/z) 901.5 [M+H]⁺. ¹H-NMR (400MHz, DMSO-*d*₆) *δ* ppm 9.93 (s, 1H), 9.79 (s, 1H), 8.34 (d, *J* = 8.2 Hz, 1H), 8.06 (t, *J* = 7.8 Hz, 1H), 7.49 (t, *J* = 8.0 Hz, 1H), 7.31 (t, *J* = 7.7 Hz, 1H), 7.21 (d, *J* = 7.3 Hz, 1H), 7.16 (t, *J* = 6.8 Hz, 1H), 3.44-3.38 (m, 4H),3.3 3 (s, 2H), 3.23 (s, 3H), 2.77-2.71 (m, 4H), 2.69-2.61 (m, 4H), 2.56-2.51 (m, 4H), 1.91-1.80 (m, 2H), 1.76-1.64 (m, 4H), 1.61-1.20 (m, 16H), 1.12 (s, 2H).

### Example 9

### 4-(2-(2-((2-chloro-3'-(5-(2-(4-((methoxy-d₃)methyl)bicyclo[2.2.1]heptane-1-yl)ethyl)-1 -(methyl-d₃)-4, 5,6,7-tetrahydro-1H-imidazo[4,5-c]pyridine-2-carboxamido)-2'-methyl-[1,1'-biph enyl]-3-yl)carbamoyl)-1-(methyl-d₃)-1,4,6,7-tetrahydro-5H-imidazo[4,5-c]pyridin-5-yl)ethyl)bic yclo[2.2.1]heptane-1-carboxylic acid I-9

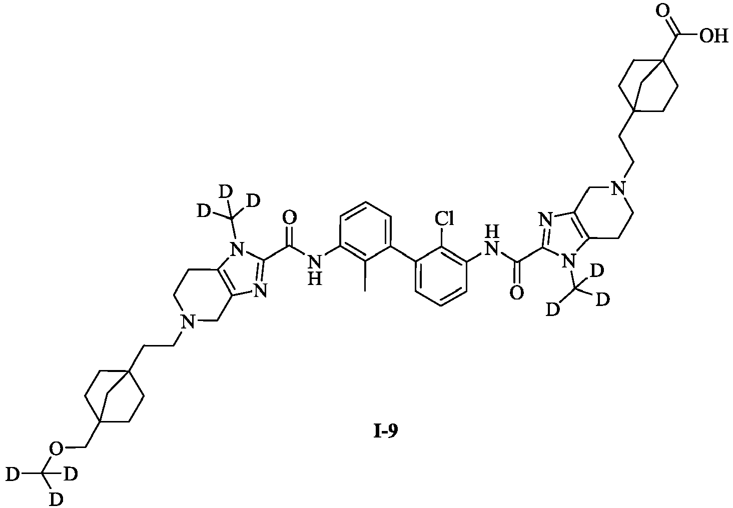

### Preparation of intermediate: tert-butyl 2-((2'-chloro-2-methyl-3'-(1-(methyl-d₃)-4,5,6,7-tetrahydro-1H-imidazo[4,5-c]pyridine-2-carboxamido)-[1,1'-biphenyl]-3-yl)carbamoyl)-1-(meth yl-d₃)-1,4,6,7-tetrahydro-5H-imidazo[4,5-c]pyridine-5-carboxylate I-9c

I-2a (1.34 g, 3.60 mmol, 1.0 eq), intermediate I-9b (1.80 g, 3.60 mmol, 1.0 eq), PdCl₂(dcypf) (271.40 mg, 0.36 mmol, 0.1 eq), and anhydrous Na₂CO₃ (1.72 g, 16.2 mmol, 4.5 eq) were subjected to a procedure similar to that of preparing the intermediate I-1c to obtain a yellow solid intermediate I-9c. (1.40 g, yield 58.5%). LC-MS MS-ESI (m/z) 665.3 [M+H]⁺.

### Preparation of intermediate: tert-butyl 2-((2'-chloro-3'-(5-(2-(4-(methoxycarbonyl) bicyclo[2.2.1]heptane-1-yl)ethyl)-1-(methyl-d₃)-4,5,6,7-tetrahydro-1H-imidazo[4,5-c]pyridine-2-carboxamido)-2-methyl-[1,1'-biphenyl]-3-yl)carbamoyl)-1-(methyl-d₃)-1,4,6,7-tetrahydro-5H-im idazo[4,5-c]pyridine-5-carboxylate I-9h

The intermediate I-9c (700.00 mg, 1.05 mmol, 1.0 eq), methyl 4-(2-oxoethyl)bicyclo[2.2.1]heptane-1-carboxylate I-1f (414.70 mg, 2.10 mmol, 2.0 eq), TEA (1 mL), and NaBH(OAc)₃ (1.12 g, 5.3 mmol, 5.0 eq) were subjected to a procedure similar to that of preparing intermediate I-1h to obtain a yellow intermediate I-9h. (650.00 mg, yield 73.2%). LC-MS MS-ESI (m/z) 845.4 [M+H]⁺.

### Preparation of intermediate: 4-(2-(2-((3'-(5-(tert-butoxycarbonyl)-1-(methyl-d₃)-4,5,6,7-tetrahydro-1H-imidazo[4,5-c]pyridine-2-carboxamido)-2-chloro-2'-methyl-[1,1'-biphenyl ]-3-yl)carbamoyl)-1-(methyl-d₃)-1,4,6,7-tetrahydro-5H-imidazo[4,5-c]pyridin-5-yl)ethyl)bicyclo[ 2.2.1]heptane-1-carboxylic acid I-9i

The intermediate I-9h (650.00 mg, 0.77 mmol, 1.0 eq) and LiOH•H₂O (340.86 mg, 7.70 mmol) were subjected to a procedure similar to that of preparing intermediate I-2i to obtain a yellow intermediate I-9i. (460.00 mg, yield 71.9%). LC-MS MS-ESI (m/z) 831.4 [M+H]⁺.

### Preparation of compound: 4-(2-(2-((2-chloro-3'-(5-(2-(4-((methoxy-d₃)methyl) bicyclo[2.2.1]heptane-1-yl)ethyl)-1-(methyl-d₃)-4,5,6,7-tetrahydro-1H-imidazo[4,5-c]pyridine-2-carboxamido)-2'-methyl-[1,1'-biphenyl]-3-yl)carbamoyl)-1-(methyl-d₃)-1,4,6,7-tetrahydro-5H-i midazo[4,5-c]pyridin-5-yl)ethyl)bicyclo[2.2.1]heptane-1-carboxylic acid I-9

The intermediate I-9i (230.00 mg, 0.28 mmol, 1.0 eq), TFA (5 mL), TEA (1 mL), 2-(4-((methoxy-*d*₃)methyl)blcyclo[2.2.1]heptan-1-yl)acetaldehyde I-1d (153.35 mg, 0.56 mmol, 2.0 eq), and NaBH(OAc)₃ (310.28 mg, 1.40 mmol, 5.0 eq) were subjected to a procedure similar to that of preparing compound I-5 to obtain a white compound I-9. (125.00 mg, yield 49.6%). LC-MS MS-ESI (m/z) 900.5 [M+H]⁺. ¹H-NMR (400MHz, DMSO-*d*₆) *δ* ppm 9.90 (s, 1H), 9.72 (s, 1H), 8.34 (dd, *J* = 8.2, 1.3 Hz, 1H), 7.73 (d, *J* = 7.9 Hz, 1H), 7.45 (t, *J* = 7.9 Hz, 1H), 7.29 (t, *J* = 7.8 Hz, 1H), 7.06 (dd, *J* = 7.6, 1.4 Hz, 1H), 7.00 (d, *J* = 7.4 Hz, 1H), 3.42-3.38 (m, 4H), 3.32 (s, 2H), 2.77-2.70 (m, 4H), 2.68-2.60 (m, 4H), 2.55-2.50 (m, 4H), 1.98 (s, 3H), 1.91-1.80 (m, 2H), 1.76-1.65 (m, 4H), 1.57-1.21 (m, 16H), 1.12 (s, 2H).

### Example 10

### 4-(2-(2-((2-chloro-3'-(5-(2-(4-(methoxymethyl)bicyclo[2.2.1]heptane-1-yl)ethyl)-1-(me thyl-d₃)-4,5,6,7-tetrahydro-1H-imidazo[4,5-c]pyridine-2-carboxamido)-2'-methyl-[1,1'-biphenyl] -3-yl)carbamoyl)-1-(methyl-d₃)-1,4,6,7-tetrahydro-5H-imidazo[4,5-c]pyridin-5-yl)ethyl)bicyclo[ 2.2.1]heptane-1-carboxylic acid 1-10

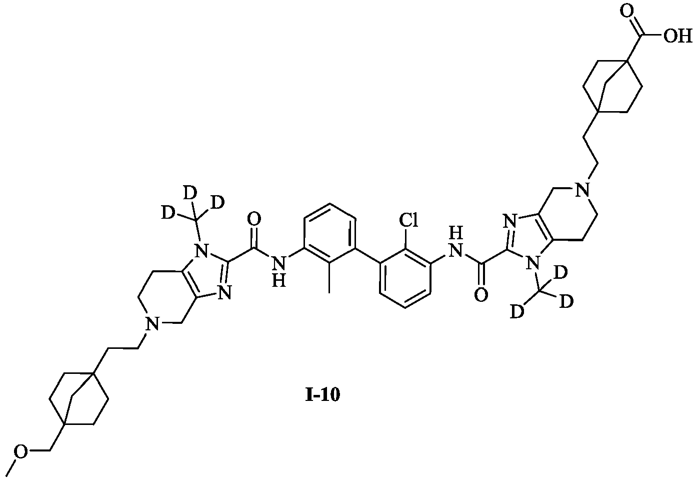

Intermediate I-9i (230.00 mg, 0.28 mmol, 1.0 eq), TFA (5 mL), TEA (1 mL) and 2-(4-((methoxy)methyl)bicyclo[2.2.1]heptane-1-yl)acetaldehyde I-2d (151.12 mg, 0.56 mmol, 2.0 eq), and NaBH(OAc)₃ (310.28 mg, 1.40 mmol, 5.0 eq) were subjected to a procedure similar to that of preparing compound I-5 to obtain a white compound I-10. (124.00 mg, yield 49.3%). LC-MS MS-ESI (m/z) 897.5 [M+H]⁺. ¹H-NMR (400MHz, DMSO-*d*₆) *δ* ppm 9.90 (s, 1H), 9.73 (s, 1H), 8.34 (d, *J* = 6.9 Hz, 1H), 7.72 (d, *J* = 8.2 Hz, 1H), 7.45 (t, *J* = 7.9 Hz, 1H), 7.30 (t, *J* = 8.0 Hz, 1H), 7.06 (dd, *J* = 7.6, 1.4 Hz, 1H), 7.00 (d, *J* = 7.7 Hz, 1H), 3.44-3.37 (m, 4H), 3.33 (s, 2H), 3.24 (s, 3H), 2.79-2.70 (m, 4H), 2.69-2.60 (m, 4H), 2.56-2.51 (m, 3H), 1.98 (s, 3H), 1.91-1.80 (m, 2H), 1.74-1.65 (m, 4H), 1.57-1.21 (m, 16H), 1.12 (s, 2H).

### Example 11

### 4-(2-(2-((2'-chloro-3'-(5-(2-(4-((methoxy-d₃)methyl)blcyclo[2.2.1]heptane-1-yl)ethyl)-1-(methyl-d₃)-4,5,6,7-tetrahydro-1H-imidazo[4,5-c]pyridine-2-carboxamido)-2-methyl-[1,1'-biph enyl]-3-yl)carbamoyl)-1-(methyl-d₃)-1,4,6,7-tetrahydro-5H-imidazo[4,5-c]pyridin-5-yl)ethyl)bic yclo[2.2.1]heptane-1-carboxylic acid I-11

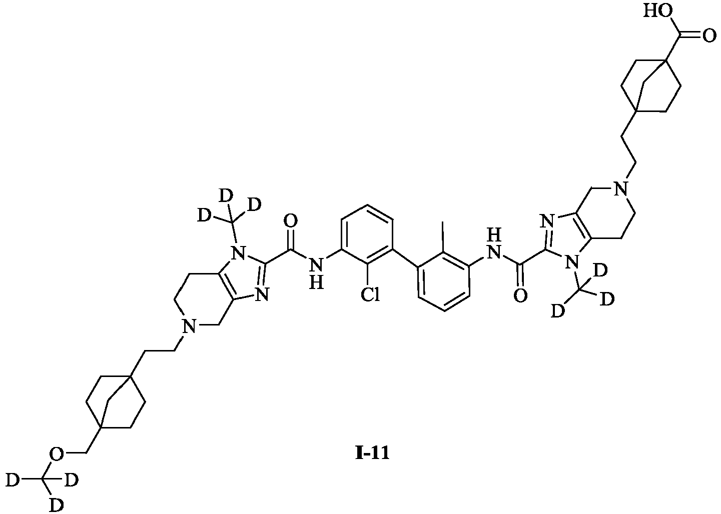

### Preparation of intermediate: tert-butyl 2-((2'-chloro-3'-(5-(2-(4-((methoxy-d₃)methyl) bicyclo[2.2.1]heptan-1-yl)ethyl)-1-(methyl-d₃)-4,5,6,7-tetrahydro-1H-imidazo[4,5-c]pyridine-2-c arboxamido)-2-methyl-[1,1'-biphenyl]-3-yl)carbamoyl)-1-(methyl-d₃)-1,4,6,7-tetrahydro-5H-imi dazo[4,5-c]pyridine-5-carboxylate 1-11e

Intermediate I-9c (350.00 mg, 0.53 mmol, 1.0 eq) and 2-(4-((methoxy-*d*₃)methyl)blcyclo[2.2.1]heptan-1-yl)acetaldehyde I- 1d (194.60 mg, 1.05 mmol, 2.0 eq) were dissolved in DCM (20 mL), added with TEA(1 mL), stirred at room temperature for 30 min, and added with NaBH(OAc)₃ (560.00 mg, 2.7 mmol, 5.0 eq) for reaction at room temperature overnight. The reaction was quenched by water (20 mL). The organic phase was separated, dried and spin-dried. The crude product was separated by silica gel column chromatography (DCM/MeOH (v/v) = 20/1) to obtain intermediate I-9e. (325.00 mg, yield 74.2%). LC-MS MS-ESI (m/z) 834.5 [M+H]⁺.

### Preparation of intermediate: methyl 4-(2-(2-((2'-chloro-3'-(5-(2-(4-((methoxy-d₃) methyl)bicyclo[2.2.1]heptane-1-yl)ethyl)-1-(methyl-d₃)-4,5,6,7-tetrahydro-1H-imidazo[4,5-c]pyri dine-2-carboxamido)-2-methyl-[1,1'-biphenyl]-3-yl)carbamoyl)-1-(methyl-d₃)-1,4,6,7-tetrahydro -5H-imidazo[4,5-c]pyridin-5-yl)ethyl)bicyclo[2.2.1]heptane-1-carboxylate I'-11

Intermediate I-9e (325.00 mg, 0.39 mmol, 1.0 eq) was dissolved in DCM (5 mL), added with TFA (5 mL) in one go, and stirred at room temperature for 30 min. The reaction solution was spin-dried to obtain a light yellow transparent oil. The light yellow transparent oil was dissolved in DCM, added with TEA (1 mL) and methyl 4-(2-oxoethyl)bicyclo[2.2.1]heptane-1-carboxylate I-1f (212.55 mg, 0.77 mmol, 2.0 eq), stirred at room temperature for 30 min, and added with NaBH(OAc)₃ (420.14 mg, 1.95 mmol, 5.0 eq) for reaction at room temperature overnight. The reaction was quenched by water (20 mL). The organic phase was separated, and spin-dried. The crude product was separated by preparative TLC (DCM/MeOH (v/v) = 10/1) to obtain intermediate I'-11. (210.00 mg, yield 58.9%). LC-MS MS-ESI (m/z) 914.5 [M+H]⁺.

### Preparation of compound: 4-(2-(2-((2'-chloro-3'-(5-(2-(4-((methoxy-d₃)methyl) bicyclo[2.2.1]heptane-1-yl)ethyl)-1-(methyl-d₃)-4,5,6,7-tetrahydro-1H-imidazo[4,5-c]pyridine-2-carboxamido)-2-methyl-[1,1'-biphenyl]-3-yl)carbamoyl)-1-(methyl-d₃)-1,4,6,7-tetrahydro-5H-im idazo[4,5-c]pyridin-5-yl)ethyl)bicyclo[2.2.1]heptane-1-carboxylic acid I-11

LiOH·H₂O (95.82 mg, 2.3 mmol) was weighed, and dissolve in water (10 mL). Intermediate I'-11 (210.00 mg, 0.23 mmol, 1.0 eq) was dissolved in THF (10 mL), and then the mixture was added to the above LiOH·H₂O solution, and stirred overnight at room temperature. The reaction solution was spin-dried to remove THF. The aqueous phase was adjusted to a pH of weak acidity, and extracted twice with EtOAc (30 mL). The organic phases were combined, dried over anhydrous Na₂SO₄, and concentrated. The crude product was separated by preparative TLC (DCM/MeOH (v/v) = 10/1) to obtain compound I-11. (122.00 mg, yield 58.9%). LC-MS MS-ESI (m/z) 900.5 [M+H]⁺. ¹H-NMR (400MHz, DMSO-*d*₆) *δ* ppm 9.90 (s, 1H), 9.73 (s, 1H), 8.34 (d, *J* = 8.3 Hz, 1H), 7.73 (d, *J* = 8.1 Hz, 1H), 7.45 (t, *J* = 8.0 Hz, 1H), 7.30 (t, *J* = 8.0 Hz, 1H), 7.06 (d, *J* = 6.5 Hz, 1H), 7.00 (d, *J* = 7.4 Hz, 1H), 3.43-3.37 (m, 4H), 3.32 (s, 2H), 2.79-2.70 (m, 4H), 2.70-2.61 (m, 4H), 2.56-2.51 (m, 4H), 1.98 (s, 3H), 1.92-1.79 (m, 2H), 1.77-1.65 (m, 4H), 1.59-1.20 (m, 16H), 1.12 (s, 2H).

### Example 12

### 4-(2-(2-((2'-chloro-3'-(5-(2-(4-(methoxymethyl)bicyclo[2.2.1]heptane-1-yl)ethyl)-1-(m ethyl-d₃)-4,5,6,7-tetrahydro-1H-imidazo[4,5-c]pyridine-2-carboxamido)-2-methyl-[1,1'-biphenyl ]-3-yl)carbamoyl)-1-(methyl-d₃)-1,4,6,7-tetrahydro-5H-imidazo[4,5-c]pyridin-5-yl)ethyl)bicyclo [2.2.1]heptane-1-carboxylic acid 1-12

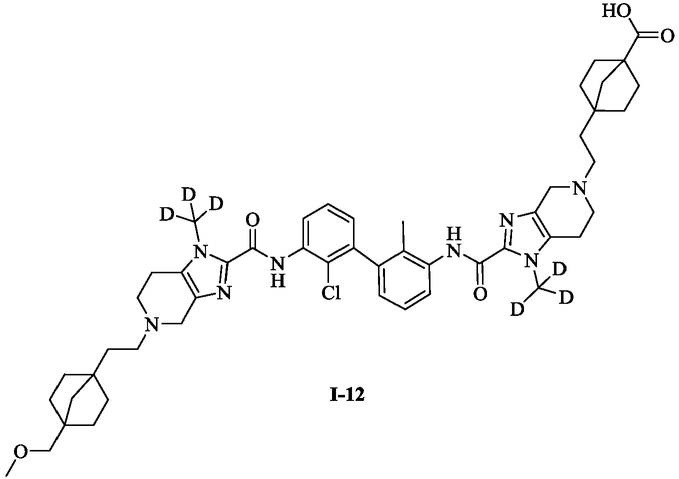

### Preparation of intermediate: tert-butyl 2-((2'-chloro-3'-(5-(2-(4-(methoxymethyl) bicyclo[22.1]heptane-1-yl)ethyl)-1-(methyl-d₃)-4,5,6,7-tetrahydro-1H-imidazo[4,5-c]pyridine-2-carboxamido)-2-methyl-[1,1'-biphenyl]-3-yl)carbamoyl)-1-(methyl-d₃)-1,4,6,7-tetrahydro-5H-im idazo [4,5 -c]pyridine-5 -carboxylate I-12e

Intermediate I-9c (350.00 mg, 0.53 mmol, 1.0 eq) and 2-(4-(methoxymethyl)bicyclo[2.2.1]heptan-1-yl)acetaldehyde I-2d (191.12 mg, 1.05 mmol, 2.0 eq) were dissolved in DCM (20 mL), added with TEA (1 mL), stirred at room temperature for 30 min, and added with NaBH(OAc)₃ (560.00 mg, 2.7 mmol, 5.0 eq) for reaction overnight at room temperature. The reaction was quenched with water (20 mL). The organic phase was separated, and spin-dried. The crude product was separated by silica gel column chromatography (DCM/MeOH (v/v) = 20/1) to obtain intermediate I-12e. (324.3 mg, yield 72.6%). LC-MS MS-ESI (m/z) 831.5 [M+H]⁺.

### Preparation of intermediate: methyl 4-(2-(2-((2'-chloro-3'-(5-(2-(4-(methoxymethyl) bicyclo[2.2.1]heptane-1-yl)ethyl)-1-(methyl-d₃)-4,5,6,7-tetrahydro-1H-imidazo[4,5-c]pyridine-2-carboxamido)-2-methyl-[1,1'-biphenyl]-3-yl)carbamoyl)-1-(methyl-d₃)-1,4,6,7-tetrahydro-5H-im idazo[4,5-c]pyridin-5-yl)ethyl)bicyclo[2.2.1]heptane-1-carboxylate I'-12

Intermediate I-9e (324.03 mg, 0.39 mmol, 1.0 eq) was dissolved in DCM (5 mL), added with TFA (5 mL) in one go, and stirred at room temperature for 30 min. The reaction solution was spin-dried to obtain a light yellow transparent oil. The light yellow transparent oil was dissolved in DCM, added with TEA (1 mL) and methyl 4-(2-oxoethyl)bicyclo[2.2.1]heptane-1-carboxylate I-1f (212.55 mg, 0.77 mmol , 2.0 eq), stirred at room temperature for 30 min, and added with NaBH(OAc)₃ (420.14 mg, 1.95 mmol, 5.0 eq) for reaction at room temperature overnight. The reaction was quenched with water (20 mL). The organic phase was separated, and spin-dried. The crude product was separated by preparative TLC (DCM/MeOH (v/v) = 10/1) to obtain intermediate I'-12. (208.00 mg, yield 58.5%). LC-MS MS-ESI (m/z) 911.5 [M+H]⁺.

### Preparation of compound: 4-(2-(2-((2'-chloro-3'-(5-(2-(4-(methoxymethyl)bicycle [2.2.1]heptane-1-yl)ethyl))-1-(methyl-d₃)-4,5,6,7-tetrahydro-1H-imidazo[4,5-c]pyridine-2-carbox amido)-2-methyl-[1,1'-biphenyl]-3-yl)carbamoyl)-1-(methyl-d₃)-1,4,6,7-tetrahydro-5H-imidazo[ 4,5-c]pyridin-5-yl)ethyl)bicyclo[2.2.1]heptane-1-carboxylic acid I-12

LiOH·H₂O (100.85 mg, 2.43 mmol) was weighed and dissolve in water (10 mL). Intermediate I'-12 (215.00 mg, 0.24 mmol, 1.0 eq) was dissolved in THF (10 mL), then added with the above LiOH·H₂O solution, and stirred overnight at room temperature. The mixture was spin-dried to remove THF. The aqueous phase was adjusted to weak acidity, and extracted twice with EtOAc (30 mL). The organic phases were combined, dried over anhydrous Na₂SO₄, and concentrated. The crude product was separated by preparative TLC (DCM/MeOH (v/v) = 10/1) to obtain compound I-12. (10.00 mg, yield 4.64%). LC-MS MS-ESI (m/z) 897.5 [M+H]⁺. ¹H-NMR (400MHz, DMSO-*d*₆) *δ* ppm 9.98 (s, 1H), 9.72 (s, 1H), 8.33 (d, *J* = 8.2 Hz, 1H), 7.72 (d, *J* = 7.5 Hz, 1H), 7.45 (t, *J* = 7.9 Hz, 1H), 7.30 (t, *J* = 7.8 Hz, 1H), 7.06 (d, *J* = 7.6 Hz, 1H), 7.00 (d, *J =* 7.4 Hz, 1H), 3.44-3.38 (m, 4H), 3.32 (s, 2H), 3.24 (s, 3H), 2.78-2.70 (m, 4H), 2.68-2.62 (m, 4H), 2.55-2.51 (m, 4H), 1.98 (s, 3H), 1.91-1.79 (m, 2H), 1.75-1.64 (m, 4H), 1.56-1.19 (m, 16H), 1.11 (s, 2H).

### Example 13

### 4-(2-(2-((2,2'-dichloro-3'-(5-(2-(4-((methoxy-d₃)methyl)bicyclo[2.2.1]heptane-1-yl)eth yl)-1-(methyl-d₃)-4,5,6,7-tetrahydro-1H-imidazo[4,5-c]pyridine-2-carboxamido)-[1,1'-biphenyl]-3-yl-4,4',5,5',6,6'-d₆)carbamoyl)-1-(methyl-d₃)-1,4,6,7-tetrahydro-5H-imidazo[4,5-c]pyridin-5-yl )ethyl)bicyclo[2.2.1]heptane-1-carboxylic acid 1-13

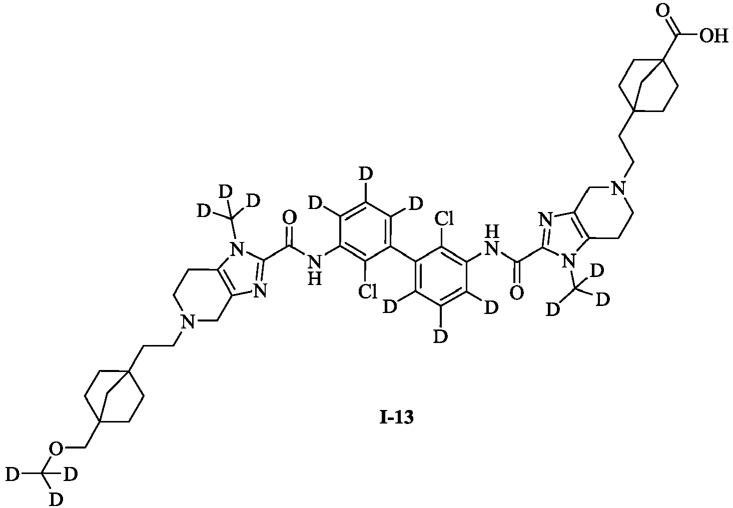

### Preparation of intermediate: tert-butyl 2-((2,2'-dichloro-3'-(1-(methyl-d₃)-4,5,6,7-tetrahydro-1H-imidazo[4,5-c]pyridine-2-carboxamido)-[1,1'-biphenyl]-3-yl-4,4',5,5',6,6'-d₆)carb amoyl)-1-(methyl-d₃)-1,4,6,7-tetrahydro-5H-imidazo[4,5-c]pyridine-5-carboxylate I-13c

Tert-butyl 2-((3-bromo-2-chlorophenyl-4,5,6-*d*₃)carbamoyl)-1-(methyl-d3)-1,4,6,7-tetrahydro-5H-imidazo[4,5-c]pyridine-5-carboxylate I-3a (950.00 mg, 1.99 mmol, 1.0 eq) was dissolved in DCM (10 mL), added with TFA (10 mL), and stirred at ambient temperature for 30 min. The reaction solution was concentrated. The residue was dissolved in 1,4-dioxane (10 mL), and tert-butyl 2-((2-chloro-3-(4,4,5,5-tetramethyl-1,3,2-dioxaborolan-2-yl)phenyl-4,5,6-*d*₃) carbamoyl)-1-(methyl-*d*₃)-1,4,6,7-tetrahydro-5H-imidazo[4,5-c]pyridine-5-carboxylate I-3b (660.00 mg, 1.26 mmol, 1.0 eq), PdCl₂(dcypf) (95.20 mg, 0.13 mmol, 0.1 eq), anhydrous Na₂CO₃ (400.60 mg, 3.78 mmol, 3.0 eq) and water (5 mL) were added. The resulting mixture was heated to 110°C by microwave for 3 h of reaction, and then cooled to ambient temperature. The reaction solution was concentrated, and the crude product was separated by silica gel column chromatography (DCM/MeOH (v/v) = 10/1) to obtain a white solid I-13c. (420.00 mg, yield 48.2%). LC-MS MS-ESI (m/z) 691.4 [M+H]⁺.

### Preparation of intermediate: tert-butyl 2-((2, 2'-dichloro-3'-(5-(2-(4-(methoxycarbonyl) bicyclo[2.2.1]heptan-1-yl)ethyl)-1-(methyl-d₃)-4,5,6,7-tetrahydro-1H-imidazo[4,5-c]pyridine-2-c arboxamido)-[1,1'-biphenyl]-3-yl-4,4',5,5',6,6'-d₆)carbamoyl)-1-(methyl-d₃)-1,4,6,7-tetrahydro-5H-imidazo[4,5-c]pyridine-5-carboxylate I-13h

I-13c (420.00 mg, 0.60 mmol, 1.0 eq) was dissolved in DCM (20 mL), and added with TEA (1 mL) and methyl 4-(2-oxoethyl)bicyclo[2.2.1]heptane-1-carboxylate I-1f (130.10 mg, 0.66 mmol, 1.1 eq). The resulting mixture was stirred at ambient temperature for 30 min, and added with NaBH(OAc)₃ (636.00 mg, 3.0 mmol, 5.0 eq) for reaction at room temperature overnight. The reaction was quenched by water (20 mL). The organic phase was separated, and spin-dried. The crude product was separated by silica gel column chromatography (DCM/MeOH (v/v) = 20/1) to obtain a yellow solid I-13h. (390.00 mg, yield 73.7%). LC-MS MS-ESI (m/z) 871.5 [M+H]⁺.

### Preparation of intermediate: methyl 4-(2-(2-((2,2'-dichloro-3'-(5-(2-(4-((methoxy-d₃) methyl)bicyclo[2.2.1]heptan-1-yl)ethyl)-1-(methyl-d₃)-4,5,6,7-tetrahydro-1H-imidazo[4,5-c]pyri dine-2-carboxamido)-[1,1'-biphenyl]-3-yl-4,4',5,5',6,6'-d₆)carbamoyl)-1-(methyl-d₃)-1,4,6,7-tetr ahydro-5H-imidazo[4,5-c]pyridin-5-yl)ethyl)bicyclo[2.2.1]heptane-1-carboxylate I'-13

Intermediate I-13h (390.00 mg, 0.44 mmol, 1.0 eq) was dissolved in DCM (5 mL), added with TFA (5 mL) in one go, and stirred at room temperature for 30 min. The reaction solution was spin-dried to obtain a light yellow transparent oil. The light yellow transparent oil was dissolved in DCM/MeOH (v/v, 10/1), added with TEA (1 mL) and methyl 4-(2-oxoethyl)bicyclo[2.2.1]heptane-1-carboxylate I-1f (99.45 mg, 0.53 mmol, 1.2 eq), stirred at room temperature for 30 min, and added with NaBH(OAc)₃ (474.13 mg, 2.23 mmol, 5.0 eq) for reaction overnight at room temperature. The reaction was quenched with water (20 mL). The organic phase was separated, and spin-dried. The crude product was separated by preparative TLC (DCM/MeOH (v/v) = 20/1) to obtain intermediate I'-13. (140.00 mg, yield 33.2%). LC-MS MS-ESI (m/z) 940.6 [M+H]⁺.

### Preparation of compound: 4-(2-(2-((2, 2'-dichloro-3'-(5-(2-(4-((methoxy-d₃)methyl) bicyclo[2.2.1]heptane-1-yl)ethyl)-1-(methyl-d₃)-4,5,6,7-tetrahydro-1H-imidazo[4,5-c]pyridine-2-carboxamido)-[1,1'-biphenyl]-3-yl-4,4',5,5',6,6'-d₆)carbamoyl)-1-(methyl-d₃)-1,4,6,7-tetrahydro-5H-imidazo[4,5-c]pyridin-5-yl)ethyl)bicyclo[2.2.1]heptane-1-carboxylic acid I-13

LiOH•H₂O (210.00 mg, 5.00 mmol) was weighed and dissolved in water (50 mL) to prepare a solution. Intermediate I'-13 (140.00 mg, 0.14 mmol, 1.0 eq) was dissolved in THF (20 mL), added with the above LiOH•H₂O solution (20 mL), and stirred overnight at room temperature. THF was concentrated. The pH of the aqueous phase was adjusted to weak acidity, and a white solid was precipitated. The mixture was filtered with suction, and dried to obtain 1-13. (84.00 mg, yield 60.9%). LC-MS MS-ESI (m/z) 926.6 [M+H]⁺. ¹H-NMR (400MHz, DMSO-*d*₆) *δ* ppm 9.89 (s, 2H), 3.42-3.30 (m, 6H), 2.79-2.70 (m, 4H), 2.69-2.62 (m, 4H), 2.55-2.51 (m, 4H), 1.90-1.79 (m, 2H), 1.75-1.66 (m,4H), 1.56-1.43 (m, 8H), 1.40 (s, 2H), 1.36-1.22 (m, 6H), 1.11 (s, 2H).

### Example 14

### 4-(2-(2-((2-chloro-2'-fluoro-3'-(5-(2-(4-((methoxy-d₃)methyl)bicyclo[2.2.1]heptane-1-y 1)ethyl)-1-methyl-4,5,6,7-tetrahydro-1H-imidazo[4,5-c]pyridine-2-carboxamido)-[1,1'-biphenyl]-3-yl-4,4',5,5',6,6'-d₆)carbamoyl)-1-methyl-1,4,6,7-tetrahydro-5H-imidazo[4,5-c]pyridin-5-yl)eth yl)bicyclo[2.2.1]heptane-1-carboxylic acid I-14

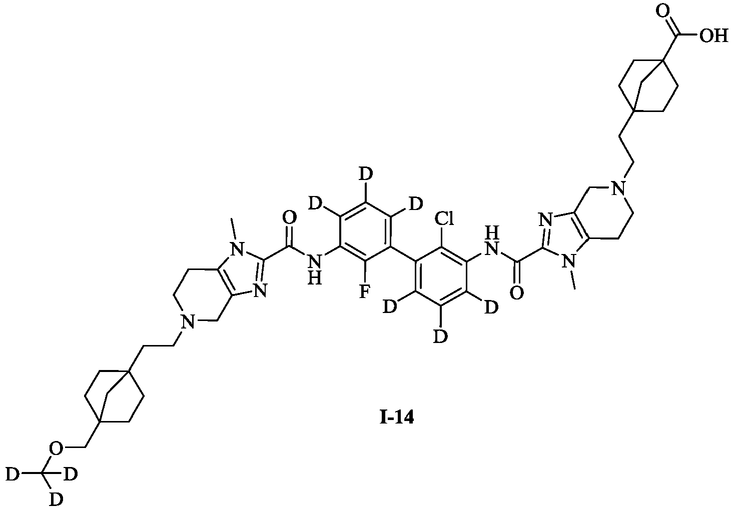

### Preparation of intermediate: tert-butyl 2-((2'-chloro-2-fluoro-3'-(1-methyl-4,5,6,7-tetrahydro-1H-imidazo[4,5-c]pyridine-2-carboxamido)-[1,1'-biphenyl]-3-yl-4,4',5,5',6,6'-d₆)carb amoyl)-1 -methyl-1,4,6,7-tetrahydro-5H-imidazo [4,5 -c]pyridine-5 -carboxylate I-14c

Intermediate I-14a (780.0 mg, 1.65 mmol, 1.0 eq), intermediate I-14b (766.78 mg, 1.52 mmol, 1.0 eq), PdCl₂(dcypf) (114.62 mg, 0.15 mmol , 0.1 eq), and anhydrous Na₂CO₃ (724.00 mg, 6.8 mmol, 4.5 eq) were subjected to a procedure similar to that of preparing intermediate I-1c to obtain a yellow intermediate I-14c. (625.65 mg, yield 59.2%). LC-MS MS-ESI (m/z) 669.3 [M+H]⁺.

### Intermediate: tert-butyl 2-((2'-chloro-2-fluoro-3'-(5-(2-(4-(methoxycarbonyl) bicyclo[2.2.1]heptan-1-yl)ethyl)-1-methyl-4,5,6,7-tetrahydro-1H-imidazo[4,5-c]pyridine-2-carbo xamido)-[1,1'-biphenyl]-3-yl-4,4',5,5',6,6'-d₆)carbamoyl)-1-methyl-1,4,6,7-tetrahydro-5H-imida zo[4,5-c]pyridine-5-carboxylate I-14h

Intermediate I-14c (625.65 mg, 0.94 mmol, 1.0 eq), commercially available methyl 4-(2-oxoethyl)bicyclo[2.2.1]heptane-1-carboxylate I-1f (370.80 mg, 1.88 mmol, 2.0 eq), TEA (1 mL), and NaBH(OAc)₃ (996.7 mg, 4.7 mmol, 5.0 eq) were subjected to a procedure similar to that of preparing intermediate I-1h to obtain a yellow intermediate I-14h. (601.08 mg, yield 75.1%). LC-MS MS-ESI (m/z) 849.4 [M+H]⁺.

### Intermediate: 4-(2-(2-((3'-(5-(tert-butoxycarbonyl)-1-methyl-4,5,6,7-tetrahydro-1H-imidazo[4,5-c]pyridine-2-carboxamido)-2-chloro-2'-ffuoro-[1,1'-biphenyl]-3-yl-4,4',5,5',6,6'-d₆) carbamoyl)-1-methyl-1,4,6,7-tetrahydro-5H-imidazo[4,5-c]pyridin-5-yl)ethyl)bicyclo[2.2.1]hepta ne-1-carboxylic acid 1-14i

Intermediate I-9h (601.08 mg, 0.71 mmol, 1.0 eq) and LiOH•H₂O (315.86 mg, 7.10 mmol) were subjected to a procedure similar to that of preparing intermediate I-2i to obtain a yellow intermediate 1-14i. (451.32 mg, yield 75.9%). LC-MS MS-ESI (m/z) 835.4 [M+H]⁺.

### Preparation of compound: 4-(2-(2-((2-chloro-2'-fluoro-3'-(5-(2-(4-((methoxy-d₃)methyl) bicyclo[2.2.1]heptane-1-yl)ethyl)-1-methyl-4,5,6,7-tetrahydro-1H-imidazo[4,5-c]pyridine-2-carb oxamido)-[1,1'-biphenyl]-3-yl-4,4',5,5',6,6'-d₆)carbamoyl)-1-methyl-1,4,6,7-tetrahydro-5H-imid azo[4,5-c]pyridin-5-yl)ethyl)bicyclo[2.2.1]heptane-1-carboxylic acid I-14

Intermediate I-14i (225.00 mg, 0.27 mmol, 1.0 eq), TFA (5 mL), TEA (1 mL) and 2-(4-((methoxy-*d*₃)methyl)blcyclo[2.2.1]heptan-1-yl)acetaldehyde I-1d (147.15 mg, 0.54 mmol, 2.0 eq), and NaBH(OAc)₃ (290.88 mg, 1.35 mmol, 5.0 eq) were subjected to a procedure similar to that of preparing compound I-5 to obtain a white compound I-14. (60.00 mg, yield 24.6%). LC-MS MS-ESI (m/z) 904.5 [M+H]⁺. ¹H-NMR (400MHz, DMSO-*d*₆) *δ* ppm 9.93 (s, 1H), 9.78 (s, 1H), 3.89 (s, 3H), 3.87 (s, 3H), 3.42-3.38 (m, 4H), 3.32 (s, 2H), 2.78-2.70 (m, 4H), 2.69-2.61(m, 4H), 2.57-2.51 (m, 4H), 1.91-1.80 (m, 2H), 1.76-1.65 (m, 4H), 1.56-1.21 (m, 16H), 1.11 (s, 2H).

### Example 15

### 4-(2-(2-((2-chloro-2'-fluoro-3'-(5-(2-(4-(methoxymethyl)bicyclo[2.2.1]heptane-1-yl)eth yl)-1-methyl-4,5,6,7-tetrahydro-1H-imidazo[4,5-c]pyridine-2-carboxamido)-[1,1'-biphenyl]-3-yl-4,4',5,5',6,6'-d₆)carbamoyl)-1-methyl-1,4,6,7-tetrahydro-5H-imidazo[4,5-c]pyridin-5-yl)ethyl)bi cyclo[2.2.1]heptane-1-carboxylic acid I-15

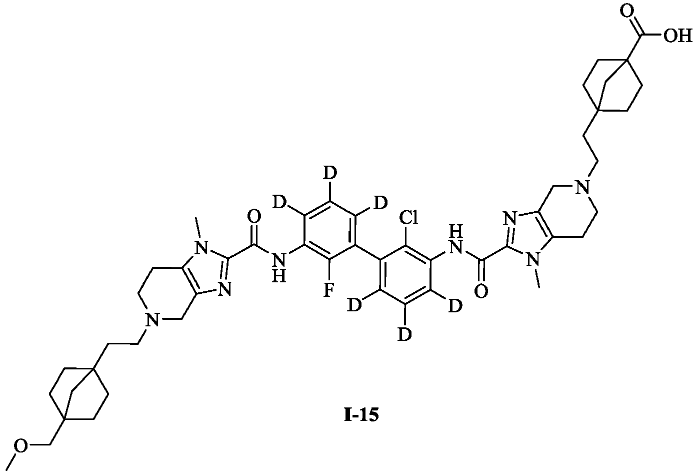

### Preparation of compound: 4-(2-(2-((2-chloro-2'-fluoro-3'-(5-(2-(4-(methoxymethyl) bicyclo[2.2.1]heptane-1-yl)ethyl)-1-methyl-4,5,6,7-tetrahydro-1H-imidazo[4,5-c]pyridine-2-carb oxamido)-[1,1'-biphenyl]-3-yl-4,4',5, 5',6,6'-d₆)carbamoyl)-1-methyl-1,4,6,7-tetrahydro-5H-imid azo[4,5-c]pyridin-5-yl)ethyl)bicyclo[2.2.1]heptane-1-carboxylic acid I-15

Intermediate I-14i (225.00 mg, 0.27 mmol, 1.0 eq), TFA (5 mL), TEA (1 mL), 2-(4-(methoxymethyl)bicyclo[2.2.1]heptane-1-yl)acetaldehyde I-2d (143.05 mg, 0.54 mmol, 2eq), and NaBH(OAc)₃ (290.88 mg, 1.35 mmol, 5.0 eq) were subjected to a procedure similar to that of preparing compound I-5 to obtain a white compound I-15. (60.00 mg, yield 25.0%). LC-MS MS-ESI (m/z) 901.5 [M+H]⁺. ¹H-NMR (400MHz, DMSO-*d*₆) *δ* ppm 9.94 (s, 1H), 9.79 (s, 1H), 3.90 (s, 3H), 3.87 (s, 3H), 3.43-3.38 (m, 4H), 3.33 (s, 2H), 3.24 (s, 3H), 2.78-2.70 (m, 4H), 2.69-2.60 (m, 4H), 2.57-2.51 (m, 4H), 1.91-1.81 (m, 2H), 1.77-1.65 (m, 4H), 1.58-1.22 (m, 16H), 1.12 (s, 2H).

### In vitro biological evaluation

This measurement method is used for evaluating the in vitro biological activity of the compound of the present invention, including an evaluation method of in vitro protein-level binding inhibitory activity and an evaluation method of cell-level biological function activity.

The purpose of this measurement is to comprehensively evaluate the inhibitory activity of different compounds on the binding of PD-1 with PD-L1 and CD80 with PD-L1 in liquid phase in vitro, and the effect of different compounds on blocking the signals of inhibiting T cell activation after binding of PD-1 with PD-L1 on cell models.

### Example A Evaluation on inhibitory activity on the binding of PD-1 and PD-L1 in vitro

### The main principle of the experiment

Homogeneous Time-Resolved Fluorescence (HTRF): This method used the recombinant human PD-L1 protein fused to express the hFc tag and the recombinant human PD-1 protein fused to express the His tag, both of which were interacting ligands and receptors. When the anti-hFc antibody containing Eu element chelation marker and the anti-His antibody labeled with XL665 fluorescein were bound with the above two corresponding labels, and excited by a laser with a wavelength of 320 nm, the ligand-receptor binding enabled the energy to be transferred from the Eu element to the XL665 fluorescein, which was excited to emit light at a wavelength of 665 nm. However, when an inhibitor of the interaction between PD-L1 and PD-1 was added, the binding of the ligand to the receptor was destroyed, so that the distance between Eu and XL665 was far away, the energy cannot be transferred, and XL665 would not be excited.

### Experimental materials and equipment

Recombinant human PD-1 protein with His tag (His-PD-1 protein, Cat #: 10377-H08H-50), and recombinant human PD-L1-Fc fusion protein (PD-L1-Fc fusion protein, Cat #: 10084-H02H-100) were purchased from Sino Biological Inc., anti-hFc-Eu³⁺ antibody and anti-His-XL665 antibody were purchased from Cisbio Company, and other related reagents such as dilution buffer (Diluent buffer 5, Cat # : 62DL5DDC), detection buffer (PPI-Europium detection buffer, Cat #: 61DB9RDF) were purchased from Cisbio Company. Fluorescence detection instrument Tecan (Spark 10M) was purchased from Switzerland Tecan Company.

### The main process of the experiment

The experimental process was carried out in accordance with the procedures required by the instructions for use of the detection reagents. The process is as follows:
(1) Experimental preparation: The test compound was diluted with dilution buffer into different concentration gradients (the highest final concentration in 20 µL final reaction system was 10 µM), His-PD-1 protein was diluted to 800 nM (the final concentration in 20 µL final reaction system was 100 nM), and the PD-L1-Fc fusion protein was diluted to 16 nM (final concentration was 2 nM). The anti-His-XI,665 antibody and anti-hFc-Eu³⁺ antibody were respectively diluted 20 times and 100 times using the detection buffer according to the reagent requirements.
(2) 5 µL of test compound, 2.5 µL of PD-L1-Fc fusion protein and 2.5 µL of His-PD-1 protein solution were mixed, and incubated at room temperature for 15 min. Then the system was added with 5 µL of anti-His-XL665 antibody and 5 µL of anti-hFc-Eu³⁺ antibody, incubated for another 3 h, and then detected.
(3) When detecting the reaction, a control group was also provided, including a 0% inhibition positive control without adding test compounds and a 100% inhibition negative control without adding PD-1 protein. All detections were performed with duplicates.
(4) The fluorescence signal of each well was measured using a fluorescence detector Tecan (Spark 10M) at an excitation wavelength of 320 nm, and emission wavelengths of 620 nm and 665 nm, respectively. The intensity of the mutual binding of PD-1 and PD-L1 referred to the ratio of fluorescence signals Em665/Em620.
(5) The binding inhibition rate of the test compound was calculated as follows: Inhibition rate (%) = [1-(Fluorescence signal ratio of detection well-fluorescence signal ratio of 100% inhibition negative control)] / (fluorescence signal ratio of 0% inhibition positive control-fluorescence signal ratio of 100 % inhibition negative control) ×100%. After calculating the inhibition rate on PD-1/PD-L1 binding of the test compounds with different concentration gradients, the 50% inhibitory concentration (IC₅₀) was calculated. The IC₅₀ data of representative compounds of the present invention inhibiting the binding of PD-1 and PD-L1 in vitro are shown in Table 2 below:

**Table 2 IC₅₀ data of representative compounds of the present invention inhibiting PD-1/PD-L1 binding in vitro**

| Number of compound | IC₅₀ (nM) | Number of compound | IC₅₀ (nM) | Number of compound | IC₅₀ (nM) |
|---|---|---|---|---|---|
| I-1 | 0.72 | I-2 | 1.52 | I-3 | 2.90 |
| I-4 | 2.80 | I-5 | 1.02 | I-6 | 1.00 |
| I-7 | 1.38 | I-8 | 1.63 | I-9 | 3.77 |
| I-10 | 3.59 | I-11 | 4.21 | I-12 | 3.80 |
| I-13 | 1.23 | I-14 | 0.51 | | |

From the above results, it can be seen that the compounds of the present invention had good activity of inhibiting PD-1/PD-L1 in vitro, and the compounds represented by general formula (I) of the present invention also had the activity of inhibiting PD-1/PD-L1.

### Example B Evaluation on inhibitory activity on binding of CD80 and PD-L1 in vitro

### The main principle of the experiment

In addition to PD-1, PD-L1 can also exert immunosuppressive activity by binding to CD80. Similarly, in vitro experiments of binding or binding inhibition of CD80 and PD-L1 can also be measured by homogeneous time-resolved fluorescence (HTRF). When the anti-hFc-Eu³⁺ antibody and anti-His-XL665 antibody were bound to the hFC tag fused to PD-L1 and the His tag fused to CD80 respectively, and excited with a 320 nm wavelength laser, the binding of PD-L1 and CD80 enabled energy to be transferred from the Eu element to the XL665 fluorescein, which was excited to emit light. When the inhibitor of the interaction between PD-L1 and CD80 was added, the combination of the two was destroyed, so that the distance between Eu and XL665 was far away, the energy cannot be transferred, and XL665 would not be excited.

### Experimental materials and equipment

Recombinant human CD80 protein with His tag (His-CD80 protein, Cat #: 10698-H08H-100), and recombinant human PD-L1-Fc fusion protein (PD-L1-Fc fusion protein, Cat #: 10084-H02H-100) were purchased from Sino Biological Inc., anti-hFc-Eu³⁺ antibody and anti-His-XL665 antibody were purchased from Cisbio Company, and other related reagents such as dilution buffer (Diluent buffer 5, Cat # : 62DL5DDC), detection buffer (PPI-Europium detection buffer, Cat #: 61DB9RDF) were purchased from Cisbio Company. Fluorescence detection instrument Tecan (Spark 10M) was purchased from Switzerland Tecan Company.

### The main process of the experiment

The experimental process was carried out in accordance with the procedures required by the instructions for use of the detection reagents (Invitrogen). The process is as follows:
(1) Experimental preparation: The test compound was diluted with dilution buffer into different concentration gradients (the highest final concentration in 20 µL final reaction system was 10 µM), His-CD80 protein was diluted to 800 nM (the final concentration in 20 µL final reaction system was 100 nM), and the PD-L1-Fc fusion protein was diluted to 16 nM (final concentration was 2 nM). The anti-His-XI,665 antibody and anti-hFc-Eu³⁺ antibody were respectively diluted 20 times and 100 times using the detection buffer according to the reagent requirements.
(2) 5 µL of test compound, 2.5 µL of Hid-CD80 protein and 2.5 µL of PD-1-Fc fusion protein solution were mixed, and incubated at room temperature for 15 min. Then the system was added with 5 µL of anti-His-XL665 antibody and 5 µL of anti-hFc-Eu³⁺ antibody, incubated for another 3 h, and then detected.
(3) When detecting the reaction, a control group was provided, including a 0% inhibition positive control without adding test compounds and a 100% inhibition negative control without adding CD-80 protein. All detections were performed with duplicates.
(4) The fluorescence signal of each well was measured using a fluorescence detector Tecan (Spark 10M) at an excitation wavelength of 320 nm, and emission wavelengths of 620 nm and 665 nm, respectively. The intensity of the mutual binding of CD80 and PD-L1 referred to the ratio of fluorescence signals Em665/Em620.
(5) The binding inhibition rate of the test compound was calculated as follows: Inhibition rate (%) = [1-(Fluorescence signal ratio of detection well-fluorescence signal ratio of 100% inhibition negative control)] / (fluorescence signal ratio of 0% inhibition positive control-fluorescence signal ratio of 100 % inhibition negative control) ×100%. After calculating the inhibition rate on CD80/PD-L1 binding of the test compounds with different concentration gradients, the 50% inhibitory concentration (IC₅₀) was calculated. The IC₅₀ data of representative compounds of the present invention inhibiting the binding of CD80 and PD-L1 in vitro are shown in Table 3 below:

**Table 3 IC₅₀ data of representative compounds of the present invention inhibiting CD80/PD-L1 binding in vitro**

| Number of compound | IC₅₀ (nM) | Number of compound | IC₅₀ (nM) | Number of compound | IC₅₀ (nM) |
|---|---|---|---|---|---|
| I-1 | 0.58 | I-2 | 1.09 | I-3 | 1.78 |
| I-4 | 1.75 | I-5 | 1.12 | I-6 | 0.95 |
| I-7 | 1.94 | I-8 | 1.77 | I-9 | 3.42 |
| I-10 | 4.26 | I-11 | 3.21 | I-12 | 2.94 |
| I-13 | 1.44 | I-14 | 0.37 | | |

From the above results, it can be seen that the compounds of the present invention had good activity of inhibiting CD80/PD-L1 in vitro, and the compounds represented by general formula (I) of the present invention also had the activity of inhibiting CD80/PD-L1.

### Example C Evaluation on immune checkpoints PD-1 and PD-L1 mediated signals of inhibiting T cell activation at the cellular level

As an immune checkpoint molecule, PD-1 is mainly expressed on the surface of activated T cells, while its ligand PD-L1 is widely expressed. In addition to antigen-presenting cells such as dendritic cells, macrophages, and B cells, many tumor cells can also suppress anti-tumor immune effects by up-regulating the expression of PD-L1. For normal immune responses, in addition to activating T cells through immune co-stimulatory molecules, antigen-presenting cells also express PD-L1 ligand molecules, etc., which bind to PD-1 molecules on the surface of activated T cells, thereby inhibiting T cell activation and avoiding damage to surrounding normal tissues caused by the excessive proliferation and activation of T cells.

### The main principle of the experiment

In order to detect the effect of the interaction between PD-1 and PD-L1 on T cell activation signals in the immune response, CHO-PD-L1-CD3L cells stably expressing human PD-L1 molecules and anti-CD3 single-chain antibody (ScFv) and Jurkat-PD-1-NFAT cells stably expressing human PD-1 molecule and NFAT reporter gene were constructed. When the two kinds of cells were co-incubated, the binding of anti-CD3ScFv on the surface of CHO cells and the membrane CD3 molecules of Jurkat cells would transmit an activation signal to Jurkat cells, but simultaneously, the binding of PD-L1 on the surface of CHO cells and PD-1 molecules on the surface of Jurkat cells would transmit an inhibitory signal inward to prevent expression of the luciferase reporter gene. When immune checkpoint antibodies or small molecule inhibitors were added, the binding of PD-1 and PD-L1 was blocked, the NFAT pathway activated by T cell activation signal mediated by cross-linking of CD3ScFv antibody and CD3 was no longer affected by the inhibitory signal, and the downstream luciferase reporter gene started to be expressed. A chemiluminescent signal proportional to reporter gene activation was detected by adding a catalytic substrate.

### Experimental materials and equipment

CHO-PD-L1-CD3L cells expressing human PD-L1 molecule and anti-CD3 single-chain antibody (ScFv) and Jurkat-PD-1-NFAT cells stably expressing human PD-1 molecule and NFAT reporter gene were independently constructed and provided by Dr. Chen Bo (KeyMed Bioscience (Chengdu) Co., Ltd.). Puromycin (Cat # 540411) and Hygromycin B (Cat # V900372) for stable culture of transfected cells were purchased from Sigma, PMA (Cat # P1585) was purchased from Sigma, anti-human PD-L1 antibody (Cat # GMP-A066) was purchased from Novoprotein Company. Luciferase substrate solution (Cat # E6485) and luciferase-specific cell lysis solution 5× (Cat # E1531) were purchased from Promega. Fluorescence detection instrument Tecan (Spark 10M) was purchased from Switzerland Tecan Company.

### The main process of the experiment

(1) The day before the experiment, 100 µL of CHO-PD-L1-CD3L cells were inoculated in each well of a 96-well cell culture plate (about 4×10⁴ cells/well) with a medium of DMEM/F12 containing 10% FBS, 8 µg/mL puromycin and 200 µg/mL hygromycin B, and cultured overnight at 37 °C.
(2) The test compounds were diluted with 0.1% PBST into different concentration gradients, added into 96-well plates, and pre-incubated for 30 min. Jurkat-PD-1-NFAT cell count was adjusted to 2×10⁵ cells/mL with the RPMI 1640 complete medium containing 10% FBS, 8 µg/mL puromycin and 200 µg/mL hygromycin B. 100 ng/mL PMA (the stock solution was prepared with DMSO to a concentration of 10 mg/mL) was added to amplify T cell activation signal. 100 µL of the above Jurkat-PD-1-NFAT cells was added to each well of the 96-well plate for co-culture.
(3) When detecting the reaction, a control group was provided, including a solvent control without adding test compounds and a positive control with addition of anti-human PD-1 antibody as the experimental system. All detections were performed with duplicates.
(4) After continuous incubation at 37°C for 6 h, 40 µL of 5x cell lysate was directly added, and mixed well. The plate was placed at room temperature for 10 min to completely lyse the cells. 50 µL of the lysed cell solution was transferred to a fluorescence detection plate, and added with 30 µL of luciferase substrate solution. The chemiluminescence detection program on the fluorescence detector was immediately selected for detection.
(5) The inhibition rate of T cell activation signal at the cellular level of the test compounds was calculated as follows: Inhibition rate of T cell activation signal (%) = (the original value of chemiluminescence in the detection well - solvent control) / (the highest original value of chemiluminescence detected in the compound detection well- solvent control) × 100 %. After calculating the inhibitory rates of T cell activation signals by the test compounds with different concentration gradients, the 50% inhibitory concentration (EC₅₀) was further calculated. The EC₅₀ data of the compound of the present invention blocking PD-1/PD-L1-mediated T cell activation inhibitory signal are shown in Table 4:

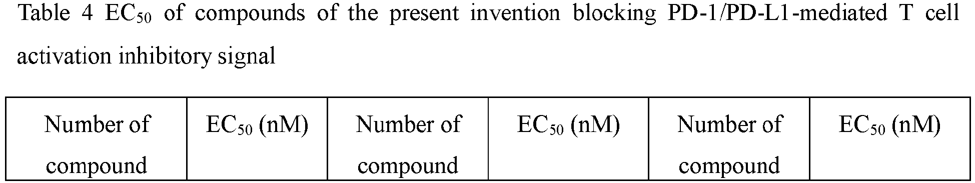

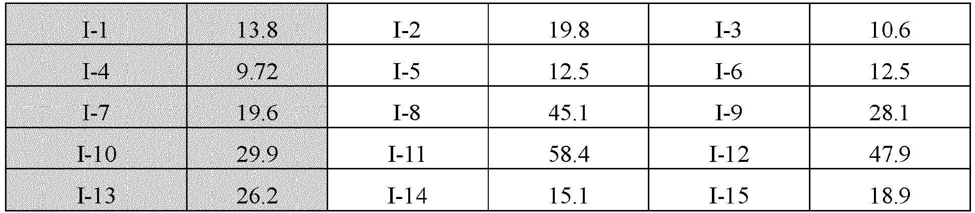

From the above results, it can be seen that the compounds of the present invention had the activity of effectively blocking the T cell activation inhibitory signal mediated by the immune checkpoint at the cellular level, and the compounds represented by general formula (I) of the present invention also had the ability of blocking PD-1/PD-L1-mediated T cell activation signal inhibitory activity.

### Example D Pharmacokinetic assay

Twenty-four female B-hPD-1/hPD-L1 mice aged 6-8 weeks were purchased from Biocytogen Jiangsu Gene Biotechnology Co., Ltd. The mice were randomly divided into 4 groups, 6 mice in each group. The test compounds were prepared in a vehicle containing 5% DMSO, 60% PEG400 and 35% purified water, including I-1 and 3 control molecules Example 17, Compound 14 (INCB086550) , and Example 180. The compounds were administered orally at 50 mg/kg, once a day. The blood was collected alternately through the fundus venous plexus after the administration on the 18th day. The blood collection time points were 15 min, 30 min, 1 h, 2 h, 4h, 8h, 24h and 32h. About 0.1 mL of blood was collected in a centrifuge tube (anticoagulated with sodium heparin), and centrifuged at 5000 rpm for 5 min. Then the plasma was separated, and frozen at -20 °C for later detection. After the plasma samples were processed, the concentration of the compounds in the plasma was determined by liquid chromatography-mass spectrometry (LC-MS/MS). The pharmacokinetic parameters were calculated using Phoenix WinNonlin 7.0. The data is summarized in Table 5.

**Table 5 Characteristics of pharmacokinetic parameters in humanized mice after repeated administration of different compounds**

| Compound | AUC₍₀₋ₜ₎(h*µg/mL) | T_{1/2}(h) | MRT (h) |
|---|---|---|---|
| I-1 | 122.6 | 10.3 | 13.5 |
| ^{a}Example 17 | 67.4 | 4.2 | 5.8 |
| ^{b}Compound 14 (INCB086550) | 15.8 | 3.9 | 5.1 |
| ^{c}Example180 | 33.7 | 3.8 | 5.6 |

"Example 17 is a compound disclosed by Incyte on page 64 of the patent document WO2019/217821. The inventor synthesized the compound by referring to the synthesis method therein, and used it as a reference molecule. The chemical structure of Example 17 was confirmed by LC-MS MS-ESI (m/z) 911.4 [M+H]⁺. Due to the poor solubility of Example 17, a sodium hydroxide solution which helped to dissolve was added to the NMR sample (the molar ratio of Example 17 to NaOH was 1:2), and MeOD was subsequently added thereto for structure confirmation. ¹H-NMR (400 MHz, MeOD) *δ* ppm 8.45 (dd, *J* = 8.3, 1.2 Hz, 2H), 7.43 (t, *J* = 8.0 Hz, 2H), 7.12-7.07 (m, 2H), 3.95 (d, *J* = 14.4 Hz, 6H), 3.55 (s, 4H), 2.96-2.84 (m, 4H), 2.72-2.82 (m, 4H), 2.70-2.62 (m, 4H), 2.00- 1.88 (m, 4H), 1.87-1.78 (m, 4H), 1.66-1.37 (m, 16H). The structural formula of the compound is as follows:

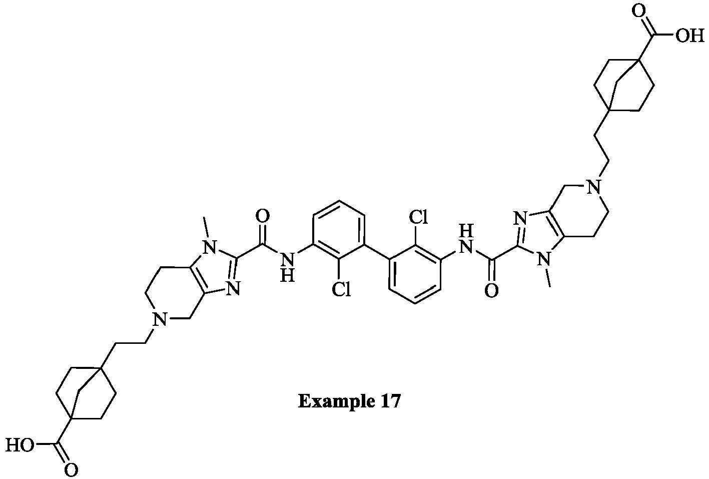

^{b}Compound 14 (INCB086550) is a compound disclosed by Incyte in Table 2 of the patent CN110267953A. The inventor synthesized the compound by referring to the synthesis method therein, and used it as a reference molecule. Compound 14 (INCB086550) is a small molecule PD-L1 inhibitor with the fastest clinical progress, which is currently in phase 2 research.

The chemical structure of compound 14 (INCB086550) was confirmed by LC-MS MS-ESI (m/z) 694.2 [M+H]⁺ and ¹H-NMR (400 MHz, DMSO) *δ* ppm 9.31 (s, 1H), 8.84 (s, 1H), 8.48 (d, *J* = 8.0 Hz, 1H), 8.17 (s, 1H), 8.13 (d, *J* = 7.7 Hz, 1H), 8.09-8.03 (m, 2H), 7.83 (s, 1H), 7.54 (t, *J* = 7.7 Hz, 1H), 7.43 (d, *J* = 7.4 Hz, 1H), 7.34 (t, *J* = 7.9 Hz, 1H), 7.18 (d, *J* = 5.8 Hz, 1H), 6.91 (d, *J* = 7.4 Hz, 1H), 4.76 (s, 1H), 4.26-4.18 (m, 1H), 3.85-3.65 (m, 4H), 2.84-2.69 (m, 3H), 2.69-2.60 (m, 2H), 2.58-2.46 (m, 3H), 2.45 (s, 3H), 2.38 (dd, *J* = 9.6, 3.6 Hz, 1H), 2.08 (s, 3H), 2.05-1.95 (m, 2H), 1.94-1.83 (m, 1H), 1.63-1.52 (m, 1H). The structural formula of the compound is as follows:

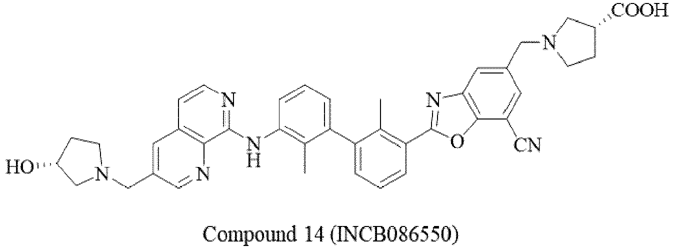

^{c}Example 180 is an example disclosed by Incyte Company in patent CN110267953A. The inventor synthesized this compound by referring to the synthesis method therein, and used it as a reference molecule. The chemical structure of Example 180 was confirmed by LC-MS MS-ESI (m/z) 775.0 [M+H]⁺and ¹H-NMR (400 MHz, DMSO) *δ* ppm 9.90 (s, 2H), 8.38 (dd, *J* = 7.6, 2.7 Hz, 2H), 7.49 (t, *J* = 8.0 Hz, 2H), 7.14 (d, *J* = 7.7 Hz, 2H), 3.90 (s, 6H), 3.57 (t, *J* = 6.0 Hz, 2H), 3.51-3.46 (m, 4H), 2.85-2.77 (m, 4H), 2.70-2.64 (m, 4H), 2.64-2.59 (m, 4H), 1.90-1.76 (m, 2H), 1.68-1.55 (m, 2H), 1.54-1.22 (m, 6H). The structural formula of the compound is as follows:

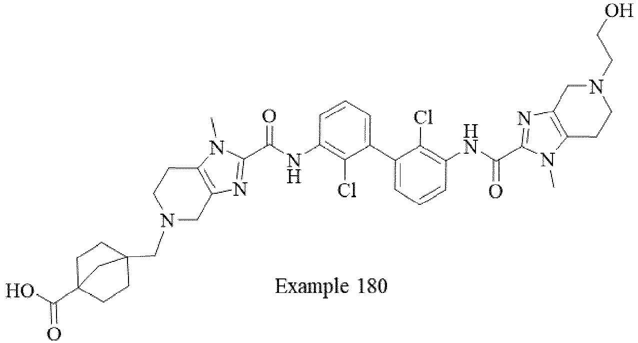

It can be seen from the above results that, after repeated administration of 50 mg/kg of representative compounds of the present invention, in vivo plasma exposure (AUC₍₀₋ₜ₎), in vivo mean residence time (MRT) and half-life (T_{1/2}) in mice were significantly higher than those of the three control molecules. The repeated administration can better reflect the pharmacokinetic characteristics under clinical treatment conditions. Compared with the reference molecules, the compounds of the present invention had unexpected increase in the in vivo exposure and sustained exposure time (AUC/T_{1/2}) after repeated administration, which is beneficial to better exert anti-tumor activity in clinical treatment.

### Example E Tumor tissue distribution assay

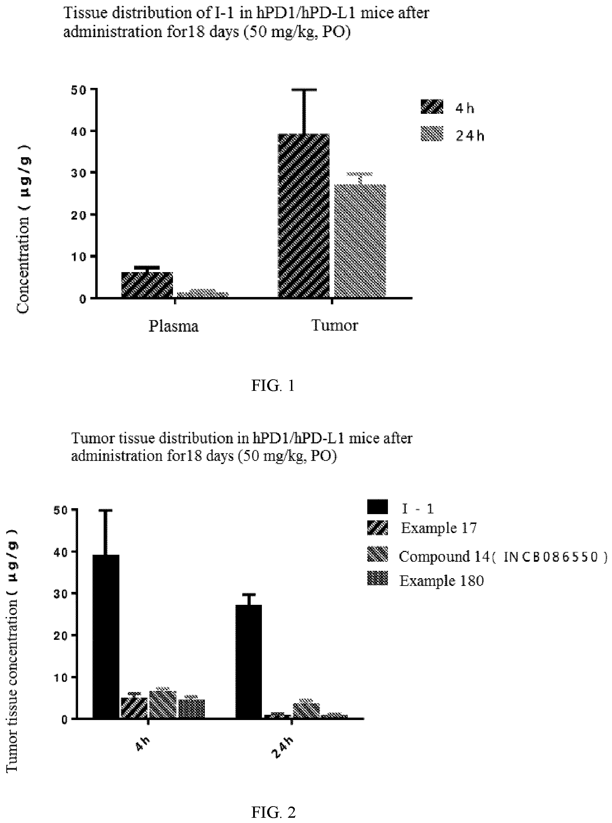

16 B-hPD-1/hPD-L1 humanized female mice, aged 6-8 weeks were purchased from Biocytogen Jiangsu Gene Biotechnology Co., Ltd. After 1 week of adaptation, the mice were subcutaneously inoculated with MC38-PD-L1 cells at 2×10⁶ cells/site. After the tumor grew to about 200 m³, the mice were divided into 4 groups, 4 mice in each group. The test compounds were formulated in a vehicle containing 5% DMSO, 60% PEG400 and 35% purified water, including I-1 and 3 control molecules Example 17, Compound 14 (INCB086550), and Example 180. The compounds were orally administered at 50 mg/kg, once/day. The blood and tumor tissue were collected 4 h and 24 h after administration on the 18th day. A certain amount of tissue was weighed, and added with phosphate-buffered saline (PBS) solution for homogenization. After the sample was processed, it was analyzed by LC-MS/MS to determine the concentration of the compound in plasma and tissue. The experimental results are shown in Table 6, FIG. 1 and FIG. 2.

**Table 6 Tumor tissue distribution in humanized mice after repeated administration of different compounds**

| Tumor tissue | Time | I-1 | Example 17 | Compound 14(INCB086550) | Exampl e 180 |
|---|---|---|---|---|---|
| Average concentration (µg/g) | 4h | 39.2 | 5.0 | 6.6 | 4.5 |
| | 24 h | 27.2 | 1.0 | 3.6 | 0.9 |
| Ratio of I-1 to control molecule | 4h | 1.0 | 7.8 | 5.9 | 8.7 |
| | 24 h | 1.0 | 27.2 | 7.6 | 30.2 |

It can be seen from the above results that after repeated administration, the concentration of the representative compound of the present invention in tumor tissue was significantly higher than that in plasma, that is, the concentration in tumor tissue at 4 h and 24 h was 4-6 times and 17-27 times that of plasma. Meanwhile, under the same administration conditions, the tumor tissue concentration at 4 h and 24 h was 5-8 times and 7-30 times that of the control molecule, indicating that the compounds of the present invention showed good tumor tissue targeting. Compared to control molecules, the compounds of the present invention had unexpected enrichment and targeting effects on tumor tissue.

### Industrial applicability

The biphenyl compounds of the present invention have excellent PD-L1 inhibitory activity, and can be used as a drug for treating or preventing diseases related to this effect.

The above are only preferred embodiments of the present invention. It should be noted that, for those of ordinary skill in the art, without departing from the principle of the present invention, several improvements and modifications can also be made, and these improvements and modifications should also be regarded as the protection scope of the present invention.

## Claims

1. A compound represented by formula (I), a stereoisomer, a pharmaceutically acceptable salt, a precursor or a metabolite thereof, 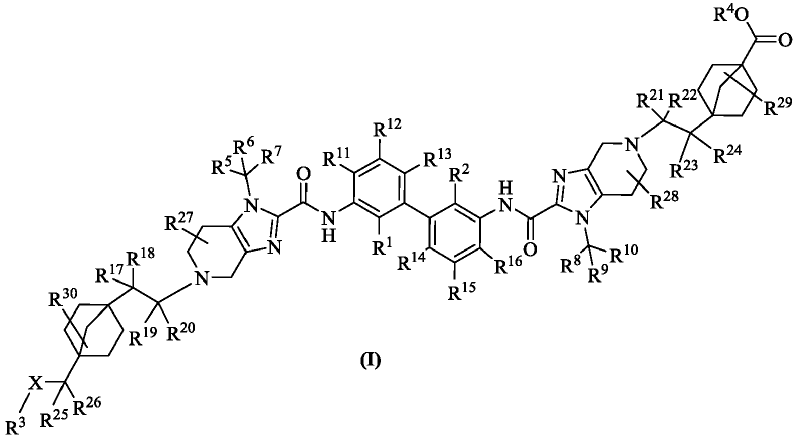 wherein,
R¹ and R² are the same or different, and are selected from the group consisting of C₁-C₆ alkyl, cyano and halogen;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono- C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, 3- to 14-membered heterocycloalkyl, C₃-C₁₄ cycloalkyl-C₁-C₄ alkyl, and 3- to 14-membered heterocycloalkyl-C₁-C₄ alkyl;
R⁴ is selected from the group consisting of hydrogen and C₁-C₆ alkyl, wherein the C₁-C₆ alkyl is optionally substituted by one or more substituents selected from the group consisting of hydroxyl, carboxyl and halogen;
X is selected from the group consisting of -O- and -S-;
R⁵-R²⁶ are selected from the group consisting of hydrogen atom;
R²⁷-R³⁰ are selected from the group consisting of hydrogen atom;
wherein, at least one hydrogen atom in each group of the R¹-R³⁰ defined above is replaced by deuterium (D).

2. The compound, stereoisomer, pharmaceutically acceptable salt, precursor or metabolite thereof according to claim 1,
wherein,
R¹ and R² are the same or different, and are selected from the group consisting of methyl, methyl-*d*₃ (CD₃), cyano, fluorine, chlorine, and bromine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono- C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, 3- to 14-membered heterocycloalkyl, C₃-C₁₄ cycloalkyl-C₁-C₄ alkyl, and 3- to 14-membered heterocycloalkyl-C₁-C₄ alkyl;
R⁴ is selected from the group consisting of hydrogen and C₁-C₆ alkyl, wherein the C₁-C₆ alkyl is optionally substituted by one or more substituents selected from the group consisting of hydroxyl and halogen;
X is selected from the group consisting of -O-;
R⁵-R²⁶ are selected from the group consisting of hydrogen atom, wherein at least one hydrogen atom is replaced by deuterium (D);
R²⁷-R³⁰ are selected from the group consisting of hydrogen atom, preferably at least one hydrogen atom is replaced by deuterium (D), wherein the deuterium is at any position on the corresponding alicyclic ring and aliphatic heterocyclic ring.

3. The compound, stereoisomer, pharmaceutically acceptable salt, precursor or metabolite thereof according to claim 1,
wherein,
R¹ and R² are the same or different, and are selected from the group consisting of methyl, methyl-*d*₃ (CD₃), cyano, fluorine, chlorine, and bromine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono- C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, 3- to 14-membered heterocycloalkyl, C₃-C₁₄ cycloalkyl-C₁-C₄ alkyl, and 3- to 14-membered heterocycloalkyl-C₁-C₄ alkyl;
R⁴ is selected from the group consisting of hydrogen and C₁-C₆ alkyl, wherein the C₁-C₆ alkyl is optionally substituted by one or more substituents selected from the group consisting of hydroxyl and halogen;
X is selected from the group consisting of -O-;
R⁵-R²⁶ are selected from the group consisting of hydrogen atom, preferably at least one hydrogen atom is replaced by deuterium (D);
R²⁷-R³⁰ are selected from the group consisting of hydrogen atom, wherein at least one hydrogen atom is replaced by deuterium (D), wherein the deuterium is at any position on the corresponding alicyclic ring and aliphatic heterocyclic ring.

4. The compound, stereoisomer, pharmaceutically acceptable salt, precursor or metabolite thereof according to claim 1,
wherein,
R¹ and R² are the same or different, and are selected from the group consisting of methyl, methyl-*d*₃ (CD₃), cyano, fluorine and chlorine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, mono- C₁-C₆ alkylamino C₁-C₆ alkyl, bis-C₁-C₆ alkylamino C₁-C₆ alkyl, halogenated C₁-C₆ alkoxy C₁-C₆ alkyl, C₃-C₁₄ cycloalkyl, and 3- to 14-membered heterocycloalkyl; and at least one hydrogen atom in these groups is replaced by deuterium (D);
R⁴ is selected from the group consisting of hydrogen and C₁-C₆ alkyl, wherein the C₁-C₆ alkyl is optionally substituted by halogen;
X is selected from the group consisting of -O-;
R⁵-R²⁶ are selected from the group consisting of hydrogen atom, preferably at least one hydrogen atom is replaced by deuterium (D);
R²⁷-R³⁰ are selected from the group consisting of hydrogen atom, preferably at least one hydrogen atom is replaced by deuterium (D), wherein the deuterium is at any position on the corresponding alicyclic ring and aliphatic heterocyclic ring.

5. The compound, stereoisomer, pharmaceutically acceptable salt, precursor or metabolite thereof according to claim 1,
wherein,
R¹ and R² are the same or different, and are selected from the group consisting of methyl, methyl-*d*₃ (CD₃), cyano, fluorine and chlorine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl;
R⁴ is selected from the group consisting of hydrogen and C₁-C₆ alkyl, wherein the C₁-C₆ alkyl is optionally substituted by halogen;
X is selected from the group consisting of -O-;
R⁵-R²⁶ are hydrogen atom, and at least one hydrogen atom is replaced by deuterium (D);
R²⁷-R³⁰ are selected from the group consisting of hydrogen atom, preferably at least one hydrogen atom is replaced by deuterium (D), wherein the deuterium is at any position on the corresponding alicyclic ring and aliphatic heterocyclic ring.

6. The compound, stereoisomer, pharmaceutically acceptable salt, precursor or metabolite thereof according to claim 1,
wherein,
R¹ and R² are the same or different, and are selected from the group consisting of methyl, methyl-*d*₃ (CD₃), cyano, fluorine and chlorine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl;
R⁴ is selected from the group consisting of hydrogen and C₁-C₆ alkyl, wherein the C₁-C₆ alkyl is optionally substituted by halogen;
X is selected from the group consisting of -O-;
R⁵-R²⁶ are selected from the group consisting of hydrogen atom, preferably at least one hydrogen atom is replaced by deuterium (D);
R²⁷-R³⁰ are selected from the group consisting of hydrogen atom, and at least one hydrogen atom is replaced by deuterium (D), wherein the deuterium is at any position on the corresponding alicyclic ring and aliphatic heterocyclic ring.

7. The compound, stereoisomer, pharmaceutically acceptable salt, precursor or metabolite thereof according to claim 1,
wherein,
R¹ and R² are the same or different, and are selected from the group consisting of methyl, methyl-*d*₃ (CD₃), cyano, fluorine and chlorine;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl, and at least one hydrogen atom in these groups is replaced by deuterium (D) ;
R⁴ is selected from the group consisting of hydrogen;
X is selected from the group consisting of -O-;
R⁵-R²⁶ are selected from the group consisting of hydrogen atom, preferably at least one hydrogen atom is replaced by deuterium (D);
R²⁷-R³⁰ are selected from the group consisting of hydrogen atom, preferably at least one hydrogen atom is replaced by deuterium (D), wherein the deuterium is at any position on the corresponding alicyclic ring and aliphatic heterocyclic ring.

8. The compound, stereoisomer, pharmaceutically acceptable salt, precursor or metabolite thereof according to claim 1,
wherein, R¹ and R² are the same or different, and are selected from the group consisting of methyl, cyano, and halogen;
R³ is selected from the group consisting of hydrogen, C₁-C₆ alkyl, C₂-C₆ alkenyl C₁-C₆ alkyl, C₂-C₆ alkynyl C₁-C₆ alkyl, halogenated C₁-C₆ alkyl, C₁-C₆ alkoxy C₁-C₆ alkyl, and halogenated C₁-C₆ alkoxy C₁-C₆ alkyl, and at least one hydrogen atom in these groups is replaced by deuterium (D);
R⁴ is selected from the group consisting of hydrogen and C₁-C₆ alkyl;
X is selected from the group consisting of -O-;
R⁵-R²⁶ are selected from the group consisting of hydrogen atom;
R²⁷-R³⁰ are selected from the group consisting of hydrogen atom.

9. The compound, stereoisomer, pharmaceutically acceptable salt, precursor or metabolite thereof according to claim 8,
wherein, R¹ and R² are the same or different, and are selected from the group consisting of methyl, cyano, fluorine, and chlorine;
preferably, R¹ and R² are the same or different, and are selected from the group consisting of methyl, fluorine, and chlorine;
preferably, R¹ and R² are the same or different, and are selected from the group consisting of methyl, and chlorine.

10. The compound, stereoisomer, pharmaceutically acceptable salt, precursor or metabolite thereof according to claim 8,
wherein, R³ is selected from the group consisting of hydrogen, C₁-C₆ alkyl, and halogenated C₁-C₆ alkyl, wherein at least one hydrogen atom in these groups is replaced by deuterium (D);
preferably, R³ is selected from the group consisting of C₁-C₆ alkyl, wherein at least one hydrogen atom in the C₁-C₆ alkyl is replaced by deuterium (D);
preferably, R³ is selected from the group consisting of methyl, ethyl, propyl, butyl, and pentyl, wherein at least one hydrogen atom in these groups is replaced by deuterium (D).

11. The compound, stereoisomer, pharmaceutically acceptable salt, precursor or metabolite thereof according to claim 10,
wherein, 1-15 hydrogen atoms in the groups defined by R³ are replaced by deuterium (D), preferably 1-9 hydrogen atoms are replaced by deuterium (D).

12. The compound, stereoisomer, pharmaceutically acceptable salt, precursor or metabolite thereof according to claim 8,
wherein, R⁴ is selected from the group consisting of hydrogen, methyl, ethyl, propyl, butyl, and pentyl;
preferably, R⁴ is selected from the group consisting of hydrogen.

13. The compound, stereoisomer, pharmaceutically acceptable salt, precursor or metabolite thereof according to any one of claims 1-12, wherein the compound is selected from the group consisting of:
4-(2-(2-((2,2'-dichloro-3'-(5-(2-(4-((methoxy-*d*₃)methyl)bicyclo[2.2.1]heptane-1-yl)ethyl)-1 -methyl-4,5,6,7-tetrahydro-1H-imidazo[4,5-c]pyridine-2-carboxamido)-[1,1'-biphenyl]-3-yl)carb amoyl)-1-methyl-1,4,6,7-tetrahydro-5H-imidazo[4,5-c]pyridin-5-yl)ethyl)bicyclo[2.2.1]heptane-1 -carboxylic acid;
4-(2-(2-((2-chloro-2'-fluoro-3'-(5-(2-(4-((methoxy-*d*₃)methyl)bicyclo[2.2.1]heptane-1-yl)eth yl)-1-methyl-4,5,6,7-tetrahydro-1H-imidazo[4,5-c]pyridine-2-carboxamido)-[1,1'-biphenyl]-3-yl) carbamoyl)-1-methyl-1,4,6,7-tetrahydro-5H-imidazo[4,5-c]pyridin-5-yl)ethyl)bicyclo[2.2.1]hepta ne-1-carboxylic acid;
4-(2-(2-((2'-chloro-3'-(5-(2-(4-((methoxy-*d*₃)methyl)bicyclo[2.2.1]heptane-1-yl)ethyl)-1-met hyl-4,5,6,7-tetrahydro-1H-imidazo[4,5-c]pyridine-2-carboxamido)-2-methyl-[1,1'-biphenyl]-3-yl) carbamoyl)-1-methyl-1,4,6,7-tetrahydro-5H-imidazo[4,5-c]pyridin-5-yl)ethyl)bicyclo[2.2.1]hepta ne-1-carboxylic acid;
4-(2-(2-((2-chloro-3'-(5-(2-(4-((methoxy-*d*₃)methyl)bicyclo[2.2.1]heptane-1-yl)ethyl)-1-met hyl-4,5,6,7-tetrahydro-1H-imidazo[4,5-c]pyridine-2-carboxamido)-2'-methyl-[1,1'-biphenyl]-3-yl )carbamoyl)-1-methyl-1,4,6,7-tetrahydro-5H-imidazo[4,5-c]pyridin-5-yl)ethyl)bicyclo[2.2.1]hept ane-1-carboxylic acid;
4-(2-(2-((2,2'-dichloro-3'-(5-(2-(4-((methoxy-*d*₃)methyl)bicyclo[2.2.1]heptane-1-yl)ethyl)-1-(methyl-*d*₃)-4,5,6,7-tetrahydro-1H-imidazo[4,5-c]pyridine-2-carboxamido)-[1,1'-biphenyl]-3-yl)c arbamoyl)-1-(methyl-*d*₃)-1,4,6,7-tetrahydro-5H-imidazo[4,5-c]pyridin-5-yl)ethyl)bicyclo[2.2.1]h eptane-1-carboxylic acid;
4-(2-(2-((2,2'dichloro-3'-(5-(2-(4-(methoxymethyl)bicyclo[2.2.1]heptane-1-yl)ethyl)-1-(met hyl-*d*₃)-4,5,6,7-tetrahydro-1H-imidazo[4,5-c]pyridine-2-carboxamido)-[1,1'-biphenyl]-3-yl)carba moyl)-1-(methyl-*d*₃)-1,4,6,7-tetrahydro-5H-imidazo[4,5-c]pyridin-5-yl)ethyl)bicyclo[2.2.1]hepta ne-1-carboxylic acid;
4-(2-(2-((2-chloro-2'-fluoro-3'-(5-(2-(4-((methoxy-*d*₃)methyl)bicyclo[2.2.1]heptane-1-yl)eth yl)-1-(methyl-*d*₃)-4,5,6,7-tetrahydro-1H-imidazo[4,5-c]pyridine-2-carboxamido)-[1,1'-biphenyl]-3-yl)carbamoyl)-1-(methyl-*d*₃)-1,4,6,7-tetrahydro-5H-imidazo[4,5-c]pyridin-5-yl)ethyl)bicyclo[2. 2.1]heptane-1-carboxylic acid;
4-(2-(2-((2-chloro-2'-fluoro-3'-(5-(2-(4-(methoxymethyl)bicyclo[2.2.1]heptane-1-yl)ethyl)-1 -(methyl-*d*₃)-4,5,6,7-tetrahydro-1H-imidazo[4,5-c]pyridine-2-carboxamido)-[1,1'-biphenyl]-3-yl) carbamoyl)-1-(methyl-*d*₃)-1,4,6,7-tetrahydro-5H-imidazo[4,5-c]pyridin-5-yl)ethyl)bicyclo[2.2.1] heptane-1-carboxylic acid;
4-(2-(2-((2-chloro-3'-(5-(2-(4-((methoxy-*d*₃)methyl)bicyclo[2.2.1]heptane-1-yl)ethyl)-1-(met hyl-*d*₃)-4,5,6,7-tetrahydro-1H-imidazo[4,5-c]pyridine-2-carboxamido)-2'-methyl-[1,1'-biphenyl]-3-yl)carbamoyl)-1-(methyl-*d*₃)-1,4,6,7-tetrahydro-5H-imidazo[4,5-c]pyridin-5-yl)ethyl)bicyclo[2. 2.1]heptane-1-carboxylic acid;
4-(2-(2-((2-chloro-3'-(5-(2-(4-(methoxymethyl)bicyclo[2.2.1]heptane-1-yl)ethyl)-1-(methyl-*d*₃)-4,5,6,7-tetrahydro-1H-imidazo[4,5-c]pyridine-2-carboxamido)-2'-methyl-[1,1'-biphenyl]-3-yl )carbamoyl)-1-(methyl-*d*₃)-1,4,6,7-tetrahydro-5H-imidazo[4,5-c]pyridin-5-yl)ethyl)bicyclo[2.2.1] heptane-1-carboxylic acid;
4-(2-(2-((2'-chloro-3'-(5-(2-(4-((methoxy-*d*₃)methyl)bicyclo[2.2.1]heptane-1-yl)ethyl)-1-(me thyl-*d*₃)-4,5,6,7-tetrahydro-1H-imidazo[4,5-c]pyridine-2-carboxamido)-2-methyl-[1,1'-biphenyl]-3-yl)carbamoyl)-1-(methyl-*d*₃)-1,4,6,7-tetrahydro-5H-imidazo[4,5-c]pyridin-5-yl)ethyl)bicyclo[2. 2.1]heptane-1-carboxylic acid;
4-(2-(2-((2'-chloro-3'-(5-(2-(4-(methoxymethyl)bicyclo[2.2.1]heptane-1-yl)ethyl)-1-(methyl-*d*₃)-4,5,6,7-tetrahydro-1H-imidazo[4,5-c]pyridine-2-carboxamido)-2-methyl-[1,1'-biphenyl]-3-yl) carbamoyl)-1-(methyl-*d*₃)-1,4,6,7-tetrahydro-5H-imidazo[4,5-c]pyridin-5-yl)ethyl)bicyclo[2.2.1] heptane-1-carboxylic acid;
4-(2-(2-((2,2'-dichloro-3'-(5-(2-(4-((methoxy-*d*₃)methyl)bicyclo[2.2.1]heptane-1-yl)ethyl)-1-(methyl-*d*₃)-4,5,6,7-tetrahydro-1H-imidazo[4,5-c]pyridine-2-carboxamido)-[1,1'-biphenyl]-3-yl-4 ,4',5,5',6,6'-*d*₆)carbamoyl)-1-(methyl-*d*₃)-1,4,6,7-tetrahydro-5H-imidazo[4,5-c]pyridin-5-yl)ethyl) bicyclo[2.2.1]heptane-1-carboxylic acid;
4-(2-(2-((2-chloro-2'-fluoro-3'-(5-(2-(4-((methoxy-*d*₃)methyl)bicyclo[2.2.1]heptane-1-yl)eth yl)-1-(methyl-*d*₃)-4,5,6,7-tetrahydro-1H-imidazo[4,5-c]pyridine-2-carboxamido)-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl) ethyl)bicyclo[2.2.1]heptane-1-carboxylic acid;
4-(2-(2-((2'-chloro-3'-(5-(2-(4-((methoxy-*d*₃)methyl)bicyclo[2.2.1]heptane-1-yl)ethyl)-1-(me thyl-*d*₃)-4,5,6,7-tetrahydro-1H-imidazo[4,5-c]pyridine-2-carboxamido)-2-methyl-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl) ethyl)bicyclo[2.2.1]heptane-1-carboxylic acid;
4-(2-(2-((2-chloro-3'-(5-(2-(4-((methoxy-*d*₃)methyl)bicyclo[2.2.1]heptane-1-yl)ethyl)-1-(met hyl-*d*₃)-4,5,6,7-tetrahydro-1H-imidazo[4,5-c]pyridine-2-carboxamido)-2'-methyl-[1,1'-biphenyl]-3-yl-4,4',5,5',6,6'-d6)carbamoyl)-1-(methyl-*d*₃)-1,4,6,7-tetrahydro-5H-imidazo[4,5-c]pyridin-5-yl )ethyl)bicyclo[2.2.1]heptane-1-carboxylic acid;
4-(2-(2-((2,2'-dichloro-3'-(5-(2-(4-(methoxymethyl)bicyclo[2.2.1]heptane-1-yl)ethyl)-1-(met hyl-*d*₃)-4,5,6,7-tetrahydro-1H-imidazo[4,5-c]pyridine-2-carboxamido)-[1,1'-biphenyl]-3-yl-4,4',5, 5',6,6'-*d*₆)carbamoyl)-1-(methyl-*d*₃)-1,4,6,7-tetrahydro-5H-imidazo[4,5-c]pyridin-5-yl)ethyl)bicyc lo[2.2.1]heptane-1-carboxylic acid;
4-(2-(2-((2-chloro-2'-fluoro-3'-(5-(2-(4-(methoxymethyl)bicyclo[2.2.1]heptane-1 -yl)ethyl)-1 -(methyl-*d*₃)-4,5,6,7-tetrahydro-1H-imidazo[4,5-c]pyridine-2-carboxamido)-[1,1'-biphenyl]-3-yl-4,4',5,5',6,6'-d₆)carbamoyl)-1-(methyl-*d*₃)-1,4,6,7-tetrahydro-5H-imidazo[4,5-c]pyridin-5-yl)ethyl )bicyclo[2.2.1]heptane-1-carboxylic acid;
4-(2-(2-((2'-chloro-3'-(5-(2-(4-(methoxymethyl)bicyclo[2.2.1]heptane-1-yl)ethyl)-1-(methyl-*d*₃)-4,5,6,7-tetrahydro-1H-imidazo[4,5-c]pyridine-2-carboxamido)-2-methyl-[1,1'-biphenyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl )bicyclo[2.2.1]heptane-1-carboxylic acid;
4-(2-(2-((2-chloro-3'-(5-(2-(4-(methoxymethyl)bicyclo[2.2.1]heptane-1-yl)ethyl)-1-(methyl-*d*₃)-4,5,6,7-tetrahydro-1H-imidazo[4,5-c]pyridine-2-carboxamido)-2'-methyl-[1,1'-biphenyl]-3-yl -4,4',5,5',6,6'-d₆)carbamoyl)-1-(methyl-*d*₃)-1,4,6,7-tetrahydro-5H-imidazo[4,5-c]pyridin-5-yl)ethy l)bicyclo[2.2.1]heptane-1-carboxylic acid;
4-(2-(2-((2'-chloro-3'-(5-(2-(4-((methoxy-*d*₃)methyl)bicyclo[2.2.1]heptane-1-yl)ethyl)-1-(me thyl-*d*₃)-4,5,6,7-tetrahydro-1H-imidazo[4,5-c]pyridine-2-carboxamido)-2-(methyl-*d*₃)-[1,1'-biphe nyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid;
4-(2-(2-((2-chloro-3'-(5-(2-(4-((methoxy-*d*₃)methyl)bicyclo[2.2.1]heptane-1-yl)ethyl)-1-(met hyl-*d*₃)-4,5,6,7-tetrahydro-1H-imidazo[4,5-c]pyridine-2-carboxamido)-2'-(methyl-*d*₃)-[1,1'-biphe nyl]-3-yl-4,4',5,5',6,6'-*d*₆)carbamoyl)-1-(methyl-*d*₃)-1,4,6,7-tetrahydro-5H-imidazo[4,5-c]pyridin-5-yl)ethyl)bicyclo[2.2.1]heptane-1-carboxylic acid;
4-(2-(2-((2-chloro-2'-fluoro-3'-(5-(2-(4-((methoxy-*d*₃)methyl)bicyclo[2.2.1]heptane-1-yl)eth yl)-1-methyl-4,5,6,7-tetrahydro-1H-imidazo[4,5-c]pyridine-2-carboxamido)-[1,1'-biphenyl]-3-yl-4,4',5,5',6,6'-*d*₆)carbamoyl)-1-methyl-1,4,6,7-tetrahydro-5H-imidazo[4,5-c]pyridin-5-yl)ethyl)bic yclo[2.2.1]heptane-1-carboxylic acid;
4-(2-(2-((2-chloro-2'-fluoro-3'-(5-(2-(4-(methoxymethyl)bicyclo[2.2.1]heptane-1-yl)ethyl)-1 -methyl-4,5,6,7-tetrahydro-1H-imidazo[4,5-c]pyridine-2-carboxamido)-[1,1'-biphenyl]-3-yl-4,4', 5,5',6,6'-*d*₆)carbamoyl)-1-methyl-1,4,6,7-tetrahydro-5H-imidazo[4,5-c]pyridin-5-yl)ethyl)bicyclo [2.2.1]heptane-1-carboxylic acid.

14. A method for preparing the compound, stereoisomer, pharmaceutically acceptable salt, precursor or metabolite thereof according to any one of claims 1-13, comprising the following steps: 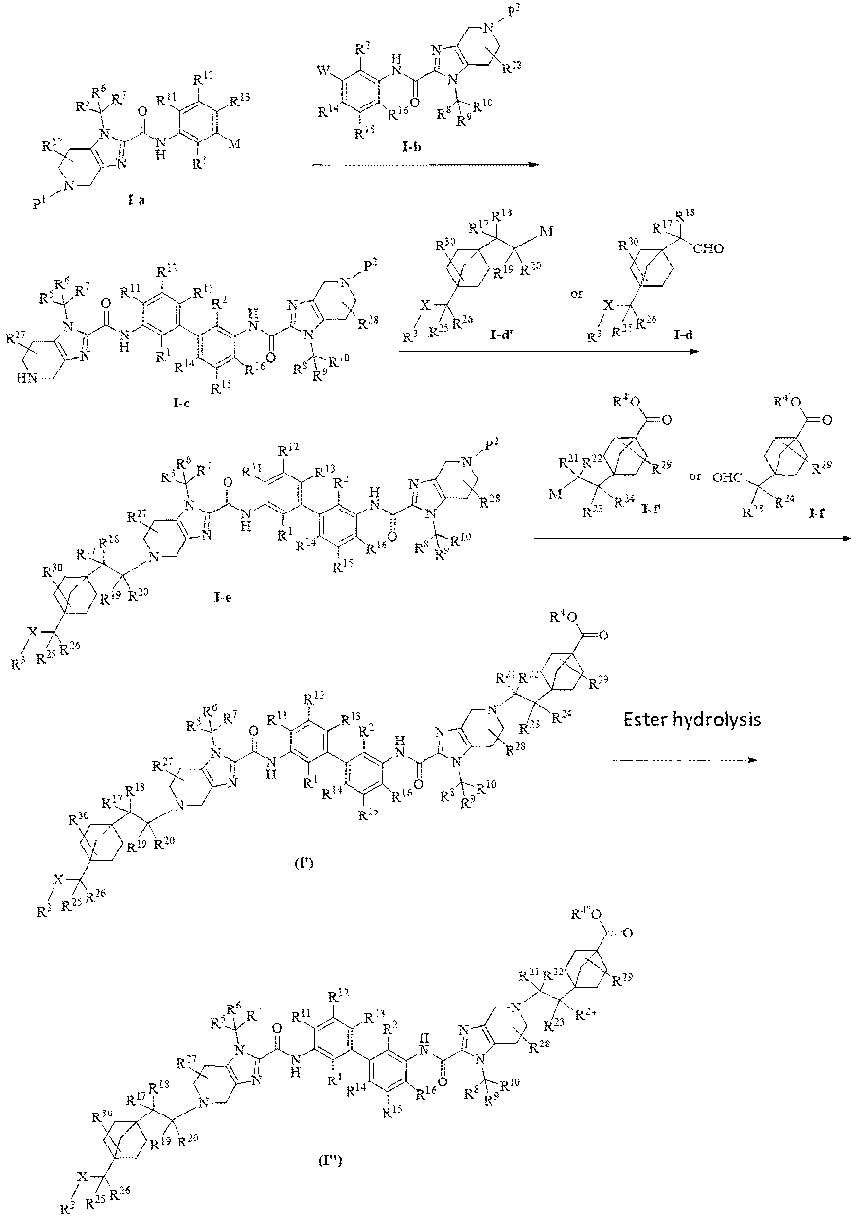
1) subjecting a compound represented by formula (I-a) to a first acid, a first base or catalytic hydrogenolysis in a first solvent to remove the protecting group P¹, further subjecting the obtained product, without separation and purification, to a Suzuki reaction with a compound represented by formula (I-b) in a second solvent and in the presence of a first catalyst and a second base to obtain a compound represented by formula (I-c);
2) subjecting the compound represented by formula (I-c) and a compound represented by formula (I-d) to a reductive amination reaction in a third solvent and in the presence of a first reducing agent to obtain a compound represented by formula (I-e); or,
subjecting the compound represented by formula (I-c) and the compound represented by formula (I-d') to a nucleophilic substitution reaction in a fourth solvent and in the presence of a third base to obtain a compound represented by formula (I-e);
3) subjecting the compound represented by formula (I-e) and a compound represented by formula (I-f) to a reductive amination reaction in a fifth solvent and in the presence of a second reducing agent to obtain a compound represented by formula (I') as the compound represented by formula (I); or,
subjecting the compound represented by formula (I-e) and a compound represented by formula (I-f') to a nucleophilic substitution reaction in a sixth solvent and in the presence of a fourth base to obtain a compound represented by formula (I') as the compound represented by formula (I);
optionally, further comprising
4) subjecting the compound represented by formula (I') to ester hydrolysis in the presence of a fifth base to obtain a compound represented by formula (I");
wherein:
the definitions of X and R¹-R³⁰ are as described according to any one of claims 1-13;
R^{4'} has the same definition as R⁴ except that it is not hydrogen;
R^{4"} is hydrogen;
M is selected from the group consisting of borate and boric acid, preferably selected from the group consisting of 4,4,5,5-tetramethyl-1,3,2-dioxaborolane, bis(neopentyl glycolato)diboron, 4,4,4',4',5,5,5',5'-octamethyl-2, 2'-bis(1,3, 2-dioxaborolane) B(OBu-*n*)₃, and B (OPr-*i*)₃; or,
M is selected from the group consisting of bromine, iodine, chlorine, fluorine, and CF₃SO₃-(OTf);
W is selected from the group consisting of borate and boric acid, preferably selected from the group consisting of 4,4,5,5-tetramethyl-1,3,2-dioxaborolane, bis(neopentyl glycolato)diboron, 4,4,4',4',5,5,5',5'-octamethyl-2, 2'-bis(1,3, 2-dioxaborolane) B(OBu-*n*)₃, and B (OPr-*i*)₃; or,
W is selected from the group consisting of bromine, iodine, chlorine, fluorine, and CF₃SO₃-(OTf);
P¹ and P² are protecting groups, which are the same or different, preferably selected from the group consisting of Boc (tert-butoxycarbonyl), Fmoc (9-fluorenylmethoxycarbonyl), Cbz (N-benzyloxycarbonyl), methanesulfonyl, p-toluenesulfonyl, acetyl, methoxycarbonyl, ethoxycarbonyl, ((2-trimethylsilyl)ethoxy)methyl (SEM), and tetrahydro-2H-pyran-2-yl (THP).

15. The method according to claim 14, wherein,
the first acid is preferably selected from the group consisting of trifluoroacetic acid (TFA), hydrochloric acid (HCl), acetic acid (HOAc), and hydrobromic acid (HBr);
the first base is preferably selected from the group consisting of piperidine and diethylamine;
the first solvent is preferably selected from the group consisting of dichloromethane (DCM), 1,2-dichloroethane, methanol (MeOH), ethanol (EtOH), 1,4-dioxane, tetrahydrofuran (THF), acetonitrile (MeCN), and N, N'-dimethylformamide (DMF);
the first catalyst is preferably selected from the group consisting of 1,1'-bis(dicyclohexylphosphino)ferrocenepalladium dichloride (PdCl₂(dcypf)), palladium acetate (Pd(OAc)₂), palladium dichloride (PdCl₂), tris(dibenzylideneacetone) dipalladium (Pd₂(dba)₃), [1,1'-bis(diphenylphosphino)ferrocene]palladium dichloride (PdCl₂(dppf)), a complex of [1,1'-bis(diphenylphosphine)ferrocene]palladium dichloride and dichloromethane (PdCl₂(dppf)·CH₂Cl₂), tetrakis(triphenylphosphine)palladium (Pd(PPh₃)₄), bis(tricyclohexylphosphine)palladium dichloride (PdCl₂(P(Cy)₃)₂), 2-dicyclohexylphosphine-2', 6'-dimethoxybiphenyl (SPhos);
the second base is selected from the group consisting of organic base and inorganic base, such as triethylamine (TEA), N,N-diisopropylethylamine (DIPEA), n-butyllithium, lithium diisopropylamide, lithium bis(trimethylsilyl)amide, potassium acetate (KOAc), sodium tert-butoxide (NaOBu-t), potassium tert-butoxide (KOBu-t), sodium hydride (NaH), potassium phosphate (K₃PO₄), sodium carbonate (Na₂CO₃), potassium carbonate (K₂CO₃), lithium hydroxide (KOH), and sodium hydroxide (NaOH);
the second solvent is preferably selected from the group consisting of 1,4-dioxane, tetrahydrofuran (THF), acetonitrile (MeCN), N,N'-dimethylformamide (DMF), and mixed solvents of these solvents with water in different ratios;
the first reducing agent and the second reducing agent are preferably selected from the group consisting of sodium acetate borohydride, sodium borohydride, and sodium cyanoborohydride;
the third solvent and fifth solvent are preferably selected from the group consisting of dichloromethane (DCM), 1,2-dichloroethane, methanol (MeOH), ethanol (EtOH), 1,4-dioxane, tetrahydrofuran (THF), acetonitrile (MeCN), and N,N'-dimethylformamide (DMF);
the fourth solvent and sixth solvent are preferably selected from the group consisting of dichloromethane (DCM), 1,2-dichloroethane, methanol (MeOH), ethanol (EtOH), 1,4-dioxane, tetrahydrofuran (THF), acetonitrile (MeCN), N,N'-dimethylformamide (DMF), N-methylpyrrolidone (NMP), and pyridine (Py);
the third base and the fourth base are preferably selected from the group consisting of triethylamine (TEA), N,N'-diisopropylethylamine (DIPEA), pyridine (Py), n-butyllithium, lithium diisopropylamide, lithium bis(trimethylsilyl)amide, sodium tert-butoxide (NaOBu-t), potassium tert-butoxide (KOBu-t), sodium hydride (NaH), sodium carbonate (Na₂CO₃), potassium carbonate (K₂CO₃), lithium hydroxide (KOH), and sodium hydroxide (NaOH);
the fifth base is preferably selected from the group consisting of lithium hydroxide (LiOH), lithium hydroxide (KOH), and sodium hydroxide (NaOH).

16. A method for preparing the compound, stereoisomer, pharmaceutically acceptable salt, precursor or metabolite thereof according to any one of claims 1-13, comprising the following steps: 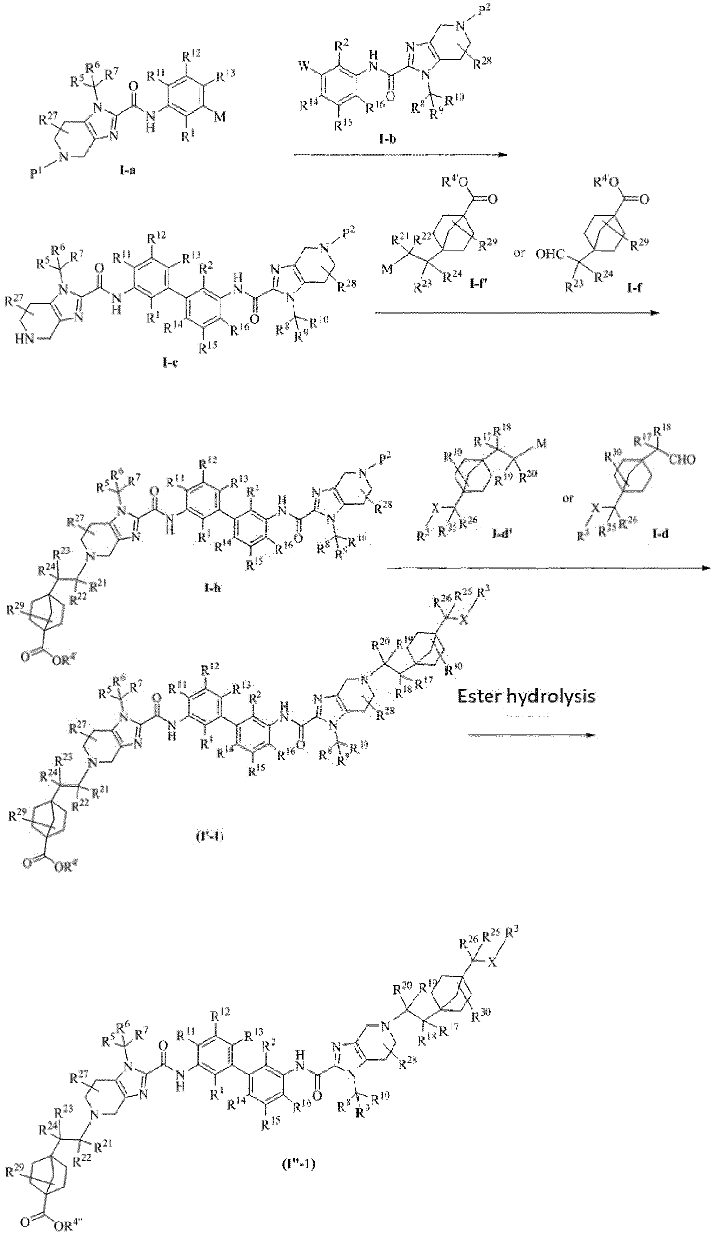
subjecting a compound represented by the formula (I-a) to removing protecting group P¹, further subjecting the obtained product, without separation and purification, to Suzuki reaction with a compound represented by the formula (I-b) to obtain a compound represented by the formula (I-c), first subjecting the compound represented by the formula (I-c) and a compound represented by formula (I-f) to a reductive amination reaction to obtain a compound represented by formula (I-h); or subjecting the compound represented by formula (I-c) and a compound represented by formula (I-f') to a nucleophilic substitution reaction to obtain a compound represented by formula (I-h), then subjecting the compound represented by (I-h) to deprotection, and then to a reductive amination reaction with a compound represented by formula (I-d) to obtain a compound represented by formula (I' -1) as the final product represented by formula (I); or subjecting the compound represented by (I-h) to deprotection, and then to a nucleophilic substitution reaction with a compound represented by formula (I-d') to obtain a compound represented by formula (I' -1) as the final product represented by formula (I), optionally further subjecting the compound represented by formula (I' -1) to an ester hydrolysis reaction to obtain a compound represented by formula (I"-1);
wherein, the deprotection reaction, Suzuki reaction, reductive amination reaction, nucleophilic substitution reaction and ester hydrolysis reaction described in each step are as described according to claim 14 or 15;
wherein, the definition of each substituent is as described according to claim 14 or 15.

17. A method for preparing the compound, stereoisomer, pharmaceutically acceptable salt, precursor or metabolite thereof according to any one of claims 1-13, comprising the following steps: 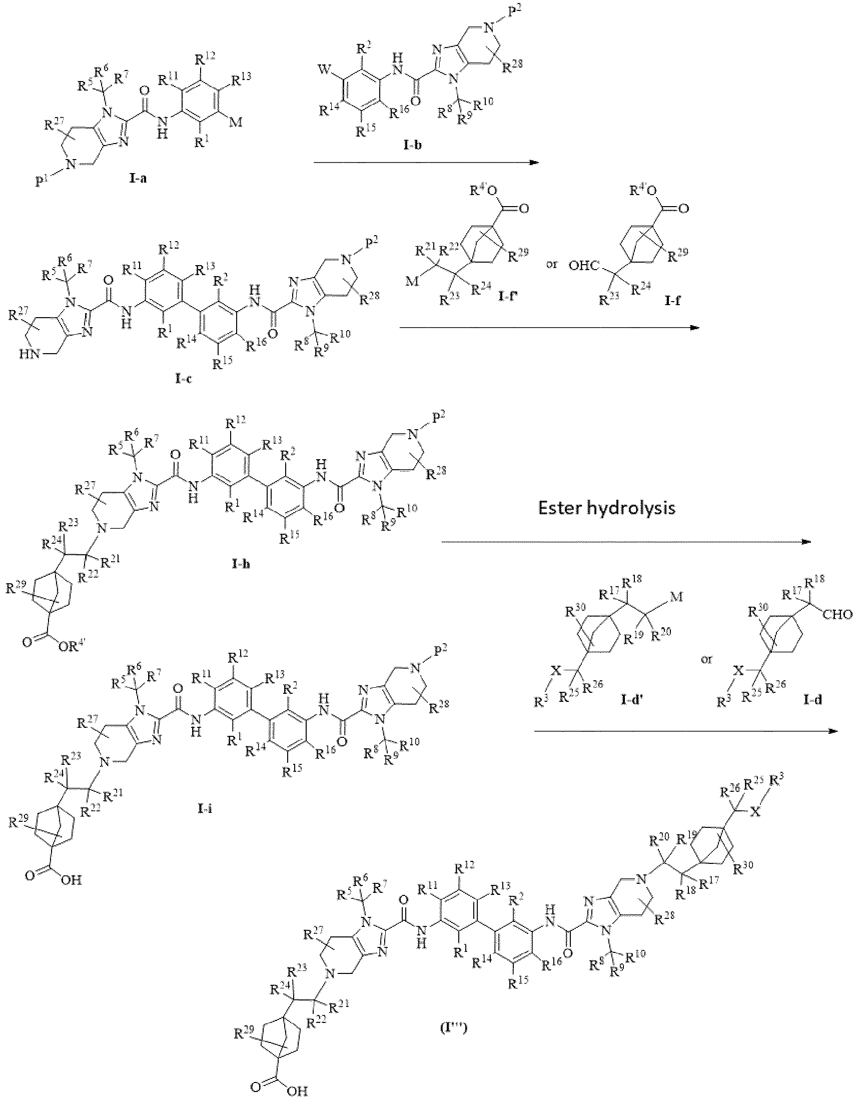
subjecting a compound represented by the formula (I-a) to removing protecting group P¹, further subjecting the obtained product, without separation and purification, to a Suzuki reaction with a compound represented by the formula (I-b) to obtain a compound represented by the formula (I-c), first subjecting the compound represented by the formula (I-c) and a compound represented by formula (I-f) to a reductive amination reaction to obtain a compound represented by formula (I-h); or subjecting the compound represented by formula (I-c) and a compound represented by formula (I-f') to a nucleophilic substitution reaction to obtain a compound represented by formula (I-h), then subjecting the compound represented by formula (I-h) to an ester hydrolysis reaction to obtain a compound represented by the formula (I-i), then subjecting the compound represented by the formula (I-i) to deprotection, and then to a reductive amination reaction as described above with a compound represented by formula (I-d) to obtain a compound represented by formula (I‴) as the final product represented by formula (I); or subjecting the compound represented by formula (I-i) to deprotection, and then to a nucleophilic substitution reaction with a compound represented by formula (I-d') to obtain a compound represented by formula (I‴) as the final product represented by formula (I);
preferably, the conditions of the deprotection reaction, Suzuki reaction, reductive amination reaction, nucleophilic substitution reaction and ester hydrolysis reaction described in each step are as described according to claim 14 or 15;
wherein, the definition of each substituent is as described according to claim 14 or 15.

18. A compound, which is selected from the group consisting of:

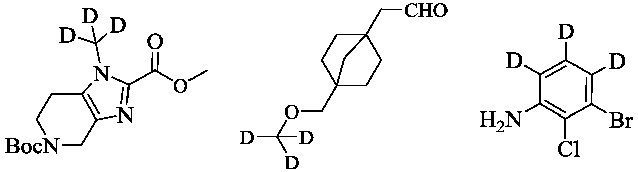

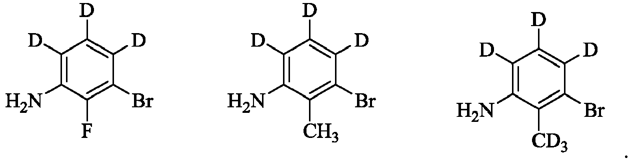

19. Use of the compound according to claim 18 in the preparation of a compound represented by formula (I).

20. A pharmaceutical composition comprising the compound, stereoisomer, pharmaceutically acceptable salt, precursor or metabolite thereof according to any one of claims 1-13, and an optional pharmaceutically acceptable carrier.

21. Use of the compound, stereoisomer, pharmaceutically acceptable salt, precursor or metabolite thereof according to any one of claims 1-13, or the pharmaceutical composition according to claim 20 in the manufacture of a medicament for treating and/or preventing a disease related to target PD-L1, or
use of the compound, stereoisomer, pharmaceutically acceptable salt, precursor or metabolite thereof according to any one of claims 1-13, or the pharmaceutical composition according to claim 20 in the manufacture of a medicament for inhibiting PD-L1 activity, or
use of the compound, stereoisomer, pharmaceutically acceptable salt, precursor or metabolite thereof according to any one of claims 1-13, or the pharmaceutical composition according to claim 20 in the manufacture of a medicament as a PD-L1 inhibitor, or
use of the compound, stereoisomer, pharmaceutically acceptable salt, precursor or metabolite thereof according to any one of claims 1-13, or the pharmaceutical composition according to claim 20 in the manufacture of a medicament as an immunomodulator targeting the PD-L1 signaling pathway.

22. The use according to claim 21, wherein the disease related to targeting PD-L1 is selected from the group consisting of tumors, cancers and other immune-related diseases.
